(19) Europäisches Patentamt
European Patent Office
Office européen des brevets

(11) Publication number: **0 035 893**
**B1**

(12) # EUROPEAN PATENT SPECIFICATION

(45) Date of publication of patent specification: **21.11.85**

(21) Application number: **81300956.0**

(22) Date of filing: **06.03.81**

(51) Int. Cl.⁴: **A 01 N 47/36,** C 07 D 239/42, C 07 D 239/46, C 07 D 251/16, C 07 D 251/22, C 07 D 251/46

(54) **Herbicidal benzene- and pyridine-sulfonamide derivatives.**

(30) Priority: **07.03.80 US 128176**
**26.03.80 US 134287**
**28.01.81 US 227886**

(43) Date of publication of application:
**16.09.81 Bulletin 81/37**

(45) Publication of the grant of the patent:
**21.11.85 Bulletin 85/47**

(84) Designated Contracting States:
**AT BE CH DE FR GB IT LI LU NL SE**

(58) References cited:
**EP-A-0 001 514**
**EP-A-0 013 480**
**EP-A-0 023 140**
**EP-A-0 023 422**
**US-A-4 169 719**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Adams, John Benjamin, Jr.**
**R. D. No. 2 Box 40 Auburn**
**Hockessin Delaware 19807 (US)**
Inventor: **Levitt, George**
**3218 Romilly Road**
**Wilmington Delaware 19810 (US)**

(74) Representative: **Hildyard, Edward Martin et al**
**Frank B. Dehn & Co. Imperial House**
**15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 035 893 B1

**Description**

*Background of the Invention*

This invention relates to novel alkyl sulfones. The compounds of this invention and their agriculturally suitable salts, are useful as agricultural chemicals, e.g. plant growth regulants and herbicides.

Netherlands Patent 121,788, published September 15, 1966, discloses the preparation of compounds of the following Formula and their use as general or selective herbicides:

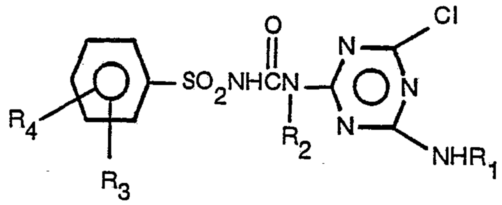

wherein

$R_1$ and $R_2$ may independently be alkyl of 1—4 carbon atoms; and

$R_3$ and $R_4$ may independently be hydrogen, chlorine or alkyl of 1—4 carbon atoms.

U.S. Patent 3,637,366 discloses compounds having the formula:

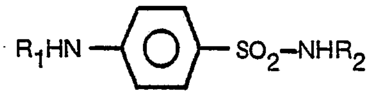

wherein

$R_1$ is hydrogen or lower saturated aliphatic acyl and

$R_2$ is hydrogen, 2-pyrimidinyl, pyridyl, amidino, acetyl or carbamoyl.

The disclosed compounds are said to provide control of crabgrass, cress, endive, clover and *Poa annua.*

French Patent 1,468,747 discloses the following *para*-substituted phenylsulfonamides as being useful as antidiabetic agents:

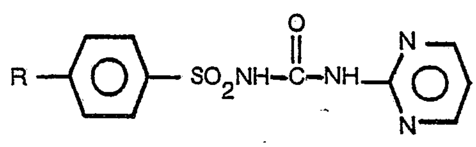

wherein

R = H, halogen, $CF_3$ or alkyl.

Logemann et al. Chem Ab., *53* 18052 g (1959), disclose a number of sulfonamides, including uracil derivatives and those having the formula:

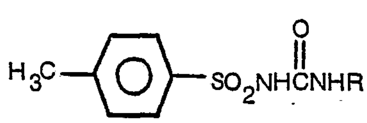

wherein

R is butyl, phenyl, or

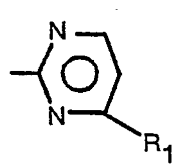

and

$R_1$ is hydrogen or methyl.

When tested for hypoglycemic effect in rats (oral doses of 25 mg/100 g), the compounds in which R is butyl and phenyl were most potent. The others were of low potency or inactive.

Wojciechowski, J. Acta. Polon. Pharm *19*, p. 121—5 (1962) [Chem. Ab., *59* 1633 e] describes the synthesis of N-[(2,6-dimethoxypyrimidin-4-yl)amino-carbonyl]-4-methylbenzenesulfonamide:

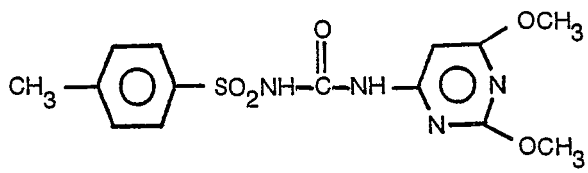

In our US—A—4,127,405 and US—A—4,169,719, agricultural sulfonamides of the following formula are taught:

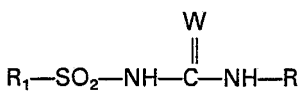

wherein

R is 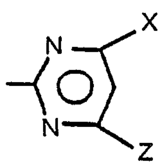 or 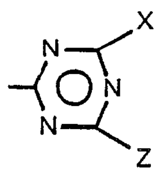 ; $R_1$ is 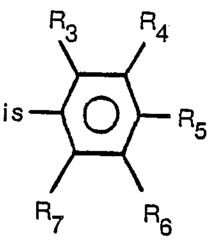 , 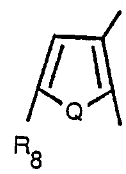 ,

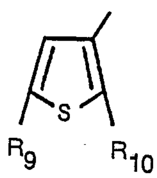 or 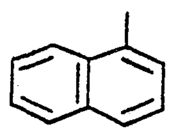 ;

$R_3$ and $R_6$ are independently hydrogen, fluorine, chlorine, bromine, iodine, alkyl of 1—4 carbon atoms, alkoxy of 1—4 carbon atoms, nitro, trifluoromethyl, cyano, $CH_3S(O)_n$— or $CH_3CH_2S(O)_n$—;

$R_4$ is hydrogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1—2 carbon atoms or alkoxy of 1—2 carbon atoms;

$R_8$ is hydrogen, methyl, chlorine or bromine;

$R_9$ and $R_{10}$ are independently hydrogen, methyl, chlorine or bromine;

W and Q are independently oxygen or sulfur;

n is 0, 1 or 2;

X is hydrogen, chlorine, bromine, methyl, ethyl, alkoxy, of 1—3 carbon atoms; trifluoro-methyl, $CH_3S$— or $CH_3OCH_2$—; and

Z is methyl or methoxy;

or their agriculturally suitable salts; provided that:

a) when $R_5$ is other than hydrogen, at least one of $R_3$, $R_4$, $R_6$ and $R_7$ is other than hydrogen and at least two of $R_3$, $R_4$, $R_6$ and $R_7$ mut be hydrogen;

b) when $R_5$ is hydrogen and all of $R_3$, $R_4$, $R_6$ and $R_7$ are other than hydrogen, then all of $R_3$, $R_4$, $R_6$ and $R_7$ must be either chlorine or methyl; and

c) when $R_3$ and $R_7$ are both hydrogen, at least one of $R_4$, $R_5$ or $R_6$ must be hydrogen.

The compounds of the present invention in general show significantly better selectivity to certain crops, such as soybeans or wheat, compared to the closest analogous compounds disclosed in the aforesaid U.S. Patents.

Other compounds more distantly related to those of the present invention are shown in our EP—A—1,514 and in our EP—A—9,419, published on 2 April 1980.

The presence of undesired vegetation causes substantial damage to useful crops, especially agricultural products that satisfy man's basic food and fiber needs, such as cotton, rice, corn, wheat and the like. The current population explosion and concomitant world food and fiber shortage demand improvements in the efficiency of producing these crops. Preventing or minimizing loss of a portion of such valuable crops by killing, or inhibiting the growth of undesired vegetation is one way of improving this efficiency. A wide variety of materials useful for killing or inhibiting (controlling) the growth of undesired vegetation is available; such materials are commonly referred to as herbicides. The need still exists however, for more effective herbicides.

*Summary of the Invention*

This invention relates to compounds of Formula (I) and their agriculturally sutiable salts, suitable agricultural compositions containing them and methods of using them as general and/or selective pre-emergence and post-emergence herbicides and as plant growth regulants.

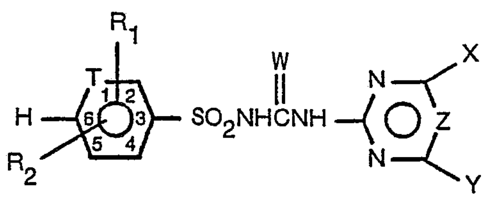

(I)

wherein

$R_1$ is $R_3S[O]_n$;

$R_3$ is $C_1$—$_4$ alkyl, $C_3$—$C_4$ alkenyl, cyclopentyl or cyclopropylmethyl;

$R_2$ is H, F, Cl, Br $CH_3$, $OCH_3$, $CF_3$, $NO_2$, CN or $NH_2$;

n is 0, 1 or 2;

W is O or S;

T is CH or N;

Z is CH or N;

X is $CH_3$, $C_2H_5$, $OCH_3$, $OC_2H_5$, or $CH_2OCH_3$; and

Y is $Ch_3$ or $OCH_3$;

and their agricultural salts; provided that

(1) when $R_2$ is CN, then T is CH and $R_2$ is not ortho to the sulfonylurea group;

(2) when W is S, then n is 0 or 2;

(3) when T is N, then n is 2;

(4) when T is CH, then $R_3$ is not $C_1$—$C_2$ alkyl and $R_1$ is at the 2- or 4- position and

(5) when T is N, then $R_3$ is not cyclopropylmethyl and $R_2$ is not $NH_2$, $CF_3$ or $NO_2$.

*Preferred Compounds*

Preferred in order of increasing activity and/or increasingly favorable ease of synthesis are:

(1) Compounds of Formula (I) wherein W is O and $R_2$ is H, F, Cl, Br, $CH_3$ or $OCH_3$;

(2) Compounds of Preferred (1) wherein $R_1$ is at the 2- or 4- position when T is N;

(3) Compounds of Preferred (2) wherein n is 2;

(4) Compounds of Preferred (3) wherein $R_2$ is H;

(5) Compounds of Preferred (4) wherein X is $CH_3$ or $OCH_3$; and

(6) Compounds of Preferred (5) wherein $R_3$ is $C_1$—$C_4$ alkyl.

Specifically preferred compounds for reasons of highest activity and/or most favorable ease of synthesis are:

N-[(4,6-dimethylpyrimidin-2-yl)aminocarbonyl]-2-[(1-methylethyl)sulfonyl]benzenesulfonamide;

N-[(4-methoxy-6-methylpyrimidin-2-yl)aminocarbonyl]-2-[(1-methylethyl)sulfonyl]benzenesulfonamide;

N-[(4,6-dimethoxypyrimidin-2-yl)aminocarbonyl]-2-[(1-methylethyl)sulfonyl]benzenesulfonamide;

N-[(4,6-dimethyl-1,3,5-triazin-2-yl)aminocarbonyl]-2-[(1-methylethyl)sulfonyl]benzenesulfonamide;

N-[(4-methoxy-6-methyl-1,3,5-triazin-2-yl)aminocarbonyl]-2-[(1-methylethyl)sulfonyl]benzenesulfonamide;

N-[(4,6-dimethoxy-1,3,5-triazin-2-yl)aminocarbonyl]-2-[(1-methylethyl)sulfonyl]benzenesulfonamide;

N-[(4,6-dimethylpyrimidin-2-yl)aminocarbonyl]-2-(propylsulfonyl)benzenesulfonamide;

N-[(4-methoxy-6-methylpyrimidin-2-yl)aminocarbonyl]-2-(propylsulfonyl)benzenesulfonamide;

N-[(4,6-dimethoxypyrimidin-2-yl)aminocarbonyl]-2-(propylsulfonyl)benzenesulfonamide;

N-[(4,6-dimethyl-1,3,5-triazin-2-yl)aminocarbonyl]-2-(propylsulfonyl)benzenesulfonamide;

N-[(4-methoxy-6-methyl-1,3,5-triazin-2-yl)aminocarbon-yl]-2-(propylsulfonyl)benzenesulfonamide;

N-[(4,6-dimethoxy-1,3,5-triazin-2-yl)aminocarbonyl]-2-(propylsulfonyl)benzenesulfonamide;

N-[(4,6-Dimethylpyrimidin-2-yl)aminocarbonyl]-2-(methylsulfonyl)-3-pyridinesulfonamide;

N-[(4-Methoxy-6-methylpyrimidin-2-yl)aminocarbonyl]-2-(methylsulfonyl)-3-pyridinesulfonamide;

N-[(4,6-Dimethoxy-1,3,5-triazin-2-yl)aminocarbonyl]-2-(methylsulfonyl)-3-pyridinesulfonamide;

N-[(4,6-Dimethoxypyrimidin-2-yl)aminocarbonyl]-2-(methylsulfonyl)-3-pyridinesulfonamide;

N-[(4,6-Dimethyl-1,3,5-triazin-2-yl)aminocarbonyl]-2-(methylsulfonyl)-3-pyridinesulfonamide; and

N-[(4-Methoxy-6-methyl-1,3,5-triazin-2-yl)aminocarbonyl]-2-(methylsulfonyl)-3-pyridinesulfonamide.

Any of these compounds may be used as their agriculturally suitable salts.

The compounds of the present invention include those compounds of the general formula (I) as defined in claims 11 and 12 herein.

*Synthesis*

As shown in reaction 1a, compounds of formula (I) can be prepared by reacting an appropriate 2-aminopyrimidine or 2-amino-1,3,5-triazine of Formula III with an appropriately substituted sulfonyl isocyanate or isothiocyanate of Formula II; $R_1$ is $R_3SO_2$ or $R_3S$ and $R_2 \neq$amino:

1a.

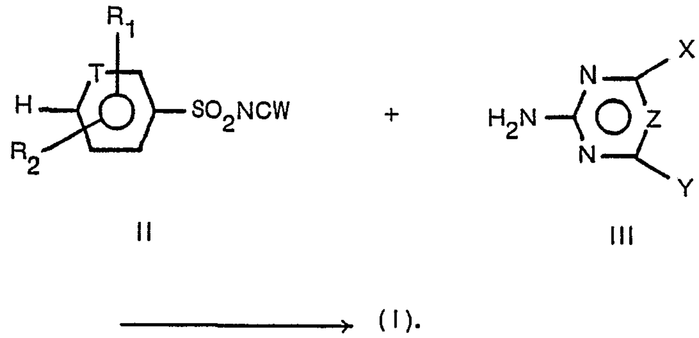

II III

(I).

This reaction is best carried out in an inert organic solvent (such as acetonitrile, tetrahydrofuran, methylene chloride, etc.). The reactants may be added in any order; the reaction is generally exothermic. Conveniently, the starting reaction temperature is ambient, but it can be varied from about 0 to 100° if desired. The product can be isolated by filtration if it precipitates from the reaction mixture; otherwise the solvent can be evaporated and the residual product obtained thereby, with optional purification through trituration with an organic solvent (e.g. diethyl ether, 1-chlorobutane, etc.) in which it is only sparingly soluble, or by recrystallization.

The compounds of Formula (I) in which $R_2$ is amino can be made by catalytic reduction of the corresponding nitro compound [R. N. Icke, et al., *Org. Syn. Coll. Vol.* 3, p. 59 (1955)], or by chemical reduction by methods known in the art.

An alternative method for preparation of compounds (I) with W = S, is to react the appropriate sulfonamide IV with a heterocyclic isothiocyanate;

1b.

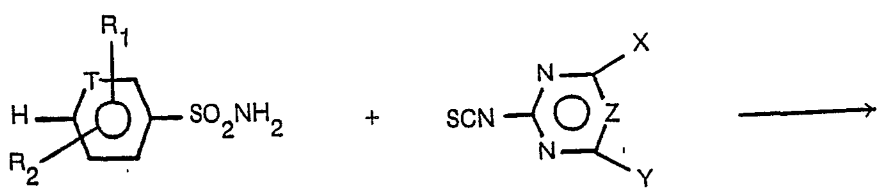

IV V

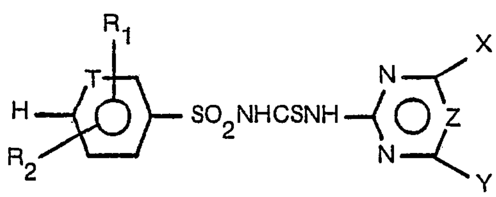

(I), with W = S.

The heterocyclic isothiocyanates used in this procedure can be made, for example, by the method of Japan Patent Application Pub: Kokai 51-143686, June 5, 1976, or that of W. Abraham and G. Barnikow Tetrahedron *29*, 691 (1973). Reaction 1b is best carried out in an inert, polar solvent (e.g., acetone or butanone) at 20 to 50°, in the presence of a basic catalyst (e.g., $K_2CO_3$ or $Na_2CO_3$), during about 1 to 10 hours. The alkali metal salt of (I), with W = S, is filtered off, suspended in water, and the pH adjusted down to 1—3 with acid (e.g. HCl or $H_2SO_4$) to form compound (I), with W = S, recovered by filtration.

Compounds of Formula (I), with W = O, can also be prepared by the reaction of the substituted sulfonamides of Formula IV with an appropriate heterocyclic isocyanate by the general method described in our European application No. 80304285.2 (EP-A-30140).

The intermediate suflonyl isocyanate IIa within Formula II can be prepared by reacting appropriate

sulfonamides IV with phosgene in the presence of *n*-butyl isocyanate at reflux in a solvent such as xylene, according to the procedure of H. Ulrich and A. A. Y. Sayigh, *Newer Methods of Preparative Organic Chemistry,* Vol. VI, p. 223—241, Academic Press, New York and London, W. Foerst Ed., as shown in reaction 2a:

2a.

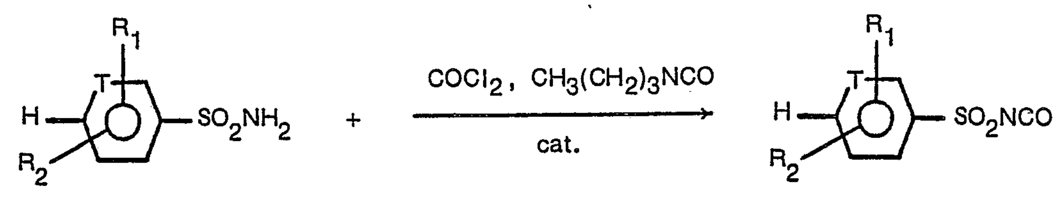


IV

IIa

The sulfonamide IV, an alkyl isocyanate (e.g. butyl isocyanate) and a catalytic amount of 1,4-diaza[2.2.2]bicyclooctane (DABCO) in xylene or other inert solvent of sufficiently high boiling point (e.g. ≥135°) are mixed and heated to about 135°, phosgene is added until an excess is present (indicated by a drop in boiling point). The mixture is further heated and excess phosgene driven off. After the mixture is cooled and filtered from insoluble material, the solvent, alkyl isocyanate and excess phosgene are evaporated, leaving the sulfonyl isocyanate IIa. If desired, the alkyl isocyanate/sulfonamide adduct can be made and isolated before reaction with the phosgene; in that case the sulfonamide IV, alkyl isocyanate and anhydrous base (e.g. $K_2CO_3$) in an unreactive solvent (e.g., acetone, butanone or acetonitrile) are mixed and boiled under reflux for 1/2 to 4 hours, followed by dilution of the reaction mixture with water and adjustment of pH to about 3 with acid (e.g., HCl, $H_2SO_4$, etc.), followed by filtration to provide the adduct, which can be reacted with phosgene as described above.

An alternative method for preparation of sulfonyl isocyanates IIa is the method of Ulrich et al. [J. Org. Chem. *34,* 3200 (1969)], from a suitably substituted sulfonamide of Formula IV as shown in reaction sequence 2b:

2b:

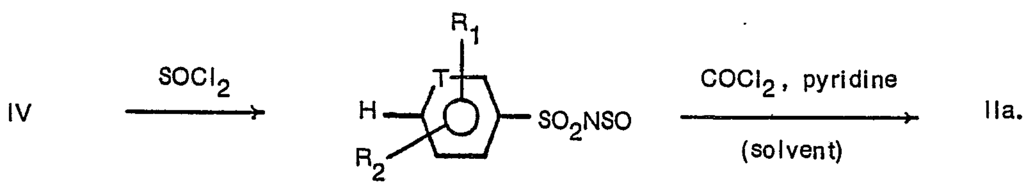


VI

The sulfonamide is boiled under reflux with an excess of thionyl chloride, which functions as a reactant and solvent. The reaction is continued until the sulfonamide protons are undetectable in the proton resonance spectrum. An overnight reaction period (about 16 hours) is frequently sufficient, though several days (e.g. 5) may be required in some cases to convert completely the sulfonamide IV to the thionylamide VI. The thionyl chloride is evaporated and the residue treated with an inert solvent (e.g., exylene, toluene, benzene, etc.) at least one equivalent of phosgene, and a catalytic amount of pyridine. The mixture is heated to about 60—140°, with 80—100° preferred. Conversion to the isocyanate is substantially complete within about 1/4 to 3 hours. The mixture containing the isocyanate can be used directly for the next reaction step (formation of compound (I), with W = O] or isolated in purified form by filtration and/or evaporation of solvent.

The intermediate sulfonyl isothiocyanates (IIb) within Formula II can be prepared by the method of Hartke [Chem. Abstr. *64,* 15783e (1966)] or U.S. Patent 3,346,590, as shown in reaction sequence 2c:

2c.

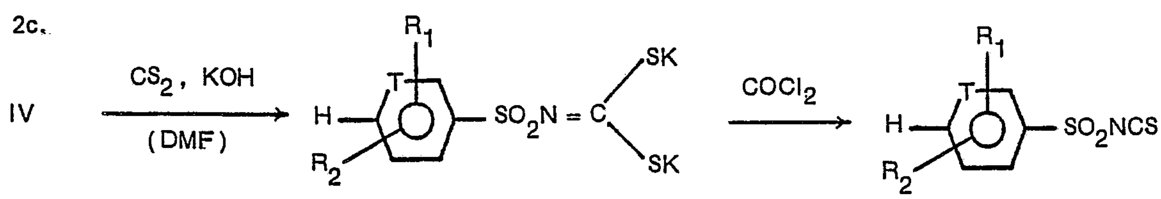


VII

IIb

The appropriate sulfonamide in DMF is treated with an equivalent of carbon disulfide and two equivalents of powdered potassium hydroxide at about 35°; other bases, including non-nucleophilic bases

such as sodium hydride, can be used instead of KOH. The mixture is stirred (about 1—8 hours) until solution, is substantially complete, then diluted with an aprotic solvent (e.g., ethyl acetate) to precipitate the intermediate potassium salt VII. The latter is separated by filtration of the reaction mixture, suspended in an inert solvent (e.g., toluene of xylene) and treated with two moles of phosgene (or thionyl chloride, etc.) at about 0°. The mixture is allowed to warm to ambient temperature, filtered and the sulfonyl isothiocyanate used as-is for formation of compound (I), with W = S, or isolated by evaporation of the solvent. The sulfonyl isothiocyanates may dimerize or trimerize in some cases, but the dimers and trimers still produce the compounds (I) on reaction with the amine III.

The intermediate sulfonamides IV can be prepared by well-known methods. For example, the preparation of sulfonamides for ammonium hydroxide and sulfonyl chloride is widely reported in the literature, e.g., Crossley et al., *J. Am. Chem. Soc. 60,* 2223 (1938). Preparation utilizing the reaction of sulfonyl chlorides with an excess of anhydrous ammonia at 0° in ethyl ether or chlorobutane is also known.

Certain sulfonyl chlorides are best prepared by chlorosulfonation of a substituted benzene according to the teaching of H. T. Clarke et al., *Org. Synth.* Coll. Vol. 1, 2nd Ed., 1941, p. 85. Pyridinesulfonyl chlorides and other benzenesulfonyl chlorides are best prepared by diazotization of the appropriate amino compound with sodium nitrite in HCl, followed by reaction of the diazonium salt with sulfur dioxide and cuprous chloride in acetic acid according to the teaching of H. L. Yale and F. Sowinski, *J. Org. Chem. 25,* 1824 (1960).

2-(Alkylsulfonyl)benzenesulfonyl isocyanates are important intermediates for the preparation of certain of the compounds of this invention. The synthesis of the sulfonamide intermediates required for these sulfonyl isocyanates is described in reaction sequences 3, 4, 5 and 6.

3.

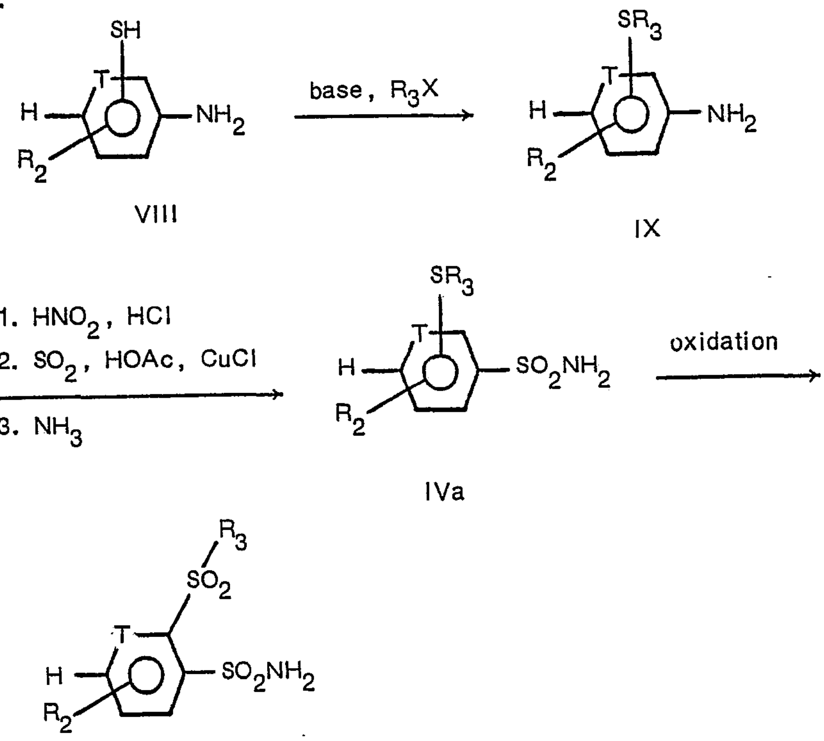


VIII

IX

IVa

IVb

The thioether of Formula VIII can be prepared from the appropriate 2-aminomercaptoaromatic and an alkyl halide $R_3X$ as described in the literature, e.g., R. N. Prasad, et al. *Can. J. Chem. 44,* 1247 (1966). The formation of the sulfonyl chloride and the corresponding sulfonamide IVa has been previously described (co-pending application U.S.S.N. 082,522, filed October 19, 1979). The oxidation of IVa to the corresponding 2-(alkylsulfonyl)benzenesulfonamides of Formula IVb can be carried out by a variety of standard literature procedures with *m*-chloroperbenzoic acid [C. R. Johnson, et al., *Tetrahedron 25,* 5649 (1969)], or with aqueous hydrogen peroxide in acetic acid (F. G. Bordwell, et al., *J. Amer. Chem. Soc. 77,* 1141 (1955)].

4.

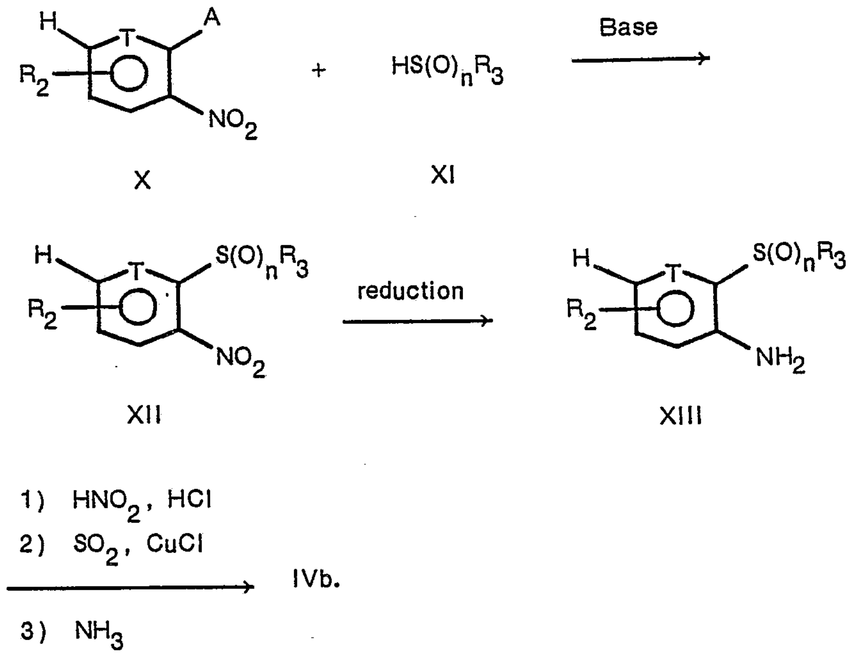


Halide displacement by thiols (n = O) or sulfinates (n = 2) is widely reported in the literature (for general reviews see, "Organic Chemistry of Sulfur", S. Oae, ed., Plenum Press, New York, 1977, pp. 232-233; Reid, "Organic Chemistry of Bivalent Sulfur," Chemical Publishing Co., New York, Vol. 2, pp. 16—21, 24—29; Vol. 3, pp 11—14; Peach, in Patai, "The Chemistry of the Thiol Group," pt. 2, pp. 735—744, John Wiley and Sons, Inc., New York, 1974). Reduction of XII to the amine XIII can be carried out by a variety of standard literature procedures, including catalytic hydrogenation (Rylander, "Catalytic Hydrogenation over Platinum Metals", pp. 168—202, Academic Press, Inc., New York, 1967) and reduction with iron [D. Cowsley et al., *Synthesis* 118 (1977)] or stannous chloride [*Org. Synth.,* Coll. Vol. 2, 130 (1943); *ibid, 3,* 240, 453 (1955)] in acidic medium.

Compounds of Formula XII can also be prepared as shown in reaction sequence 5, where n = 0 or 2 [in addition to references cited above, see, *Zh Obshch. Khim, 35* (8) 1361 (1965), and *J. Chem. Soc. 763.* (1946)]. The mercaptan or sulfinic acid is alkylated with an alkyl halide $R_3X$ in the presence of a base (e.g., tertiary amine; metal oxide, hydroxide, hydride or carbonate) to produce the nitroaryl sulfide or sulfone XII, which is converted to the sulfonamide IVc:

5.

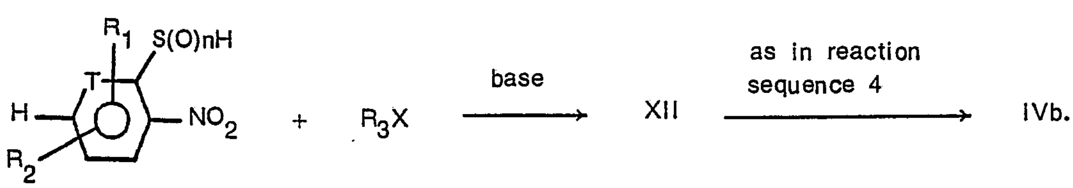


Starting pyridinesulfonamides IVd can be made by the method shown in reaction sequence 6 [Ref.: Chem. Abstr. *83,* 163951 p (1975)]. Thus, a mercaptan reacts with the chloropyridine compound XIV in the presence of a base to form the sulfide XV, which is then oxidized to the sulfone IVd.

6.

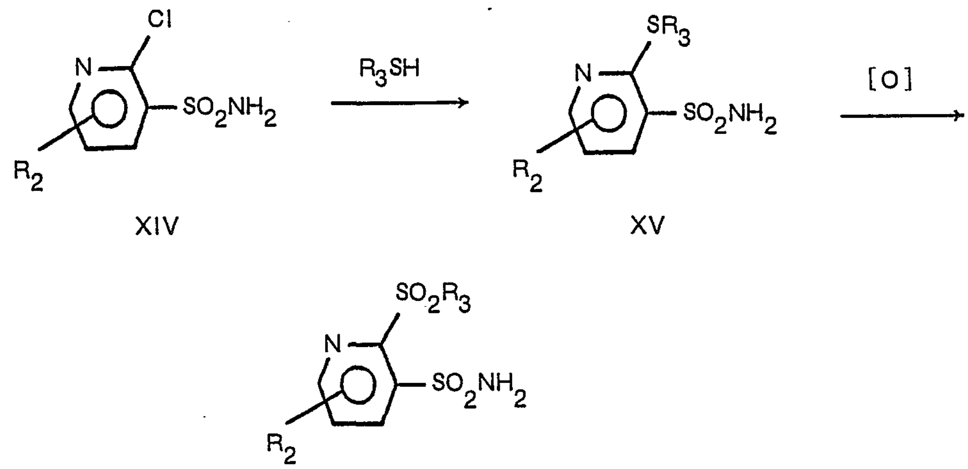


XIV

XV

IVd

Compounds of Formula Ib, were $R_1 = SOR_3$ and $W = O$ can be prepared from the appropriate compounds of Formula Ia where $R_1 = SR_3$ and $W = O$, by oxidation with *m*-chloroperbenzoic acid according to reaction 7.

7.

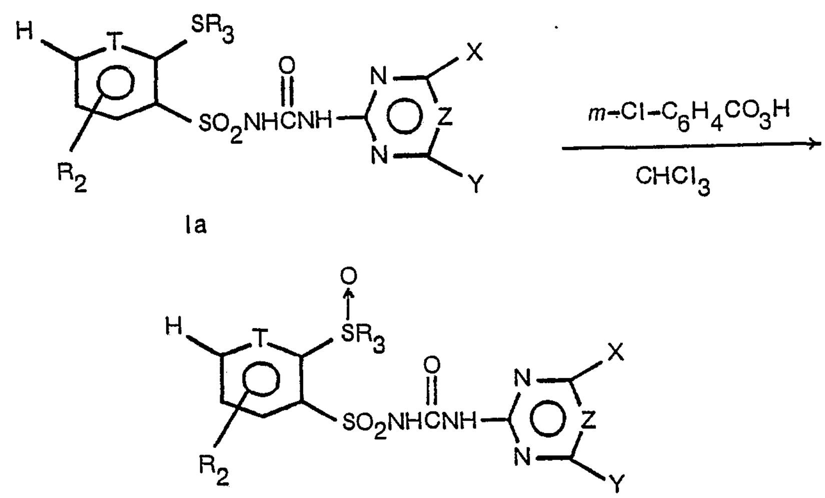


Ia

Ib

The reaction can be carried out by mixing equivalent amounts of Ia with *m*-chloroperbenzoic acid in an inert solvent such as chloroform and stirring at 0° to reflux for 12—24 hours, after which the insoluble *m*-chlorobenzoic acid produced is removed by filtration and the chloroform solution containing the desired sulfoxide is concentrated to yield the crude product. The product can be purified further by dissolving it in aqueous base of pH 10 and adjusting the pH to 4 to precipitate the desired compound while leaving the *m*-chlorobenzoic acid in solution as its sodium salt. Oxidation of Ia with 1 equivalent of hydrogen peroxide in acetic acid at 0° to room temperature will also produce Ib.

The synthesis of heterocyclic amine derivatives has been reviewed in "The Chemistry of Heterocyclic Compounds," a series published by Interscience Publ., New York and London. 2-Aninopyrimidines are described by D. J. Brown in "The Pyrimidines", Vol. XVI of the above series. 2-Amino-1,3,5-triazines can be synthesized according to the methods described by E. M. Smolin and L. Rapaport in "*s*-Triazines and Derivatives," Vol. XIII of the same series.

The synthesis of sulfur compounds of pyridine has been reviewed in "The Chemistry of Heterocyclic Compounds", a series published by Interscience Publ., N.Y. and London. Pyridinesulfonamides are described by H. L. Tale in "Pyridine and Its Derivatives" Supplement, Part 4 (1975), which is incorporated herein by reference.

Agriculturally suitable salts of compounds of Formula (I) are also useful herbicides and can be prepared in a number of ways known to the art. For example, metal salts can be made by contacting

compounds of Formula (I) with a solution of an alkali or alkaline earth metal salt having a sufficiently basic anion (e.g., hydroxide, alkoxide, carbonate or hydroxide). Quaternary amine salts can be made by similar techniques.

Salts of compounds of Formula (I) can also be prepared by exchange of one cation for another. Cationic exchange can be effected by direct contact of an aqueous solution of a salt of a compound of Formula (I) (e.g., alkali or quaternary amine salt) with a solution containing the cation to be exchanged. This method is most effective when the desired salt containing the exchanged cation is insoluble in water and can be separated by filtration.

Exchange may also be effected by passing an aqueous solution of a salt of a compound of Formula (I) (e.g., an alkali metal or quaternary amine salt) through a column packed with a cation exchange resin containing the cation to be exchanged for that of the original salt and the desired product is eluted from the column. This method is particularly useful when the desired salt is water-soluble.

Acid addition salts, useful in this invention, can be obtained by reacting a compound of Formula (I) with a suitable acid, e.g., *p*-toluenesulfonic acid, trichloroacetic acid or the like.

The compounds of this invention and their preparation are further illustrated by the following examples wherein temperatures are given in degrees centigrade and parts are by weight unless otherwise designated.

## Example 1

2-(1-Methylethylthio)benznamine

To a solution of 2-aminothiophenol (90%, 100 g, 0.72 mol) and sodium hydroxide (31.7 g. 0.792 mol) in 90% aqueous ethanol (500 ml) was added dropwise 2-bromopropane (89 g, 0.72 mol) at room temperature. When the addition was complete, the reaction mixture was refluxed for 3 hours, cooled to room temperature, and then concentrated *in vacuo.* The residue was diluted with water (300 ml) and extracted with ether (3 × 500 ml). The ether extracts were washed with water and brine, dried with $MgSO_4$ and concentrated *in vacuo* to yield the crude product. Distillation through a Vigreaux column gave 2-(1-methylethylthio)-benzenamine: bp 94° (2.2—2.4 mm).

NMR ($CDCl_3$)δ: 1.25 (d, 6H, —$CH_3$), 3.15 (hept., 1H, —CH—); 4.25 (broad s, 2H, —$NH_2$); 6.40—7.40 (m, 4H, Ar—H).

## Example 2

2-(1-Methylethylthio)benzenesulfonamide

To a solution of 2-(1-methylethylthio)benzenamine (200 g, 1.20 mol) in a mixture of concentrated hydrochloric acid (420 ml) and glacial acetic acid (120 ml) was added a solution of sodium nitrite (91.2 g, 1.32 mol) in water (180 ml) at −5° to 0°. The solution was stirred at 0° for 1/2 hour and then poured, in several portions, into a mixture of cupric chloride dihydrate (24 g) and liquid sulfur dioxide (370 ml, 8.28 mol), in glacial acetic acid (1200 ml) at 0°. The resulting solution was stirred at 0° for one hour and then allowed to warm to room temperature. After several hours, ether (1000 ml) was added, and the reaction mixture was stirred overnight and then poured into ice-water. The resulting oil was extracted into dichloromethane. The organic extracts were washed with water and 5% aqueous sodium bicarbonate (until neutral), dried ($MgSO_4$) and concentrated *in vacuo* to give an oil. The crude sulfonyl chloride was dissolved in dry THF (2000 ml) and treated with anhydrous liquid ammonia (50 ml, 2.3 mol) at 0°. The solution was stirred at 0° for one hour, allowed to warm to room temperature, and stirred an additional 2.5 hours. The reaction mixture was concentrated *in vacuo*, and the residue was diluted with water (1000 ml) and extracted with dichloromethane. The extracts were washed with water, dried ($MgSO_4$) and concentrated to yield the crude product. The oil was dissolved in hot (60°) hexane/*n*-butyl chloride (5/2). Upon cooling, 2-(1-methylethylthio)benzensulfonamide settled out as a dark oil.

NMR ($CCCl_3$)δ: 1.30 (d, —$CH_3$); 3.60 (hept. —CH—); 5.75 (broad s, —$NH_2$); 7.0—8.2 (m, Ar—H);
IR (Nujol): 3200—3300 doublet); 1300 and 1150 $cm^{-1}$.

## Example 3

2-[(1-Methylethyl)sulfonyl]benzenesulfonamide

To a solution of 2-[(1-Methylethyl)thio]benzenesulfonamide (79 g, 0.34 mol) in glacial acetic acid (200 ml) was added dropwise 30% aqueous hydrogen peroxide (100 ml, 1.16 mol) at room temperature. When the addition was complete, the reaction mixture was heated to 80° for 2 hours and then allowed to cool to room temperature. Addition of ice-water resulted in the precipitation of 2-[(1-Methylethyl)sulfonyl]benzenesulfonamide as a light-yellow solid, m.p. 150—153°.

NMR ($CDCl_3$)δ: 1.25 (d, —$CH_3$); 4.20 (hept, —CH—); 6.90 (broad s, —$NH_2$); 7.60—8.40 (m, Ar—H);
IR (KBr) 3400—3300 (d), 1330, 1290, 1160 and 1130 $cm^{-1}$.

## Example 4

N-[Butylaminocarbonyl]-2-[(1-methylethyl)sulfonyl]benzenesulfonamide

A mixture of 2-[(1-methylethyl)sulfonyl]benzenesulfonamide (29 g, 0.11 mol), *n*-butyl isocyanate (14.7 g, 0.15 mol) and anhydrous potassium carbonate (16.6 g, 0.120 mol) in methyl ethyl ketone (325 ml) was refluxed with stirring for 3 hours. The reaction mixture was cooled to 0° and then poured into ice-water.

The aqueous solution was acidified to pH 1 with concentrated hydrochloric acid. The resulting precipitate was filtered off, washed with water and recrystallized from ethanol to give N-[butylaminocarbonyl]-2-[(1-methylethyl)sulfonyl]benzenesulfonamide, m.p. 164.5—165.5°.

NMR (DMSO)δ: 0.6—1.6 (m, 13H, $-(CH_2)_2-$, $-CH_3$); 2.7-3.20 (m,2H, $-CH_2-$); 4.15 (hept, 1H —CH—); 6.85 (t, 1H, —NH—); 7.8—8.5 (m, 4.6H, Ar—H, —NH—);

IR (KBr) 3300, 1660, 1430, 1310, 1170 and 1130 $cm^{-1}$.

Example 5

2-[(1-Methylethyl)sulfonyl]benzenesulfonyl isocyanate

A solution of N-[butylaminocarbonyl]-2-[(1-methylethyl)sulfonyl]benzenesulfonamide (17.4 g, 0.048 mol) and 1,4-diaza[2.2.2]bicyclooctane (0.1 g) in mixed xylenes (90 ml) was heated to 135—140°. Liquid phosgene (3.6 ml, 0.050 mol) was added at a rate to maintain an internal temperature between 125—135°. When the addition was complete, the solution was refluxed for 2 hours, cooled to room temperature and then filtered under a nitrogen atmosphere. Removal of solvent *in vacuo* gave 2-[(1-methylethyl)sulfonyl]benzenesulfonyl isocyanate, which was used immediately wihout further purification.

Example 6

N-[(4,6-Dimethylpyrimidin-2-yl)aminocarbonyl]-2-[(1-methylethyl)sulfonyl]benzenesulfonamide

A solution of 2-[(1-methylethyl)sulfonyl]benzenesulfonyl isocyanate, (2.3 g, 0.008 mol) in dichloromethane (12 ml) was added to 2-amino-4,6-dimethylpyrimidine (0.74 g, 0.006 mol) and the suspension was stirred overnight at room temperature. The solvent was removed *in vacuo* and acetonitrile was added to the residue. The precipitated solid was filtered off and dried to give N-[(4,6-dimethylpyrimidin-2-yl)aminocarbonyl]-2-[(1-methylethyl)sulfonyl]benzenesulfonamide as a white powder, m.p. 188—193° (dec.).

NMR ($CDC1_3$)δ: 1.30 (d, $-C(CH_3)_2$); 2.45 (s, het—$CH_3$); 6.75 (s, het—H). IR (KBr) 1700, 1600, 1550, 1420, 1330, 1300, 1160 and 1130 $cm^{-1}$.

Example 7

Preparation of 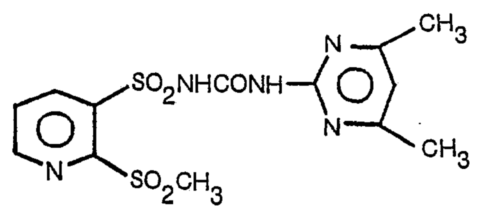

a. 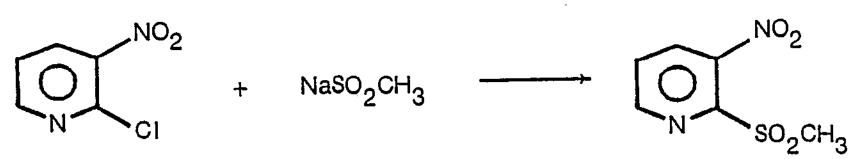


A B

A mixture of 22.1 g (0.139 mole) of 2-chloro-3-nitropyridine, 14.5 g (0.142 mole) of sodium methanesulfinate and 150 ml of DMF was boiled under reflux for 1 hour. The DMF was evaporated in vacuum and the residue extracted with ethyl acetate. The ethyl acetate extract was washed with water, dilute brine and saturated brine, dried ($MgSO_4$) and evaporated in vacuum to an oil. The oil was crystallized from butyl chloride and the solid twice recrystallized from acetone/hexane to provide 9.1 g of the sulfone B as a tan solid, m.p. 104—107°.

b. 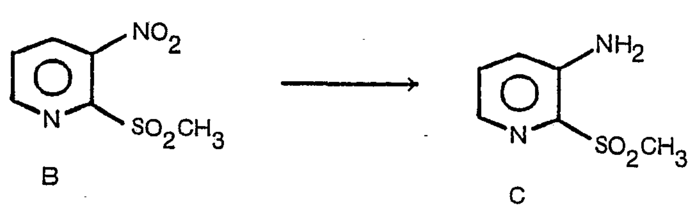

B C

A solution of 9.68 g (0.0479 mole) of the sulfone B in 50 ml of acetic acid was treated with 12.5 ml of water, then, portionwise with 11.5 g of powdered iron. The temperature was kept at ≤95° during the exothermic reaction by periodic cooling. After an additional 10 minutes at about 83° the mixture was filtered, the filtrate diluted with water and the pH raised to about 6 by gradual addition of 50% NaOH, with

cooling to ≤25°; the solution was then evaporated to dryness in vacuum. The residue was treated with ethyl acetate and sodium bicarbonate, the ethyl acetate solution dried ($MgSO_4$), filtered, and evaporated to 7.41 g of a syrup, which was the amine C. Mass spectral analysis of the syrup showed the expected molecular ion, m/e 172, for amine C.

c. 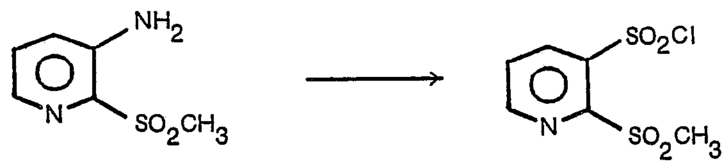

C D

A solution of 6.73 g (0.0391 mole) of the aminopyridyl sulfone D in 9 ml of acetic acid was added to 29 ml of cold conc. HCl at 0 to 10°. The solution was treated, portionwise, at 0 to 5°, with a solution of 3.83 g of sodium nitrite in 10.2 ml of water, with development of an orange color. After an additional 15 minutes at this temperature, the diazonium mixture was added, in portions, to a stirred mixture of 1.1 g of cuprous chloride, 8 ml (liquid) of sulfur dioxide and 42 ml of acetic acid at 5 to 15°; gas evolution occurred rapidly throughout the addition. After an additional 15 minutes, the mixture was warmed to 25°, and the resulting mixture poured into excess ice water. Precipitated white solid was filtered off, washed with water, and dried, providing 5.26 g of the sulfonyl chloride D as a white solid, m.p. 162—163° (dec.).

d. 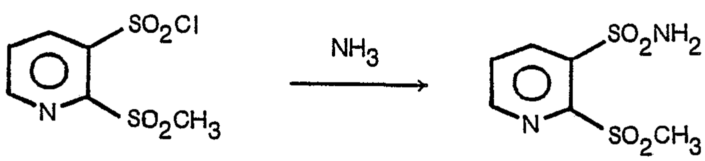


D E

The Sulfonyl chloride D obtained in Part c was dissolved in THF, cooled in an ice bath and gassed with ammonia, with a resulting exothermic reaction along with the precipitation of a white solid. The mixture was evaporated in vacuum to a white solid, which was washed with water to remove ammonium chloride, leaving the sulfonamide E as a white solid, m.p. 195-196.5°. Mass spectral analysis of the solid showed a molecular ion m/e 237 (m + 1).

e. 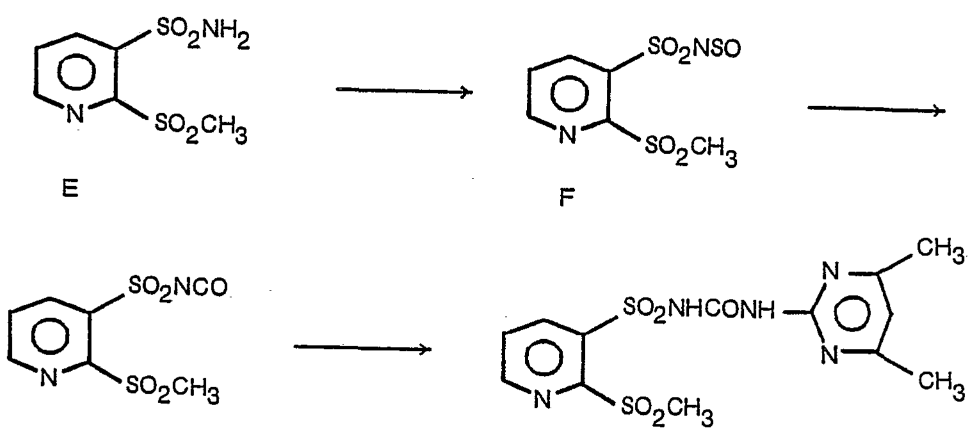

E F G H

One gram (0.00423 mole) of the sulfonamide E was suspended in 100 ml of thionyl chloride. The mixture was boiled under reflux for about 3 days, dissolution of the sulfonamide occurring in 10-15 minutes. The solution was evaporated to a clear, brown oil F. An excess of solution of phosgene in toluene (13.8% phosgene solution) was added along with 3-4 drops of pyridine. The mixture was heated at 85° under phosgene reflux for 2 hours, cooled, and filtered. The filtrate was evaporated to a solid G, which showed a strong isocyanate absorption peak, in the infrared spectrum (*ca.* 2250 $cm^{-1}$, nujol mull).

The solid isocyanate was dissolved in a little acetonitrile and treated with 0.5g of 2-amino-4,6-dimethylpyrimidine, a reaction quickly occurring with precipitation of white solid. After a few minutes the mixture was filtered and the white solid washed with acetonitrile and butyl chloride, leaving 0.71 g of the pyridyl sulfone H as a white solid, m.p. 234° (dec.). Mass spectral analysis

showed m/e 236, 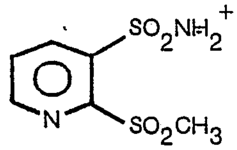 , and m/e 149, 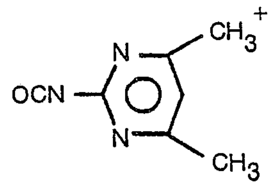 .

Anal. Calcd. for H, $C_{13}H_{15}N_5O_5S_2$(M.W. 385.42): C, 40.5; H, 3.9; N, 18.2.
Anal. Found: C, 40.8; H, 4.0; N, 18.5.

By procedures given in the discussion, included by reference, or illustrated in Examples 1—7, the illustrative compounds in the following tables can readily be made by one skilled in the art.

TABLE I

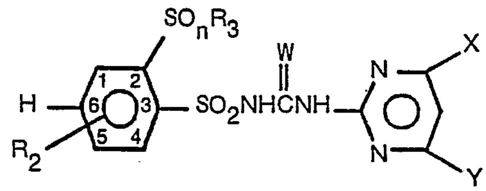

| n | $R_2$ | $R_3$ | X | Y | W | m.p. (°C) |
|---|---|---|---|---|---|---|
| 2 | H | $—n-C_3H_7$ | $CH_3$ | $CH_3$ | O | 200—202 (D) |
| 2 | H | $—n-C_3H_7$ | $OCH_3$ | $CH_3$ | O | 183—185 (D) |
| 2 | H | $—n-C_3H_7$ | $OCH_3$ | $OCH_3$ | O | 215—217 |
| 2 | H | $—CH(CH_3)_2$ | $CH_3$ | $CH_3$ | O | 188—193 (D) |
| 2 | H | $—CH(CH_3)_2$ | $OCH_3$ | $CH_3$ | O | 189—192 |
| 2 | H | $—CH(CH_3)_2$ | $OCH_3$ | $OCH_3$ | O | 237—239 |
| 2 | H | $—n-C_4H_9$ | $CH_3$ | $CH_3$ | O | 179—181.5 (D) |
| 2 | H | $—n-C_4H_9$ | $OCH_3$ | $CH_3$ | O | 152—155 |
| 2 | H | $—n-C_4H_9$ | $OCH_3$ | $OCH_3$ | O | 177—183 |
| 2 | H | $CH_2CH(CH_3)_2$ | $CH_3$ | $CH_3$ | O | 185—191 |
| 2 | H | $CH_2CH(CH_3)_2$ | $OCH_3$ | $CH_3$ | O | 180—185 |
| 2 | H | $CH_2CH(CH_3)_2$ | $OCH_3$ | $OCH_3$ | O | 215—218 |
| 2 | H | $CH(CH_3)C_2H_5$ | $CH_3$ | $CH_3$ | O | 174—178 (D) |
| 2 | H | $CH(CH_3)C_2H_5$ | $OCH_3$ | $CH_3$ | O | 198—203 |
| 2 | H | $CH(CH_3)C_2H_5$ | $OCH_3$ | $OCH_3$ | O | 208—213 |
| 2 | H | $C(CH_3)_3$ | $CH_3$ | $CH_3$ | O | 190—195 (D) |
| 2 | H | $C(CH_3)_3$ | $OCH_3$ | $CH_3$ | O | 192—195 |
| 2 | H | $C(CH_3)_3$ | $OCH_3$ | $OCH_3$ | O | 217—218 |
| 0 | H | $CH_2CH{=}CH_2$ | $CH_3$ | $CH_3$ | S | |
| 0 | 5-F | $CH{=}CHC_2H_5$ | $C_2H_5$ | $OCH_3$ | S | |
| 0 | 1-Cl | $CH_2CH_2CH{=}CH_2$ | $OC_2H_5$ | $OCH_3$ | S | |
| 0 | 4-Br | $CH{=}CHCH_3$ | $CH_2OCH_3$ | $CH_3$ | S | |
| 1 | 1-$CH_3$ | cyclopentyl | $C_2H_5$ | $OCH_3$ | O | |
| 1 | 5-$OCH_3$ | cyclopentyl | $CH_3$ | $OCH_3$ | O | |
| 1 | 5-$CF_3$ | cyclopentyl | $OC_2H_5$ | $CH_3$ | O | |

TABLE I (continued)

| n | $R_2$ | $R_3$ | X | Y | W | m.p. (°C) |
|---|---|---|---|---|---|---|
| 1 | 4-$NO_2$ | 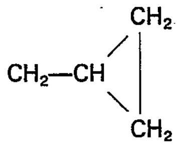 | $C_2H_5$ | $CH_3$ | O | |
| 1 | 5-CN | 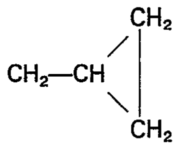 | $OCH_3$ | $OCH_3$ | O | |
| 1 | 4-$NH_2$ | 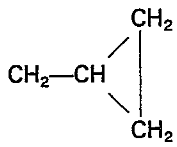 | $CH_2OCH_3$ | $OCH_3$ | O | |
| 0 | H | n-$C_3H_7$ | $C_2H_5$ | $CH_3$ | O | |
| 0 | H | n-$C_3H_7$ | $OC_2H_5$ | $CH_3$ | O | |
| 0 | H | n-$C_3H_7$ | $CH_2OCH_3$ | $CH_3$ | O | |
| 0 | H | n-$C_3H_7$ | $C_2H_5$ | $OCH_3$ | O | |
| 0 | H | n-$C_3H_7$ | $OC_2H_5$ | $OCH_3$ | O | |
| 0 | H | n-$C_3H_7$ | $CH_2OCH_3$ | $OCH_3$ | O | |
| 2 | H | n-$C_3H_7$ | $C_2H_5$ | $CH_3$ | O | |
| 2 | H | n-$C_3H_7$ | $OC_2H_5$ | $CH_3$ | O | |
| 2 | H | n-$C_3H_7$ | $CH_2OCH_3$ | $CH_3$ | O | |
| 2 | H | n-$C_3H_7$ | $C_2H_5$ | $OCH_3$ | O | |
| 2 | H | n-$C_3H_7$ | $OC_2H_5$ | $OCH_3$ | O | |
| 2 | H | n-$C_3H_7$ | $CH_2OCH_3$ | $OCH_3$ | O | |
| 2 | H | n-$C_3H_7$ | $CH_3$ | $CH_3$ | S | |
| 2 | H | n-$C_3H_7$ | $CH_3$ | $OCH_3$ | S | |
| 2 | H | n-$C_3H_7$ | $OCH_3$ | $OCH_3$ | S | |
| 0 | H | $—CH(CH_3)_2$ | $C_2H_5$ | $CH_3$ | O | |
| 0 | H | $—CH(CH_3)_2$ | $OC_2H_5$ | $CH_3$ | O | |
| 0 | H | $—CH(CH_3)_2$ | $CH_2OCH_3$ | $CH_3$ | O | |
| 0 | H | $—CH(CH_3)_2$ | $C_2H_5$ | $OCH_3$ | O | |
| 0 | H | $—CH(CH_3)_2$ | $OC_2H_5$ | $OCH_3$ | O | |
| 0 | H | $—CH(CH_3)_2$ | $CH_2OCH_3$ | $OCH_3$ | O | |

TABLE I (continued)

| n | $R_2$ | $R_3$ | X | Y | W | m.p. (°C) |
|---|---|---|---|---|---|---|
| 2 | H | $—CH(CH_3)_2$ | $C_2H_5$ | $CH_3$ | O | |
| 2 | H | $—CH(CH_3)_2$ | $OC_2H_5$ | $CH_3$ | O | |
| 2 | H | $—CH(CH_3)_2$ | $CH_2OCH_3$ | $CH_3$ | O | |
| 2 | H | $—CH(CH_3)_2$ | $C_2H_5$ | $OCH_3$ | O | |
| 2 | H | $—CH(CH_3)_2$ | $OC_2H_5$ | $OCH_3$ | O | |
| 2 | H | $—CH(CH_3)_2$ | $CH_2OCH_3$ | $OCH_3$ | O | |
| 2 | H | $—CH(CH_3)_2$ | $CH_3$ | $CH_3$ | S | |
| 2 | H | $—CH(CH_3)_2$ | $CH_3$ | $OCH_3$ | S | |
| 2 | H | $—CH(CH_3)_2$ | $OCH_3$ | $OCH_3$ | S | |
| 0 | H | $n-C_3H_7$ | $CH_3$ | $CH_3$ | O | 191—200 (D) |
| 0 | H | $n-C_3H_7$ | $CH_3$ | $OCH_3$ | O | 160—165 |
| 0 | H | $n-C_3H_7$ | $OCH_3$ | $OCH_3$ | O | 165—170 |
| 1 | H | $n-C_3H_7$ | $CH_3$ | $CH_3$ | O | 110—116 |
| 1 | H | $n-C_3H_7$ | $CH_3$ | $OCH_3$ | O | 80—96 |
| 1 | H | $n-C_3H_7$ | $OCH_3$ | $OCH_3$ | O | 175—178 |
| 0 | H | $—CH(CH_3)_2$ | $CH_3$ | $CH_3$ | O | |
| 0 | H | $—CH(CH_3)_2$ | $CH_3$ | $OCH_3$ | O | |
| 0 | H | $—CH(CH_3)_2$ | $OCH_3$ | $OCH_3$ | O | |
| 1 | H | $—CH(CH_3)_2$ | $CH_3$ | $CH_3$ | O | |
| 1 | H | $—CH(CH_3)_2$ | $CH_3$ | $OCH_3$ | O | |
| 1 | H | $—CH(CH_3)_2$ | $OCH_3$ | $OCH_3$ | O | |
| 0 | H | 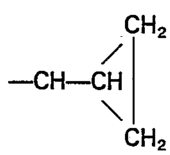 | $CH_3$ | $CH_3$ | O | |
| 0 | H | 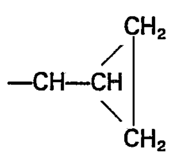 | $CH_3$ | $OCH_3$ | O | |
| 0 | H | 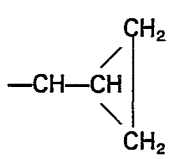 | $OCH_3$ | $OCH_3$ | O | |

TABLE I (continued)

| n | $R_2$ | $R_3$ | X | Y | W | m.p. (°C) |
|---|---|---|---|---|---|---|
| 2 | H | 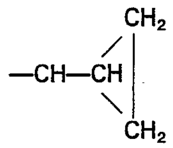 | $CH_3$ | $CH_3$ | O | |
| 2 | H | 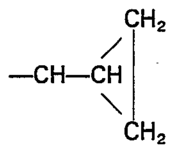 | $CH_3$ | $OCH_3$ | O | |
| 2 | H | 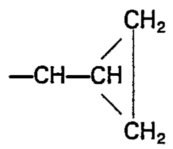 | $OCH_3$ | $OCH_3$ | O | |
| 0 | H | cyclopentyl | $CH_3$ | $CH_3$ | O | |
| 0 | H | cyclopentyl | $CH_3$ | $OCH_3$ | O | |
| 0 | H | cyclopentyl | $OCH_3$ | $OCH_3$ | O | |
| 2 | H | cyclopentyl | $CH_3$ | $CH_3$ | O | 206—208 (D) |
| 2 | H | cyclopentyl | $CH_3$ | $OCH_3$ | O | 211—214 |
| 2 | H | cyclopentyl | $OCH_3$ | $OCH_3$ | O | 219—224 |
| 0 | H | $—CH_2CH=CH_2$ | $CH_3$ | $CH_3$ | O | |
| 0 | H | $—CH_2CH=CH_2$ | $CH_3$ | $OCH_3$ | O | |
| 0 | H | $—CH_2CH=CH_2$ | $OCH_3$ | $OCH_3$ | O | |
| 2 | H | $—CH_2CH=CH_2$ | $CH_3$ | $CH_3$ | O | |
| 2 | H | $—CH_2CH=CH_2$ | $CH_3$ | $OCH_3$ | O | |
| 2 | H | $—CH_2CH=CH_2$ | $OCH_3$ | $OCH_3$ | O | |
| 2 | H | $—CH(CH_3)CH=CH_2$ | $CH_3$ | $CH_3$ | O | |
| 2 | H | $—CH(CH_3)CH=CH_2$ | $CH_3$ | $OCH_3$ | O | |
| 2 | H | $—CH(CH_3)CH=CH_2$ | $OCH_3$ | $OCH_3$ | O | |
| 2 | H | $—CH_2CH_2CH=CH_2$ | $CH_3$ | $CH_3$ | O | |
| 2 | H | $—CH_2CH_2CH=CH_2$ | $CH_3$ | $OCH_3$ | O | |
| 2 | H | $—CH_2CH_2CH=CH_2$ | $OCH_3$ | $OCH_3$ | O | |
| 0 | H | $n-C_3H_7$ | $CH_3$ | $CH_3$ | S | |
| 0 | H | $n-C_3H_7$ | $CH_3$ | $OCH_3$ | S | |
| 0 | H | $n-C_3H_7$ | $OCH_3$ | $OCH_3$ | S | |

TABLE II

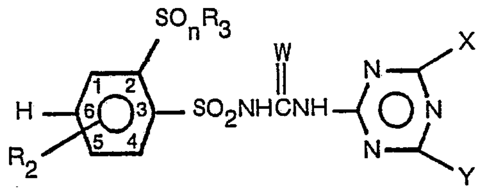

| n | $R_2$ | $R_3$ | X | Y | W | m.p. (°C) |
|---|---|---|---|---|---|---|
| 2 | H | $—n-C_3H_7$ | $CH_3$ | $CH_3$ | O | 189—191 (D) |
| 2 | H | $—n-C_3H_7$ | $OCH_3$ | $CH_3$ | O | 200—201 (D) |
| 2 | H | $—n-C_3H_7$ | $OCH_3$ | $OCH_3$ | O | 184—187 |
| 2 | H | $—CH(CH_3)_2$ | $CH_3$ | $CH_3$ | O | 115—119 (D) |
| 2 | H | $—CH(CH_3)_2$ | $OCH_3$ | $CH_3$ | O | 108—126 |
| 2 | H | $—CH(CH_3)_2$ | $OCH_3$ | $OCH_3$ | O | 108—130 |
| 2 | H | $—n-C_4H_9$ | $CH_3$ | $CH_3$ | O | 173—178 |
| 2 | H | $—n-C_4H_9$ | $OCH_3$ | $CH_3$ | O | 173—177 |
| 2 | H | $—n-C_4H_9$ | $OCH_3$ | $OCH_3$ | O | 175—179 |
| 2 | H | $CH_2CH(CH_3)_2$ | $CH_3$ | $CH_3$ | O | 180—183 |
| 2 | H | $CH_2CH(CH_3)_2$ | $OCH_3$ | $CH_3$ | O | 175—178 |
| 2 | H | $CH_2CH(CH_3)_2$ | $OCH_3$ | $OCH_3$ | O | 180—188 |
| 2 | H | $CH(CH_3)C_2H_5$ | $CH_3$ | $CH_3$ | O | 160—163 |
| 2 | H | $CH(CH_3)C_2H_5$ | $OCH_3$ | $CH_3$ | O | 170—173.5 |
| 2 | H | $CH(CH_3)C_2H_5$ | $OCH_3$ | $OCH_3$ | O | 173—181 |
| 2 | H | $C(CH_3)_3$ | $CH_3$ | $CH_3$ | O | 193—195 |
| 2 | H | $C(CH_3)_3$ | $OCH_3$ | $CH_3$ | O | 175—179 |
| 2 | H | $C(CH_3)_3$ | $OCH_3$ | $OCH_3$ | O | 178—181 |
| 2 | 1-$NH_2$ | $CH_2CH{=}CH_2$ | $CH_3$ | $CH_3$ | S | |
| 0 | 4-F | $CH{=}CHC_2H_5$ | $CH_2OCH_3$ | $OCH_3$ | S | |
| 0 | 1-Br | $CH_2CH_2CH{=}CH_2$ | $C_2H_5$ | $CH_3$ | S | |
| 0 | 5-Cl | 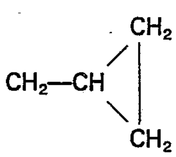 | $CH_2OCH_3$ | $CH_3$ | S | |
| 0 | 4-$CH_3$ | 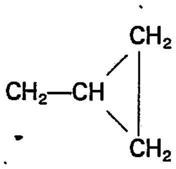 | $OC_2H_5$ | $OCH_3$ | S | |

TABLE II (continued)

| n | $R_2$ | $R_3$ | X | Y | W | m.p. (°C) |
|---|---|---|---|---|---|---|
| 1 | 1-$OCH_3$ | cyclopentyl | $CH_3$ | $CH_3$ | O | |
| 1 | 5-$CF_3$ | cyclopentyl | $OC_2H_5$ | $CH_3O$ | O | |
| 1 | 4-$NO_2$ | cyclopentyl | $CH_3$ | $CH_3$ | O | |
| 1 | 5-CN | cyclopentyl | $CH_2OCH_3$ | $CH_3$ | O | |
| 0 | H | n-$C_3H_7$ | $C_2H_5$ | $CH_3$ | O | |
| 0 | H | n-$C_3H_7$ | $OC_2H_5$ | $CH_3$ | O | |
| 0 | H | n-$C_3H_7$ | $CH_2OCH_3$ | $CH_3$ | O | |
| 0 | H | n-$C_3H_7$ | $C_2H_5$ | $OCH_3$ | O | |
| 0 | H | n-$C_3H_7$ | $OC_2H_5$ | $OCH_3$ | O | |
| 0 | H | n-$C_3H_7$ | $CH_2OCH_3$ | $OCH_3$ | O | |
| 2 | H | n-$C_3H_7$ | $C_2H_5$ | $CH_3$ | O | |
| 2 | H | n-$C_3H_7$ | $OC_2H_5$ | $CH_3$ | O | |
| 2 | H | n-$C_3H_7$ | $CH_2OCH_3$ | $CH_3$ | O | |
| 2 | H | n-$C_3H_7$ | $C_2H_5$ | $OCH_3$ | O | |
| 2 | H | n-$C_3H_7$ | $OC_2H_5$ | $OCH_3$ | O | |
| 2 | H | n-$C_3H_7$ | $CH_2OCH_3$ | $OCH_3$ | O | |
| 2 | H | n-$C_3H_7$ | $CH_3$ | $CH_3$ | S | |
| 2 | H | n-$C_3H_7$ | $CH_3$ | $OCH_3$ | S | |
| 2 | H | n-$C_3H_7$ | $OCH_3$ | $OCH_3$ | S | |
| 0 | H | $-CH(CH_3)_2$ | $C_2H_5$ | $CH_3$ | O | |
| 0 | H | $-CH(CH_3)_2$ | $OC_2H_5$ | $CH_3$ | O | |
| 0 | H | $-CH(CH_3)_2$ | $CH_2OCH_3$ | $CH_3$ | O | |
| 0 | H | $-CH(CH_3)_2$ | $C_2H_5$ | $OCH_3$ | O | |
| 0 | H | $-CH(CH_3)_2$ | $OC_2H_5$ | $OCH_3$ | O | |
| 0 | H | $-CH(CH_3)_2$ | $CH_2OCH_3$ | $OCH_3$ | O | |
| 2 | H | $-CH(CH_3)_2$ | $C_2H_5$ | $CH_3$ | O | |
| 2 | H | $-CH(CH_3)_2$ | $OC_2H_5$ | $CH_3$ | O | |
| 2 | H | $-CH(CH_3)_2$ | $CH_2OCH_3$ | $CH_3$ | O | |
| 2 | H | $-CH(CH_3)_2$ | $C_2H_5$ | $OCH_3$ | O | |
| 2 | H | $-CH(CH_3)_2$ | $OC_2H_5$ | $OCH_3$ | O | |
| 2 | H | $-CH(CH_3)_2$ | $CH_2OCH_3$ | $OCH_3$ | O | |

TABLE II (continued)

| n | $R_2$ | $R_3$ | X | Y | W | m.p. (°C) |
|---|---|---|---|---|---|---|
| 2 | H | $-CH(CH_3)_2$ | $CH_3$ | $CH_3$ | S | |
| 2 | H | $-CH(CH_3)_2$ | $CH_3$ | $OCH_3$ | S | |
| 2 | H | $-CH(CH_3)_2$ | $OCH_3$ | $OCH_3$ | S | |
| 0 | H | n-$C_3H_7$ | $CH_3$ | $CH_3$ | O | 150—157 |
| 0 | H | n-$C_3H_7$ | $CH_3$ | $OCH_3$ | O | 128—135 |
| 0 | H | n-$C_3H_7$ | $OCH_3$ | $OCH_3$ | O | 152—155 |
| 1 | H | n-$C_3H_7$ | $CH_3$ | $CH_3$ | O | 163—166 |
| 1 | H | n-$C_3H_7$ | $CH_3$ | $OCH_3$ | O | 75—95 |
| 1 | H | n-$C_3H_7$ | $OCH_3$ | $OCH_3$ | O | 122—125 |
| 0 | H | $-CH(CH_3)_2$ | $CH_3$ | $CH_3$ | O | |
| 0 | H | $-CH(CH_3)_2$ | $CH_3$ | $OCH_3$ | O | |
| 0 | H | $-CH(CH_3)_2$ | $OCH_3$ | $OCH_3$ | O | |
| 1 | H | $-CH(CH_3)_2$ | $CH_3$ | $CH_3$ | O | |
| 1 | H | $-CH(CH_3)_2$ | $CH_3$ | $OCH_3$ | O | |
| 1 | H | $-CH(CH_3)_2$ | $OCH_3$ | $OCH_3$ | O | |
| 0 | H | 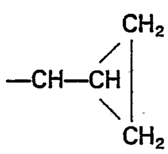 | $CH_3$ | $CH_3$ | O | |
| 0 | H | 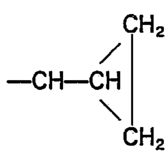 | $CH_3$ | $OCH_3$ | O | |
| 0 | H | 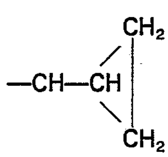 | $OCH_3$ | $OCH_3$ | O | |
| 2 | H | 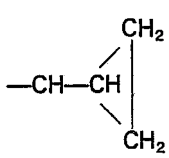 | $CH_3$ | $CH_3$ | O | |
| 2 | H | 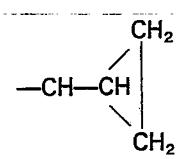 | $CH_3$ | $OCH_3$ | O | |

TABLE II (continued)

| n | $R_2$ | $R_3$ | X | Y | W | m.p. (°C) |
|---|---|---|---|---|---|---|
| 2 | H | 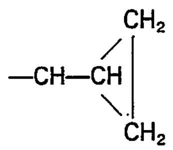 | $OCH_3$ | $OCH_3$ | O | |
| 0 | H | cyclopentyl | $CH_3$ | $CH_3$ | O | |
| 0 | H | cyclopentyl | $CH_3$ | $OCH_3$ | O | |
| 0 | H | cyclopentyl | $OCH_3$ | $OCH_3$ | O | |
| 2 | H | cyclopentyl | $CH_3$ | $CH_3$ | O | 183—186 |
| 2 | H | cyclopentyl | $CH_3$ | $OCH_3$ | O | 191—200 |
| 2 | H | cyclopentyl | $OCH_3$ | $OCH_3$ | O | 174—179 |
| 0 | H | $—CH_2CH{=}CH_2$ | $CH_3$ | $CH_3$ | O | |
| 0 | H | $—CH_2CH{=}CH_2$ | $CH_3$ | $OCH_3$ | O | |
| 0 | H | $—CH_2CH{=}CH_2$ | $OCH_3$ | $OCH_3$ | O | |
| 2 | H | $—CH_2CH{=}CH_2$ | $CH_3$ | $CH_3$ | O | |
| 2 | H | $—CH_2CH{=}CH_2$ | $CH_3$ | $OCH_3$ | O | |
| 2 | H | $—CH_2CH{=}CH_2$ | $OCH_3$ | $OCH_3$ | O | |
| 2 | H | $—CH(CH_3)CH{=}CH_2$ | $CH_3$ | $CH_3$ | O | |
| 2 | H | $—CH(CH_3)CH{=}CH_2$ | $CH_3$ | $OCH_3$ | O | |
| 2 | H | $—CH(CH_3)CH{=}CH_2$ | $OCH_3$ | $OCH_3$ | O | |
| 2 | H | $—CH_2CH_2CH{=}CH_2$ | $CH_3$ | $CH_3$ | O | |
| 2 | H | $—CH_2CH_2CH{=}CH_2$ | $CH_3$ | $OCH_3$ | O | |
| 2 | H | $—CH_2CH_2CH{=}CH_2$ | $OCH_3$ | $OCH_3$ | O | |
| 0 | H | $n\text{-}C_3H_7$ | $CH_3$ | $CH_3$ | S | |
| 0 | H | $n\text{-}C_3H_7$ | $CH_3$ | $OCH_3$ | S | |
| 0 | H | $n\text{-}C_3H_7$ | $OCH_3$ | $OCH_3$ | S | |

TABLE III

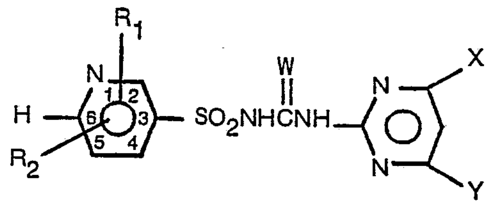

| $R_1$ | $R_2$ | X | Y | W |
|---|---|---|---|---|
| 2-$CH_3SO_2$ | H | $CH_3$ | $CH_3$ | O |
| 2-$CH_3SO_2$ | H | $CH_3$ | $OCH_3$ | O |
| 2-$CH_3SO_2$ | H | $OCH_3$ | $OCH_3$ | O |
| 2-$CH_3SO_2$ | H | $C_2H_5$ | $CH_3$ | O |
| 2-$CH_3SO_2$ | H | $OC_2H_5$ | $OCH_3$ | O |
| 2-$CH_3SO_2$ | H | $CH_2OCH_3$ | $CH_3$ | O |
| 2-$C_2H_5SO_2$ | H | $CH_3$ | $CH_3$ | O |
| 2-$C_2H_5SO_2$ | H | $CH_3$ | $OCH_3$ | O |
| 2-$C_2H_5SO_2$ | H | $OCH_3$ | $OCH_3$ | O |
| 2-$C_2H_5SO_2$ | H | $C_2H_5$ | $OCH_3$ | O |
| 2-$C_2H_5SO_2$ | H | $CH_2OCH_3$ | $CH_3$ | O |
| 2-$C_2H_5SO_2$ | H | $OC_2H_5$ | $CH_3$ | O |
| 2-*n*-$C_3H_7SO_2$ | H | $CH_3$ | $CH_3$ | O |
| 2-*n*-$C_3H_7SO_2$ | H | $OCH_3$ | $CH_3$ | O |
| 2-*n*-$C_3H_7SO_2$ | H | $OCH_3$ | $OCH_3$ | O |
| 2-$(CH_3)_2CHSO_2$ | H | $CH_3$ | $CH_3$ | O |
| 2-$(CH_3)_2CHSO_2$ | H | $CH_3$ | $OCH_3$ | O |
| 2-$(CH_3)_2CHSO_2$ | H | $OCH_3$ | $OCH_3$ | O |
| 2-$(CH_3)_2CHSO_2$ | H | $OC_2H_5$ | $CH_3$ | O |
| 2-n-$C_4H_9SO_2$ | H | $CH_3$ | $CH_3$ | O |
| 2-n-$C_4H_9SO_2$ | H | $OCH_3$ | $CH_3$ | O |
| 2-n-$C_4H_9SO_2$ | H | $OCH_3$ | $OCH_3$ | O |
| 2-$CH_3CH{=}CHSO_2$ | H | $CH_2OCH_3$ | $CH_3$ | O |
| 2-$CH_2{=}CHCH_2SO_2$ | H | $CH_3$ | $OCH_3$ | O |
| 2-$CH_3CH{=}CHCH_2SO_2$ | H | $OCH_3$ | $OCH_3$ | O |
| 2-$CH_2{=}CHCH_2CH_2SO_2$ | H | $CH_3$ | $OCH_3$ | O |
| 2-$CH_2{=}CHCH(CH_3)SO_2$ | H | $CH_3$ | $CH_3$ | O |

TABLE III (continued)

| $R_1$ | $R_2$ | X | Y | W |
|---|---|---|---|---|
| 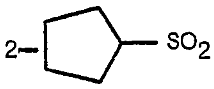 | H | $CH_3$ | $CH_3$ | O |
| 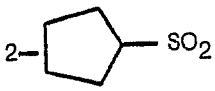 | H | $OCH_3$ | $CH_3$ | O |
| 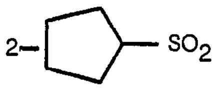 | H | $OCH_3$ | $OCH_3$ | O |
| 4-$CH_3SO_2$ | H | $CH_3$ | $CH_3$ | O |
| 4-$CH_3SO_2$ | H | $CH_3$ | $OCH_3$ | O |
| 4-$CH_3SO_2$ | H | $OCH_3$ | $OCH_3$ | O |
| 4-$CH_3SO_2$ | H | $CH_2OCH_3$ | $CH_3$ | O |
| 4-$C_2H_5SO_2$ | H | $C_2H_5$ | $OCH_3$ | O |
| 4-$C_2H_5SO_2$ | H | $OC_2H_5$ | $CH_3$ | O |
| 4-*n*-$C_3H_7SO_2$ | H | $CH_3$ | $CH_3$ | S |
| 4-*n*-$C_3H_7SO_2$ | H | $OCH_3$ | $CH_3$ | S |
| 4-*n*-$C_3H_7SO_2$ | H | $OCH_3$ | $OCH_3$ | S |
| 4-$(CH_3)_2CHCH_2SO_2$ | H | $CH_3$ | $CH_3$ | S |
| 4-$(CH_3)_2CHCH_2SO_2$ | H | $OCH_3$ | $CH_3$ | S |
| 4-$(CH_3)_2CHCH_2SO_2$ | H | $OCH_3$ | $OCH_3$ | S |
| 4-$CH_3CH_2CH(CH_3)SO_2$ | 2-F | $CH_3$ | $CH_3$ | S |
| 4-$CH_3CH_2CH(CH_3)SO_2$ | 2-Cl | $OCH_3$ | $CH_3$ | S |
| 4-$CH_3CH_2CH(CH_3)SO_2$ | 5-Br | $OCH_3$ | $OCH_3$ | S |
| 4-$(CH_3)_3CSO_2$ | 2-$CH_3$ | $CH_3$ | $CH_3$ | S |
| 4-$(CH_3)_3CSO_2$ | 2-$OCH_3$ | $C_2H_5$ | $OCH_3$ | S |
| 4-$CH_2{=}CHCH_2SO_2$ | 2-Cl | $OCH_3$ | $CH_3$ | S |
| 4-$CH_3CH_2CH{=}CHSO_2$ | H | $CH_3$ | $CH_3$ | S |
| 4-$CH_3CH_2CH{=}CHSO_2$ | H | $OCH_3$ | $CH_3$ | S |
| 4-$CH_3CH_2CH{=}CHSO_2$ | H | $OCH_3$ | $OCH_3$ | S |
| 4-$(CH_3)_2C{=}CHSO_2$ | H | $CH_2OCH_3$ | $CH_3$ | S |
| 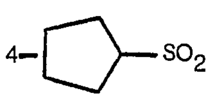 | 2-Br | $OC_2H_5$ | $CH_3$ | S |
| 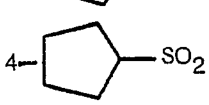 | 2-Cl | $OCH_3$ | $OCH_3$ | S |

TABLE III (continued)

| $R_1$ | $R_2$ | X | Y | W |
|---|---|---|---|---|
| 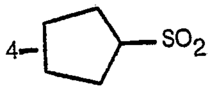 | 5-$OCH_3$ | $CH_3$ | $CH_3$ | S |
| 5-$CH_3SO_2$ | H | $CH_3$ | $CH_3$ | O |
| 5-$CH_3SO_2$ | H | $CH_3$ | $OCH_3$ | O |
| 5-$CH_3SO_2$ | H | $OCH_3$ | $OCH_3$ | O |
| 5-$CH_3SO_2$ | 2-F | $C_2H_5$ | $CH_3$ | O |
| 5-$CH_3SO_2$ | 2-$CH_3$ | $OCH_3$ | $OCH_3$ | O |
| 5-$C_2H_5SO_2$ | 2-Cl | $CH_2OCH_3$ | $CH_3$ | O |
| 5-$(CH_3)_2CHSO_2$ | 4-$OCH_3$ | $OC_2H_5$ | $OCH_3$ | O |
| 5-$C_2H_5CH(CH_3)SO_2$ | H | $OCH_3$ | $CH_3$ | O |
| 5-$CH_2$=$CHCH_2SO_2$ | H | $CH_3$ | $CH_3$ | S |
| 5-$CH_3CH$=$CHSO_2$ | H | $CH_2OCH_3$ | $CH_3$ | S |
| 5-$CH_2$=$CHCH_2CH_2SO_2$ | H | $OC_2H_5$ | $CH_3$ | S |
| 5-$CH_3CH$=$CHCH_2SO_2$ | 2-Cl | $CH_3$ | $CH_3$ | S |
| 5-$CH_3CH_2CH$=$CHSO_2$ | 2-Br | $CH_2OCH_3$ | $OCH_3$ | S |
| 5-$CH_2$=$C(CH_3)CH_2SO_2$ | 2-F | $CH_3$ | $OCH_3$ | S |
| 5-$(CH_3)_2C$=$CHSO_2$ | H | $OCH_3$ | $OCH_3$ | S |
| 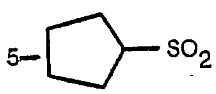 | 4-$NO_2$ | $OC_2H_5$ | $OCH_3$ | S |
| 2-$CH_3SO_2$ | 4-Cl | $CH_3$ | $CH_3$ | O |
| 2-$CH_3SO_2$ | 4-Br | $CH_3$ | $CH_3$ | O |
| 2-$CH_3SO_2$ | 4-F | $CH_3$ | $CH_3$ | O |
| 2-$CH_3SO_2$ | 4-$CH_3$ | $CH_3$ | $CH_3$ | O |
| 2-$CH_3SO_2$ | 4-$CH_3O$ | $CH_3$ | $CH_3$ | O |

TABLE IV

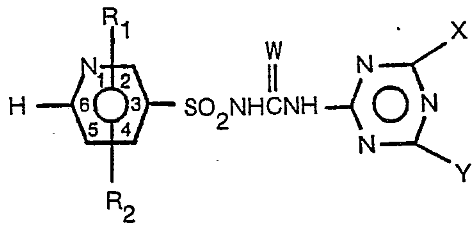

| $R_1$ | $R_2$ | X | Y | W |
|---|---|---|---|---|
| 2-$CH_3SO_2$ | H | $CH_3$ | $CH_3$ | O |
| 2-$CH_3SO_2$ | H | $CH_3$ | $OCH_3$ | O |
| 2-$CH_3SO_2$ | H | $OCH_3$ | $OCH_3$ | O |
| 2-$CH_3SO_2$ | H | $C_2H_5$ | $CH_3$ | O |
| 2-$CH_3SO_2$ | H | $C_2H_5$ | $OCH_3$ | O |
| 2-$CH_3SO_2$ | H | $OC_2H_5$ | $CH_3$ | O |
| 2-$CH_3SO_2$ | H | $OC_2H_5$ | $OCH_3$ | O |
| 2-$CH_3SO_2$ | H | $CH_2OCH_3$ | $CH_3$ | O |
| 2-$CH_3SO_2$ | H | $CH_2OCH_3$ | $OCH_3$ | O |
| 2-$C_2H_5SO_2$ | H | $CH_3$ | $CH_3$ | O |
| 2-$C_2H_5SO_2$ | H | $CH_3$ | $OCH_3$ | O |
| 2-$C_2H_5SO_2$ | H | $OCH_3$ | $OCH_3$ | O |
| 2-$C_2H_5SO_2$ | H | $C_2H_5$ | $CH_3$ | O |
| 2-$C_2H_5SO_2$ | H | $OC_2H_5$ | $OCH_3$ | O |
| 2-$C_2H_5SO_2$ | H | $CH_2OCH_3$ | $CH_3$ | O |
| 2-*n*-$C_3H_7SO_2$ | H | $CH_3$ | $CH_3$ | O |
| 2-*n*-$C_3H_7SO_2$ | H | $OCH_3$ | $CH_3$ | O |
| 2-*n*-$C_3H_7SO_2$ | H | $OCH_3$ | $OCH_3$ | O |
| 2-$(CH_3)_2CHSO_2$ | H | $CH_3$ | $CH_3$ | O |
| 2-$(CH_3)_2CHSO_2$ | H | $OCH_3$ | $CH_3$ | O |
| 2-$(CH_3)_2CHSO_2$ | H | $OCH_3$ | $OCH_3$ | O |
| 2-*n*-$C_4H_9SO_2$ | H | $CH_3$ | $CH_3$ | O |
| 2-*n*-$C_4H_9SO_2$ | H | $OCH_3$ | $CH_3$ | O |
| 2-*n*-$C_4H_9SO_2$ | H | $OCH_3$ | $OCH_3$ | O |
| 2-$(CH_3)_2CHCH_2SO_2$ | H | $CH_3$ | $CH_3$ | O |
| 2-$(CH_3)_2CHCH_2SO_2$ | H | $OCH_3$ | $CH_3$ | O |
| 2-$(CH_3)_2CHCH_2SO_2$ | H | $OCH_3$ | $OCH_3$ | O |

TABLE IV (continued)

| $R_1$ | $R_2$ | X | Y | W |
|---|---|---|---|---|
| $2\text{-}CH_3CH_2CH(CH_3)SO_2$ | H | $CH_3$ | $CH_3$ | O |
| $2\text{-}CH_3CH_2CH(CH_3)SO_2$ | H | $OCH_3$ | $CH_3$ | O |
| $2\text{-}CH_3CH_2CH(CH_3)SO_2$ | H | $OCH_3$ | $OCH_3$ | O |
| $2\text{-}(CH_3)_3CSO_2$ | H | $CH_3$ | $CH_3$ | O |
| $2\text{-}(CH_3)_3CSO_2$ | H | $OCH_3$ | $CH_3$ | O |
| $2\text{-}(CH_3)_3CSO_2$ | H | $OCH_3$ | $OCH_3$ | O |
| $2\text{-}CH_2{=}CHCH_2SO_2$ | H | $CH_3$ | $CH_3$ | O |
| $2\text{-}CH_3CH{=}CHSO_2$ | H | $OCH_3$ | $CH_3$ | O |
| $2\text{-}CH_2{=}C(CH_3)SO_2$ | H | $OCH_3$ | $OCH_3$ | O |
| $2\text{-}CH_2{=}CHCH_2CH_2SO_2$ | H | $CH_3$ | $CH_3$ | O |
| $2\text{-}CH_2{=}C(CH_3)CH_2SO_2$ | H | $OCH_3$ | $OCH_3$ | O |
| $2\text{-}CH_2{=}CHCH(CH_3)SO_2$ | H | $CH_3$ | $CH_3$ | O |
| $2\text{-}CH_3CH{=}CHCH_2SO_2$ | H | $OCH_3$ | $CH_3$ | O |
| $2\text{-}CH_3CH_2CH{=}CHSO_2$ | H | $CH_3$ | $CH_3$ | O |
| $2\text{-}(CH_3)_2C{=}CHSO_2$ | H | $OC_2H_5$ | $OCH_3$ | O |
| 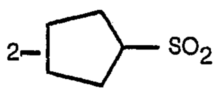 | H | $CH_3$ | $CH_3$ | O |
| 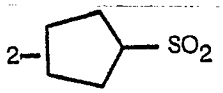 | H | $OCH_3$ | $CH_3$ | O |
| 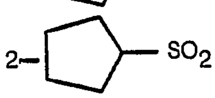 | H | $OCH_3$ | $OCH_3$ | O |
| $2\text{-}CH_3SO_2$ | 4-F | $CH_3$ | $CH_3$ | O |
| $2\text{-}CH_3SO_2$ | 4-Cl | $OCH_3$ | $CH_3$ | O |
| $2\text{-}CH_3SO_2$ | 4-Br | $CH_3$ | $CH_3$ | O |
| $2\text{-}CH_3SO_2$ | $4\text{-}CH_3$ | $OCH_3$ | $OCH_3$ | S |
| $2\text{-}CH_3SO_2$ | 5-Cl | $CH_2OCH_3$ | $CH_3$ | S |
| $2\text{-}CH_3SO_2$ | 5-Br | $CH_3$ | $CH_3$ | S |
| $2\text{-}CH_3SO_2$ | 5-F | $OCH_3$ | $OCH_3$ | S |
| $2\text{-}CH_3SO_2$ | $5\text{-}CH_3$ | $CH_3$ | $CH_3$ | S |
| $2\text{-}CH_3SO_2$ | $5\text{-}OCH_3$ | $OCH_3$ | $OCH_3$ | S |
| $4\text{-}CH_3SO_2$ | H | $CH_3$ | $CH_3$ | O |
| $4\text{-}CH_3SO_2$ | H | $OCH_3$ | $CH_3$ | O |

TABLE IV (continued)

| $R_1$ | $R_2$ | X | Y | W |
|---|---|---|---|---|
| 4-$CH_3SO_2$ | H | $OCH_3$ | $OCH_3$ | O |
| 4-$C_2H_5SO_2$ | H | $CH_3$ | $CH_3$ | O |
| 4-$C_2H_5SO_2$ | H | $OCH_3$ | $CH_3$ | O |
| 4-$C_2H_5SO_2$ | H | $OCH_3$ | $OCH_3$ | O |
| 4-*n*-$C_3H_7SO_2$ | H | $CH_3$ | $CH_3$ | O |
| 4-*n*-$C_3H_7SO_2$ | H | $OCH_3$ | $CH_3$ | O |
| 4-*n*-$C_3H_7SO_2$ | H | $OCH_3$ | $OCH_3$ | O |
| 4-$(CH_3)_2CHSO_2$ | H | $CH_3$ | $CH_3$ | O |
| 4-$(CH_3)_2CHSO_2$ | H | $OCH_3$ | $CH_3$ | O |
| 4-$(CH_3)_2CHSO_2$ | H | $OCH_3$ | $OCH_3$ | O |
| 4-*n*-$C_4H_9SO_2$ | H | $CH_3$ | $CH_3$ | O |
| 4-*n*-$C_4H_9SO_2$ | H | $OCH_3$ | $CH_3$ | O |
| 4-*n*-$C_4H_9SO_2$ | H | $OCH_3$ | $OCH_3$ | O |
| 4-$(CH_3)_2CHCH_2SO_2$ | H | $C_2H_5$ | $CH_3$ | O |
| 4-$CH_3CH_2CH(CH_3)SO_2$ | H | $OC_2H_5$ | $OCH_3$ | O |
| 4-$(CH_3)_3CSO_2$ | H | $CH_2OCH_3$ | $CH_3$ | O |
| 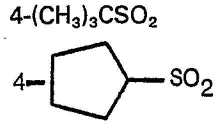 | H | $CH_3$ | $CH_3$ | O |
| 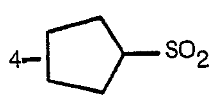 | H | $OCH_3$ | $CH_3$ | O |
| 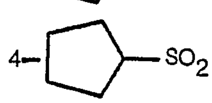 | H | $OCH_3$ | $OCH_3$ | O |
| 4-$CH_3SO_2$ | 2-Cl | $CH_3$ | $CH_3$ | S |
| 4-$CH_3SO_2$ | 2-$CH_3$ | $CH_3$ | $OCH_3$ | S |
| 4-$C_2H_5SO_2$ | 2-$OCH_3$ | $CH_3$ | $OCH_3$ | S |
| 4-*n*-$C_3H_7SO_2$ | 5-Br | $CH_3$ | $CH_3$ | S |
| 4-*n*-$C_3H_7SO_2$ | 5-F | $OCH_3$ | $OCH_3$ | S |
| 4-*n*-$C_4H_9SO_2$ | 5-Cl | $C_2H_5$ | $OCH_3$ | S |
| 5-$CH_3SO_2$ | H | $CH_3$ | $CH_3$ | S |
| 5-$CH_3SO_2$ | H | $OCH_3$ | $CH_3$ | S |
| 5-$CH_3SO_2$ | H | $OCH_3$ | $OCH_3$ | S |

TABLE IV (continued)

| $R_1$ | $R_2$ | X | Y | W |
|---|---|---|---|---|
| 5-$C_2H_5SO_2$ | H | $CH_3$ | $CH_3$ | S |
| 5-$C_2H_5SO_2$ | H | $OCH_3$ | $CH_3$ | S |
| 5-$C_2H_5SO_2$ | H | $OCH_3$ | $OCH_3$ | S |
| 5-$C_3H_7SO_2$ | H | $CH_3$ | $CH_3$ | O |
| 5-$C_3H_7SO_2$ | H | $OCH_3$ | $CH_3$ | O |
| 5-$C_3H_7SO_2$ | H | $OCH_3$ | $OCH_3$ | O |
| 5-$(CH_3)_2CHSO_2$ | H | $CH_3$ | $CH_3$ | O |
| 5-$(CH_3)_2CHSO_2$ | H | $OCH_3$ | $CH_3$ | O |
| 5-$(CH_3)_2CHSO_2$ | H | $OCH_3$ | $OCH_3$ | O |
| 5-*n*-$C_4H_9SO_2$ | H | $CH_3$ | $CH_3$ | O |
| 5-*n*-$C_4H_9SO_2$ | H | $OCH_3$ | $CH_3$ | O |
| 5-*n*-$C_4H_9SO_2$ | H | $OCH_3$ | $OCH_3$ | O |
| 5-$(CH_3)_2CH_2CH_2SO_2$ | H | $C_2H_5$ | $OCH_3$ | O |
| 5-$CH_3CH_2CH(CH_3)SO_2$ | H | $CH_2OCH_3$ | $CH_3$ | O |
| 5-$(CH_3)_3CSO_2$ | H | $CH_2OCH_3$ | $OCH_3$ | O |
| 5-$CH_2{=}CHCH_2SO_2$ | H | $CH_3$ | $CH_3$ | O |
| 5-$CH_3CH{=}CHSO_2$ | H | $OCH_3$ | $OCH_3$ | O |
| 5-$CH_2{=}CHCH_2CH_2SO_2$ | H | $C_2H_5$ | $CH_3$ | O |
| 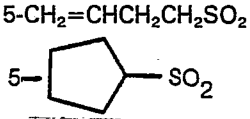 | H | $CH_3$ | $CH_3$ | O |
| 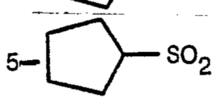 | H | $OCH_3$ | $CH_3$ | O |
| 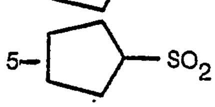 | H | $OCH_3$ | $OCH_3$ | O |
| 5-$C_2H_5SO_2$ | 2-Br | $CH_3$ | $CH_3$ | O |
| 5-$C_2H_5SO_2$ | 4-Cl | $OCH_3$ | $OCH_3$ | O |
| 5-*n*-$C_4H_9SO_2$ | 4-$CH_3$ | $CH_3$ | $CH_3$ | O |
| 5-$(CH_3)_3CSO_2$ | 4-$OCH_3$ | $CH_3$ | $OCH_3$ | S |
| 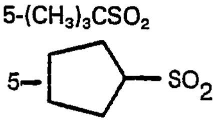 | 2-$CH_3$ | $OCH_3$ | $OCH_3$ | S |

*Formulations*

Useful formulations of the compounds of Formula (I) can be prepared in conventional ways. They include dusts, granules, pellets, solutions, suspensions, emulsions, wettable powders and the like. Many of these may be applied directly. Sprayable formulations can be extended in suitable media and used at spray volumes of from a few litres to several hundred liters per hectare. High-strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid of liquid diluent(s). More specifically, they will contain these ingredients in the following approximate proportions:

TABLE V

| | Active Ingredient | Weight Percent* Diluent(s) | Surfactant(s) |
|---|---|---|---|
| Wettable Powders | 20—90 | 0—74 | 1—10 |
| Oil Suspensions, Emulsions, Solutions (including Emulsifiable, Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High-strength Compositions | 90—99 | 0—10 | 0—2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recomended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer- or fluid energy-mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering,* December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley & Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 8

*Wettable Powder*

| | |
|---|---|
| N-[(4-methoxy-6-methylpyrimidin-2-yl)aminocarbonyl]-[(1-methylethyl)sulfonyl]benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 μm and then reblended.

Example 9

*Wettable Powder*

| | |
|---|---|
| N-[(4-methoxy-6-methyl-1,3,5-triazin-2-yl)aminocarbonyl]-2-[(1-methylethyl)sulfonyl]benzenesulfonamide | 50% |
| sodium alkylnaphthalenesulfonate | 2% |
| low-viscosity methyl cellulose | 2% |
| diatomaceous earth | 46% |

The ingredients are blended, coarsely hammer-milled and then air-milled to produce particles essentially all below 10 μm in diameter. The product is reblended before packaging.

Example 10

*Granule*

| | |
|---|---|
| Wettable Powder of Example 9 | 5% |
| attapulgite granules (U.S.S. 20—40 mesh; 0.84—0.42 mm) | 95% |

A slurry of wettable powder containing 25% solids is sprayed on the surface of attapulgite granules in a double-cone blender. The granules are dried and packaged.

Example 11

*Extruded Pellet*

| | |
|---|---|
| N-[(4,6-dimethylpyrimidin-2-yl)aminocarbonyl]-2-[(1-methylethyl)sulfonyl]benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packed for use and the finest recycled.

Example 12

*Oil Suspension*

| | |
|---|---|
| N-[(4,6-dimethoxylpyrimidin-2-yl)aminocarbonyl]-2-(propylsulfonyl)-benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 µm. The resulting thick suspension may be applied directly, but preferably after being extended with oils or emulsified in water.

Example 13

*Wettable Powder*

| | |
|---|---|
| N-[(4,6-dimethoxy-1,3,5-triazin-2-yl)aminocarbonyl]-2-(propylsulfonyl)benzenesulfonamide | 20% |
| sodium alkylnaphthalenesulfonate | 4% |
| sodium ligninsulfonate | 4% |
| low-viscosity methyl cellulose | 3% |
| attapulgite | 69% |

The ingredients are thoroughly blended. After grinding in a hammer-mill to produce particles essentially all below 100 µm, the material is reblended and sifted through a U.S.S. No. 50 sieve (0.3 mm opening) and packaged.

Example 14

*Low-Strength Granule*

| | |
|---|---|
| N-[(4-methoxy-6-methyl-1,3,5-triazin-2-yl)aminocarbonyl]-2-(propylsulfonyl)benzenesulfonamide | 1% |
| N,N-dimethylformamide | 9% |
| attapulgite granules (U.S.S. 20—40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 15

*Aqueous Suspension*

| | |
|---|---|
| N-[(4,6-dimethyl-1,3,5-triazin-2-yl)aminocarbonyl]-2-(propylsulfonyl)benzenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polvyinyl alcohol | 1.0% |
| water | 56.7% |

The ingredients are blended and ground together in a sand mill to produce artificial particles essentially all under 5 μm in size.

Example 16

*Solution*

| | |
|---|---|
| N-[(4-methoxy-6-methyl-1,3,5-triazin-2-yl)aminocarbonyl]-2-[(1-methylethyl)sulfonyl]benzenesulfonamide, sodium salt | 5% |
| water | 95% |

The salt is added directly to the water with stirring to produce the solution, which may then be packaged for use.

Example 17

*Low Strength Granule*

| | |
|---|---|
| N-[(4-methoxy-6-methylpyrimidin-2-yl)aminocarbonyl]-2-(propylsulfonyl)benzenesulfonamide | 0.1% |
| attapulgite granules (U.S.S. 20—40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 18

*Granule*

| | |
|---|---|
| N-[(4,6-dimethylpyrimidin-2-yl)aminocarbonyl]-2-(propylsulfonyl)benzenesulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh sceen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 μm), and packaged for use.

Example 19

*High Strength Control*

| | |
|---|---|
| N-[(4-methoxy-6-methylpyrimidin-2-yl)aminocarbonyl]-2-[(1-methylethyl)sulfonyl]benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 20

*Wettable Powder*

| | |
|---|---|
| N-[(4,6-dimethyl-1,3,5-triazin-2-yl)aminocarbonyl]-2-[(1-methylethyl)sulfonyl]benzenesulfonamide | 90% |
| dioctyl sodium sulfosuccinate | 0.1% |
| synthetic fine silica | 9.9% |

The ingredients are blended and ground in a hammer-mill to produce particles essentially all below 100 µm. The material is sifted through a U.S.S. No. 50 screen and then packaged.

Example 21

*Wettable Powder*

| | |
|---|---|
| N-[(4,6-dimethylpyrimidin-2-yl)aminocarbonyl]-2-[(1-methylethyl)sulfonyl]benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 µm in size. The material is reblended and then packaged.

Example 22

*Oil Suspension*

| | |
|---|---|
| N-[(4,6-dimethoxy-1,3,5-triazin-2-yl)aminocarbonyl]-2-[(1-methylethyl)sulfonyl]benzenesulfonamide | 35% |
| blend of polyalcohol carboxylic esters and oil-soluble petroleum sulfonates | 6% |
| xylene | 59% |

The ingredients are combined and ground together in a sand mill to produce particles essentially all below 5 µm. The product can be used directly, extended with oils, or emulsified in water.

Example 23

*Dust*

| | |
|---|---|
| N-[(4-methoxy-6-methyl-1,3,5-triazin-2-yl)aminocarbonyl]-2-[(1-methylethyl)sulfonyl]benzenesulfonamide | 10% |
| attapulgite | 10% |
| Pyrophyllite | 80% |

The active ingredient is blended with attapulgite and then passed through a hammer-mill to produce particles substantially all below 200 µm. The ground concentrate is then blended with powdered pyrophyllite until homogeneous.

Example 24

*Wettable Powder*

| | |
|---|---|
| N-[(4,6-Dimethylpyrimidin-2-yl)aminocarbonyl]-2-(methylsulfonyl)-3-pyridinesulfonamide | 95% |
| dioctyl sodium sulfosuccinate | 0.1% |
| sodium ligninsulfonate | 1% |
| synthetic fine silica | 3.9% |

The ingredients are blended and ground in a hammer-mill to produce particles almost all of which are below 10 µm in size. That material is sifted through a U.S.S. No. 50 screen and packaged.

Example 25

*Wettable Powder*

| | |
|---|---|
| N-[(4,6-Dimethylpyrimidin-2-yl)aminocarbonyl]-2-(methylsulfonyl)-3-pyridinesulfonamide | 40% |
| dioctyl sodium sulfosuccinate | 1.5% |
| sodium ligninsulfonate | 3% |
| low-viscosity methyl cellulose | 1.5% |
| attapulgite | 54% |

The ingredients are thoroughly blended and passed through an air mill to produce an average particle size under 15 µm, reblended, and sifted through a U.S.S. No. 50 sieve (0.3 mm opening) before packaging.

Example 26

*Granule*

| | |
|---|---|
| wettable powder of Example 25 | 25% |
| gypsum | 64% |
| potassium sulfate | 11% |

The ingredients are blended in a rotating mixer, and water is sprayed onto that blend so as to effect granulation. When most of the granules have reached 1.0 to 0.42 mm (U.S.S. #18 to 40 sieves) in size, they are removed, dried, and screened. Oversize material is crushed to produce additional material in the desired range. The resulting granules contain 10% of the active ingredient.

Example 27

*Wettable Powder*

| | |
|---|---|
| N-[(4,6-Dimethylpyrimidin-2-yl)aminocarbonyl]-2-(methylsulfonyl)-3-pyridinesulfonamide | 65% |
| dodecylphenyl polyethylene glycol ether | 2% |
| sodium ligninsulfonate | 4% |
| sodium silicoaluminate | 6% |
| montmorillonite (calcined) | 23% |

The ingredients are thoroughly blended. The liquid surfactant is added by spraying on the solid ingredients in a blender. After grinding in a hammer-mill to produce particles almost all of which are below 100 µm in size, the material is reblended, sifted through a U.S.S. #50 sieve (0.3 mm opening) and packaged.

Example 28

*Oil Suspension*

| | |
|---|---|
| N-[(4,6-Dimethylpyrimidin-2-yl)aminocarbonyl]-2-(methylsulfonyl)-3-pyridinesulfonamide | 25% |
| polyoxyethylene sorbitol hexaoleate | 5% |
| highly aliphatic hydrocarbon oil | 70% |

The ingredients are ground together in a sand mill until the solid particles have been reduced to under 5 μm. The resulting suspension may be applied directly, but preferably after being extended further with oils or emulsified in water.

Example 29

*Aqueous Suspension*

| | |
|---|---|
| N-[(4,6-Dimethylpyrimidin-2-yl)aminocarbonyl]-2-(methylsulfonyl)-3-pyridinesulfonamide | 25% |
| hydrated attapulgite | 3% |
| crude calcium ligninsulfonate | 10% |
| sodium dihydrogen phosphate | 0.5% |
| Water | 61.5% |

The ingredients are ground together in a ball or roller mill until the solid particles have been reduced to sizes under 10 μm, and then packaged.

Example 30

*Extruded Pellet*

| | |
|---|---|
| N-[(4,6-Dimethylpyrimidin-2-yl)aminocarbonyl]-2-(methylsulfonyl)-3-pyridinesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer milled and then moistened with about 12% water. The mixture is extruded in the form of cylinders about 3 mm in diameter which are cut to produce pellets of about 3 mm long. The pellets may be used directly, after drying, or dried pellets may be crushed to pass a U.S.S. No. 20 sieve (0.84 mm openings). The granules held on a U.S.S. No. 40 sieve (0.42 mm openings) may be packaged for use and the fines recycled.

Example 31

*Solution*

| | |
|---|---|
| N-[(4,6-Dimethylpyrimidin-2-yl)aminocarbonyl]-2-(methylsulfonyl)-3-pyridinesulfonamide | 5% |
| dimethylformamide | 95% |

The ingredients are combined and stirred to produce a solution, which can be used for low-volume applications.

Example 32

*Wettable Powder*

| | |
|---|---|
| N-[(4,6-Dimethylpyrimidin-2-yl)aminocarbonyl]-2-(methylsulfonyl)-3-pyridinesulfonamide | 80% |
| sodium alkylnaphthalenesulfonate | 2% |
| sodium ligninsulfonate | 2% |
| synthetic amorphous silica | 3% |
| kaolinite | 13% |

The ingredients are thoroughly blended after grinding in a hammer mill to produce particles essentially all of which are under 100 µm in size; the material is reblended, sifted through a U.S.S. No. 50 sieve and packaged.

## Example 33

*Granule*

| | |
|---|---|
| N-[(4,6-Dimethylpyrimidin-2-yl)aminocarbonyl]-2-(methylsulfonyl)-3-pyridinesulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

The compounds of Formula I can be formulated using the procedures of Examples 8—33.

*Utility*

The compounds of the present invention are active herbicides. They have utility for broad-spectrum pre- and/or post-emergence weed control in areas where complete control of all vegetation is desired, such as around fuel storage tanks, ammunition depots, industrial storage areas, MX missile sites, parking lots, drive-in theaters, around billboards, highway and railroad structures. In addition, the subject compounds distinguish themselves in being highly useful for the selective pre- or post-emergence weed control in a wide range of crops, such as soybeans, dry beans, green beans, alfalfa, tobacco, corn, cotton, wheat, barley, cucumbers, tomatoes, flax and potatoes. By properly selecting rate and time of application, compounds of this invention may be used also to modify plant growth beneficially.

The rates of application for the compounds of the invention are determined by a number of factors, including their use as selective or general herbicides, the crop species involved, the types of weeds to be controlled, weather and climate, formulations selected, mode of application, amount of foliage present, etc. In general terms, the subject compounds should be applied at levels of around 0.01 to 10 kg/ha, the lower rates being suggested for use on lighter soils and/or those having a low organic matter content, for selective weed control or for situations where only short-term persistence is required.

The compounds of this invention may be used in combination with other commercial herbicides. They are particularly useful in combination with the following herbicides.

| *Common Name* | *Chemical Name* |
|---|---|
| acifluorfen | 5-[2-chloro-4-(trifluoromethyl)phenoxy]-2-nitrobenzoic acid |
| alachlor | 2-chloro-2′,6′-diethyl-N-(methoxymethyl)acetanilide |
| amitrole | 3-amino-*s*-triazole |
| atrazine | 2-chloro-4-(ethylamino)-6-(isopropylamino)-*s*-triazine |
| barban | 4-chloro-2-butynyl *m*-chlorocarbanilate |
| bentazon | 3-isopropyl-1H-2,1,3-benzothiadiazin-4(3H)-one-2,2-dioxide |
| benzoylprop | N-benzoyl-N-(3,4-dichlorophenyl)-DL-alaine |
| bromacil | 5-bromo-3-*sec*-butyl-6-methyluracil |
| bromoxynil | 3,5-dibromo-4-hydroxybenzonitrile |
| butylate | S-ethyl-diisobutylthiocarbamate |
| cyanazine | 2-[(4-chloro-6-(ethylamino)-*s*-triazin-2-yl]amino]-2-methylpropionitrile |
| diallate | S-(2,3-dichloroallyl)diisopropylthiocarbamate |
| dicamba | 3,6-dichloro-*o*-anisic acid |
| dichloroprop | 2-(2,4-dichlorophenoxy)propionic acid |
| diclofop | 2-[4-(2,4-dichlorophenoxy)phenoxy]propanoic acid |
| difenzoquat | 1,2-dimethyl-3,5-diphenyl-1H-pyrazolion |
| dinoseb | 2-*sec*-butyl-4,6-dinitrophenol |
| diuron | 3-(3,4-dichlorophenyl)-1,1-dimethylurea |
| EPTC | S-ethyl-dipropylthiocarbamate |
| flamprop | N-benzoyl-N-(3-chloro-4-fluorophenyl)-DL-alanine |

| | |
|---|---|
| fluometuron | 1,1-dimethyl-3-(α,α,α-trifluoro-*m*-tolyl)-urea |
| fosamine | ethyl hydrogen (aminocarbonyl)phosphonate |
| glyphosphate | N-(phosphonomethyl)glycine |
| hexazinone | 3-cyclohexyl-6-(dimethylamino)-1-methyl-1,3,5-triazine-2,4(1H,3H)-dione |
| ioxynil | 4-hydroxy-3,5-diiodobenzonitrile |
| lenacil | 3-cyclohexyl-6,7-dihydro-1H-cyclopentapyrimidine-2,4(3H,5H)-dione |
| linuron | 3-(3,4-dichlorophenyl)-1-methoxy-1-methylurea |
| MCPA | [(4-chloro-*o*-tolyl)oxy]acetic acid |
| MCPB | 4-[(4-chloro-*o*-tolyl)oxy]propionic acid |
| metolachlor | 2-chloro-N-(2-ethyl-6-methylphenyl)-N-(2-methoxy-1-methylethyl)acetamide |
| metribuzin | 4-amino-6-*tert*-butyl-3-(methylthio)-as-triazin-5(4H)-one |
| MSMA | monosodium methanearsonate |
| neburon | 1-butyl-3-(3,4-dichlorophenyl)-1-methylurea |
| paraquat | 1,1′-dimethyl-4,4′-bipyridinium ion |
| propanil | 3′,4′-dichloropropionalide |
| siduron | 1-(2-methylcyclohexyl)-3-phenylurea |
| terbacil | 3-*tert*-butyl-5-chloro-6-methyluracil |
| triallate | S-(2,3,3-trichloroallyl)diisopropylthiocarbamate |
| trifluralin | α,α,α-trifluoro-2,6-dinitro-N,N-dipropyl-*p*-toluidine |
| 2,4-D | (2,4-dichlorophenoxy)acetic acid |
| 2,4-DB | 4-(2,4-dichlorophenoxy)butyric acid 3,4-diaryl-4-cyanobutyrates |
| methabenzthiazuron | 1,3-dimethyl-3-(2-benzothiazolyl)urea |
| chlortoluron | N′-(3-chloro-4-methylphenyl-N′,N′-dimethylurea |
| isoproturon | N-(4-isopropylphenyl)-N′,N′-dimethylurea |
| methoxuran | N′-(3-chloro-4-methoxyphenyl)N,N-dimethylurea |

The activity of these compounds was discovered in a number of greenhouse and field tests. The tests are described and the data resulting from them are shown below.

O = no effect
1O = maximum effort
C = chlorosis or necrosis
D = defoliation
E = emergence inhibition
G = growth retardation
H = formative effects
U = unusual pigmentation
X = axillary stimulation
5F = ealier flowering
6Y = abscised buds or flowers.

*Test A*

Seeds of crabtree grass (*Digitaria* spp.), barnyardgrass (*Echinochloa crusgalli)*, wild oats (*Avena fatua)*, cassia (*Cassia tora)*, morningglory (*Ipomoea* spp.), cocklebur (*Xanthium* spp.), sorghum, corn, soybean, rice, wheat and nutsedge tubers (*Cyperus rotundus)* were planted in a growth medium and treated pre-emergence with a non-phytotoxic solvent solution of the compounds of Table A. At the same time, cotton having five leaves (including cotyledonary ones), bush beans with the third trifoliate leaf expanding, crabgrass, barnyardgrass and wild oats with two leaves, cassia with three leaves (including cotyledonary ones), morningglory and cocklebur with four leaves (including the cotyledonary ones), sorghum and corn with four leaves, soybean with two cotyledonary leaves, rice with three leaves, wheat with one leaf, and nutsedge with three-five leaves were sprayed with a non-phytotoxic solvent solution of the compounds of Table A. Other containers of the above-mentioned weeds and crops were treated pre- or post-emergence with the same non-phytotoxic solvent so as to provide a solvent control. A set of untreated control plants was also included for comparison. Pre-emergence and post-emergence treated plants and controls were maintained in a greenhouse for sixteen days, then all treated plants were compared with their respective controls and rated visually for response to teatment. The data in Table A shows that compounds of this invention are very effective as herbicides and may have utility for selective post-emergence weed control in wheat.

COMPOUND No. 1

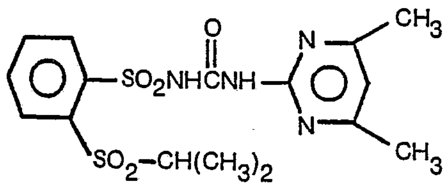

COMPOUND No. 2

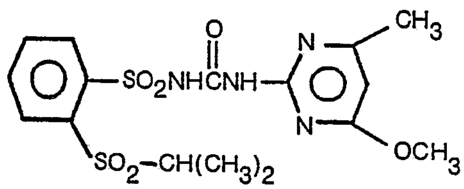

COMPOUND No. 3

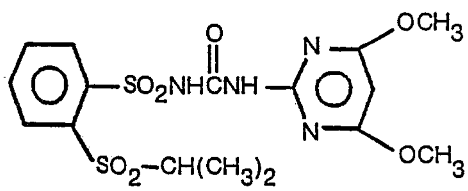

COMPOUND No. 4

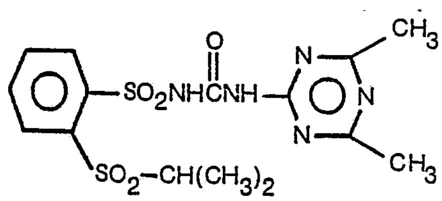

COMPOUND No. 5

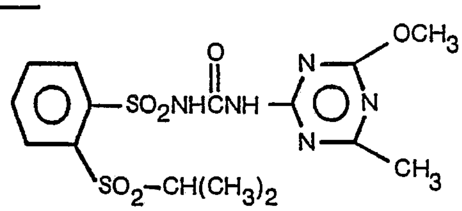

COMPOUND No. 6

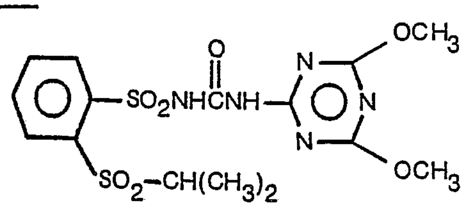

COMPOUND No. 7

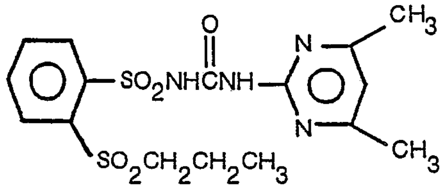

COMPOUND No. 8

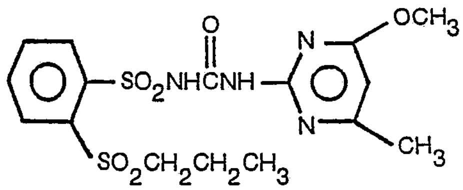

COMPOUND No. 9

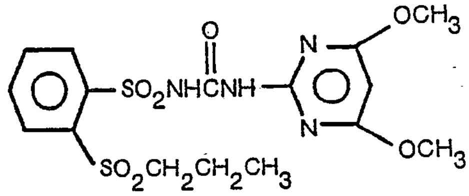

COMPOUND No. 10

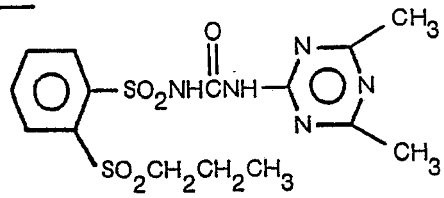

COMPOUND No. 11

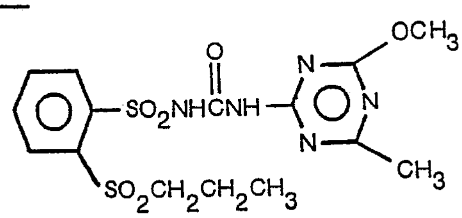

COMPOUND No. 12

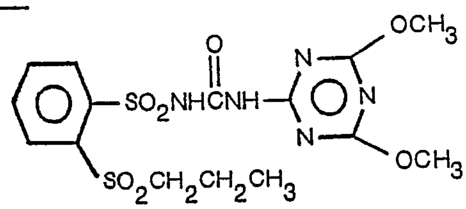

TABLE A

| | Compound No. | | |
|---|---|---|---|
| | 1 | 2 | 3 |
| kg/ha | 0.1 | 0.1 | 0.1 |
| POST-EMERGENCE | | | |
| Bushbean | 2C, 5G, 6Y | 5C, 8G, 6Y | 9C |
| Cotton | 3C, 3H, 6G | 4C, 7G | 5C, 9G |
| Morningglory | 2C, 5G, 5F | 10C | 1C, 2H |
| Cocklebur | 2C, 5G | 9C | 9C |
| Cassia | 3C | 5C, 8G | 4C, 8G |
| Nutsedge | 2G | 5G | 7G |
| Crabgrass | 2G | 2G | 2G |
| Barnyardgrass | 2C, 5H | 9C | 8H |
| Wild Oats | 2C, 6G | 2C, 5G | 1C |
| Wheat | 1C, 2G | 2C, 3G | 1C |
| Corn | 2C | 1C, 3G | 1C, 5H |
| Soybean | 2C, 6G | 5C, 9G | 2C, 8G |
| Rice | – | – | – |
| Sorghum | 3C, 9H | 3C, 9G | 2C, 9G |
| PRE-EMERGENCE | | | |
| Morningglory | 8G | 9G | 9G |
| Cocklebur | 5H | 9H | 9H |
| Cassia | 1C | 8G | 8G |
| Nutsedge | 0 | 10E | 10E |
| Crabgrass | 2G | 5G | 1C, 8G |
| Barnyardgrass | 1C, 3G | 9H | 9H |
| Wild Oats | 1C, 2G | 2C, 8H | 7G |
| Wheat | 1C | 1C, 5G | 4G |
| Corn | 3G | 1C, 7G | 1C, 9H |
| Soybean | 0 | 8H | 7H |
| Rice | 2C, 6H | 9H | 10E |
| Sorghum | 1C, 8G | 5C, 9H | 2C, 9H |

TABLE A (Continued)

| | Compound No. 4 | 5 | 6 |
|---|---|---|---|
| kg/ha | 0.1 | 0.1 | 0.1 |
| POST-EMERGENCE | | | |
| Bushbean | 3C, 6Y | 9C | 9C |
| Cotton | 3C, 3H | 6C, 8G | 6C, 9G |
| Morningglory | 2C, 4G, 5F | 6C, 9G | 10C |
| Cocklebur | 2C, 2H | 5C, 9G | 9C |
| Cassia | 1C | 2C, 5G | 5C, 8G |
| Nutsedge | 0 | 1C | 5G |
| Crabgrass | 1C, 2G | 1C, 5G | 2C, 8G |
| Barnyardgrass | 9C | 5C, 9H | 2C, 9H |
| Wild Oats | 2C, 3G | 2C | 1C, 2G |
| Wheat | 1C | 1C, 2G | 1G |
| Corn | 1C, 5H | 4C, 9H | 9C |
| Soybean | 2C | 5C, 9G | 6C, 9G |
| Rice | – | – | – |
| Sorghum | 1C, 9G | 2C, 9G | 4C, 9G |
| PRE-EMERGENCE | | | |
| Morningglory | 7G | 9C, 9G | 9C, 9G |
| Cocklebur | 4G | 9H | 9H |
| Cassia | 1C | 2C, 9G | 2C, 9G |
| Nutsedge | 1C, 5G | 9G | 10E |
| Crabgrass | 2C | 2C, 8G | 2C, 8G |
| Barnyardgrass | 2C, 4G | 2C, 9H | 3C, 9H |
| Wild Oats | 2C | 2C, 7G | 1C, 2G |
| Wheat | 0 | 1C, 5G | 5G |
| Corn | 1C, 6G | 1U, 9G | 1U, 9G |
| Soybean | 0 | 8H | 8H |
| Rice | 9H | 9H | 9H |
| Sorghum | 1C, 9G | 5C, 9H | 3C, 9H |

TABLE A (Continued)

| | Compound No. | | |
|---|---|---|---|
| | 7 | 8 | 9 |
| kg/ha | 0.4 | 0.4 | 0.4 |
| POST-EMERGENCE | | | |
| Bushbean | 9C | 9C | 9C |
| Cotton | 2U, 6C, 9G | 9C | 9C |
| Morningglory | 10C | 10C | 10C |
| Cocklebur | 9C | 10C | 9C |
| Cassia | 4C, 8G | 9C | 9C |
| Nutsedge | 1C, 8G | 1C, 9G | 5C, 9G |
| Crabgrass | 3C, 9G | 5C, 9G | 7C, 9G |
| Barnyardgrass | 9C | 9C | 9C |
| Wild Oats | 9C | 9C | 5C, 9H |
| Wheat | 3C, 9G | 3C, 9G | 3C, 9G |
| Corn | 2U, 9H | 7U, 9G | 3U, 9H |
| Soybean | 9C | 9C | 9C |
| Rice | 9C | 9C | 8C |
| Sorghum | 9C | 10C | 9C |
| PRE-EMERGENCE | | | |
| Morningglory | 9C | 9H | 9H |
| Cocklebur | 9H | 10E | 9H |
| Cassia | 2C, 9G | 2C, 9G | 2C, 9G |
| Nutsedge | 10E | 10E | 10E |
| Crabgrass | 1C, 5G | 5C, 9G | 5C, 9H |
| Barnyardgrass | 3C, 9H | 5C, 9H | 5C, 9H |
| Wild Oats | 4C, 9H | 5C, 9H | 5C, 9H |
| Wheat | 1C, 9G | 2C, 9G | 3C, 9H |
| Corn | 2C, 9G | 9H | 9G |
| Soybean | 9H | 9H | 9H |
| Rice | 10E | 10E | 10E |
| Sorghum | 3C, 9H | 6C, 9H | 10H |

TABLE A (Continued)

| | Compound No. 10 | 11 | 12 |
|---|---|---|---|
| kg/ha | 0.4 | 0.4 | 0.4 |
| POST-EMERGENCE | | | |
| Bushbean | 9C | 9C | 5C, 9G |
| Cotton | 2U, 5C, 9G | 9C | 9C |
| Morningglory | 9C | 10C | 10C |
| Cocklebur | 9C | 9C | 10C |
| Cassia | 5C, 8G | 9C | 9C |
| Nutsedge | 4G | 6G | 1C, 6G |
| Crabgrass | 2C, 8G | 6C, 9G | 4C, 9G |
| Barnyardgrass | 2C, 9H | 9C | 9C |
| Wild Oats | 5C, 8G | 6C, 9G | 3C, 7H |
| Wheat | 1C, 7G, 5X | 2C, 9G, 5X | 1C, 6G, 5X |
| Corn | 2U, 9G | 8U, 9G | 6U, 9C |
| Soybean | 2C, 8G, 5X | 9C | 9C |
| Rice | 5C, 9G | 7C, 9G | 9C |
| Sorghum | 9C | 9C | 4U, 9C |
| PRE-EMERGENCE | | | |
| Morningglory | 9G | 9H | 9H |
| Cocklebur | 9H | 9H | 9H |
| Cassia | 6C, 9G | 2C, 9G | 1C, 9G |
| Nutsedge | 4G | 2C, 8G | 8G |
| Crabgrass | 2G | 4C, 9G | 2C, 8G |
| Barnyardgrass | 4C, 9H | 5C, 9H | 4C, 9H |
| Wild Oats | 2C, 8G | 5C, 9H | 5C, 9H |
| Wheat | 1C, 8G | 3C, 9G | 1C, 9G |
| Corn | 9H | 10E | 10E |
| Soybean | 1C, 2G | 9H | 2C, 9H |
| Rice | 10E | 10E | 10E |
| Sorghum | 3C, 9H | 10H | 6C, 9H |

TABLE A (Continued

| | 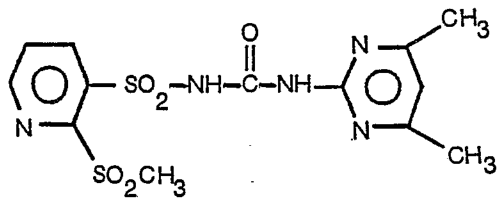 |
|---|---|
| kg/ha | 0.4 |
| POST-EMERGENCE | |
| Bushbean | 5C, 9G, 6Y |
| Cotton | 9C |
| Morningglory | 10C |
| Cocklebur | 9C |
| Cassia | 5C, 8G |
| Nutsedge | 8G |
| Crabgrass | 9C |
| Barnyardgrass | 7C, 9H |
| Wild Oats | 7C |
| Wheat | 5C, 8G |
| Corn | 5U, 9G |
| Soybean | 9C |
| Rice | 5C, 7G |
| Sorghum | 9C |
| PRE-EMERGENCE | |
| Morningglory | 9G |
| Cocklebur | 9H |
| Cassia | 9G |
| Nutsedge | 10E |
| Crabgrass | 2C, 9G |
| Barnyardgrass | 9H |
| Wild Oats | 3C, 8H |
| Wheat | 9H |
| Corn | 9H |
| Soybean | 9H |
| Rice | 10E |
| Sorghum | 5C, 9H |

*Test B*

Two plastic bulb pans were filled with fertilized and limed Fallsington silt loam soil. One pan was planted with corn, sorghum, Kentucky bluegrass and several grassy weeds. The other pan was planted with cotton, soybeans, purple nutsedge (*Cyperus rotundus*), and several broadleaf weeds. The following grassy and broadleaf weeds were planted: crabgrass (*Digitaria sanguinalis*), barnyard grass (*Echinochloa crusgalli*), wild oats (*Avena fatua*), johnsongrass (*Sorghum halepense*), dallisgrass (*Paspalum dilatatum*), giant foxtail (*Setaria faberii*), cheatgrass (*Bromus secalinus*), mustard (*Brassica arvensis*), cocklebur (*Xanthium pensylvanicum*), pigweed (*Amaranthus retroflexus*), morningglory (*Ipomoea hederaces*), cassia (*Cassia tora*), teaweed (*Side spinosa*), velvetleaf (*Abutilon theophrasti*), and jimsonweed (*Datura stramonium*). A 12.5 cm diameter plastic pot was also filled with prepared soil and planted with rice and wheat. Another 12.5 cm pot was planted with sugarbeets. The above four containers were treated pre-emergence with several test compounds within the scope of the invention.

Twenty-eight days after treatment, the plants were evaluated and visually rated for response to the chemical treatments utilizing the ration system described previously for Test A. The data are summarized in Table B. It will be readily seen that certain compounds are useful as pre-emergence treatments for weed control in crops such as soybeans, wheat, corn and cotton.

TABLE B

PRE-EMERGENCE ON FALLSINGTON SILT LOAM

| | Compound No. | | | |
| --- | --- | --- | --- | --- |
| | 7 | | 8 | |
| kg/ha | 0.03 | 0.12 | 0.03 | 0.12 |
| Crabgrass | 0 | 2G | 5G | 6G |
| Barnyardgrass | 5G | 7G, 3H | 9G, 8C | 9G, 9C |
| Sorghum | 9G, 5H | 9G, 9C | 10C | 10C |
| Wild Oats | 7G, 3H | 7G, 4C | 7G, 6C | 7G, 6C |
| Johnsongrass | 6G, 3H | 8G, 3H | 8G, 4C | 7G, 4C |
| Dallisgrass | 0 | 6G | 8G | 9G, 8C |
| Giant Foxtail | 0 | 6G, 3H | 7G, 4C | 7G, 4C |
| Ky. Bluegrass | 5G | 7G | 9G, 9C | 10C |
| Cheatgrass | 7G, 5E | 9G, 9C | 10C | 10C |
| Sugarbeets | 6G, 7C | 8G, 8C | 8G, 8C | 8G, 9C |
| Corn | 2G | 4G, 2C | 6G, 3H | 8G, 5H |
| Mustard | 9G, 9C | 9G, 9C | 9G, 9C | 10C |
| Cocklebur | 3G | 5G | 7G | 8G |
| Pigweed | 7G | 7G | 10C | 10C |
| Nutsedge | 0 | 3G | 8G | 7G |
| Cotton | 0 | 4G, 2H | 7G, 5H | 7G, 5H |
| Morningglory | 7G, 5H | 8G, 5H | 7G, 5H | 8G, 5H |
| Cassia | 5G | 5G | 7G, 4C | 10E |
| Teaweed | 0 | 8G, 3C | 5G, 3C | 6G, 3C |
| Velvetleaf | 5G, 3H | 7G, 3H | 8G, 5C | 9G, 9C |
| Jimsonweed | 4G | 5G | 6G, 3C | 7G, 5C |
| Soybean | 0 | 3G | 4G, 3H | 6G, 5H |
| Rice | 7G | 10E | 9G, 9C | 10C |
| Wheat | 3G | 5G | 5G | 6G |

TABLE B (Continued)

PRE-EMERGENCE ON FALLSINGTON SILT LOAM

Compound No.

2

| kg/ha | 0.03 | 0.06 | 0.12 | 0.25 |
|---|---|---|---|---|
| Crabgrass | 0 | 6G | 3G | 5G |
| Barnyardgrass | 6G, 3C | 8G, 3H | 8G, 4C | 9G, 9C |
| Sorghum | 8G, 5H | 9G, 9C | 10C | 10C |
| Wild Oats | 4G | 4G, 3C | 7G, 4C | 6G, 5C |
| Johnsongrass | 6G, 3H | 8G, 5H | 7G, 3H | 9G, 7C |
| Dallisgrass | 6G | – | 7G | – |
| Giant Foxtail | 5G | 6G, 3C | 7G, 3C | 7G, 3C |
| Ky. Bluegrass | 8G, 5C | 6G, 3C | 8G, 8C | 8G, 5C |
| Cheatgrass | 7G, 3C | 8G, 8C | 8G, 3C | 10E |
| Sugarbeets | 5G | 7G, 5H | 6G, 3C | 8G, 8C |
| Corn | 0 | 5G | 2H | 5G, 3H |
| Mustard | 9G, 9C | 9G, 8C | 10C | 10C |
| Cocklebur | 6G | 5G, 3H | 6G | 6G, 5H |
| Pigweed | 3G | 6G | 10C | 8G, 8C |
| Nutsedge | 5G | 6G | 6G | 7G |
| Cotton | 0 | 4G | 5G, 5H | 6G, 5H |
| Morningglory | 4G | 4G, 2H | 8G | 8G, 5H |
| Cassia | 0 | 2G, 2C | 5G, 3C | 8G, 3H |
| Teaweed | 2G | 0 | 3G | 5G, 3H |
| Velvetleaf | 3H | 3H | 6G, 5H | 6G, 5H |
| Jimsonweed | 4G, 3C | 7G | 7G, 5C | 7G, 3C |
| Soybean | 0 | 4G | 4G, 2C | 6G, 3H |
| Rice | 5G | 6G, 3H | 7G, 3C | 7G, 5H |
| Wheat | 2G | 2G | 5G | 3G |

TABLE B (Continued)

PRE-EMERGENCE ON FALLSINGTON SILT LOAM

Compound No.

9

| kg/ha | 0.03 | 0.06 | 0.12 | 0.25 |
|---|---|---|---|---|
| Crabgrass | 5G | 7G | 6G | 8G |
| Barnyardgrass | 8G, 3H | 9G, 5H | 9G, 9C | 10C |
| Sorghum | 9C, 9C | 10C | 10C | 10C |
| Wild Oats | 5G, 3C | 3G | 7G, 3C | 6G, 3C |
| Johnsongrass | 7G, 3H | 8G, 3H | 8G, 3C | 9G, 9C |
| Dallisgrass | 7G | – | 8G | – |
| Giant Foxtail | 6G, 3C | 7G, 5H | 8G, 6C | 9G, 8C |
| Ky. Bluegrass | 8G, 7C | 8G, 5C | 8G, 8C | 8G, 8C |
| Cheatgrass | 8G, 4C | 10E | 8G, 6C | 10E |
| Sugarbeets | 7G, 7C | 6G, 3H | 7G, 7C | 8G, 8C |
| Corn | 0 | 5G, 2C | 4G, 3H | 6G, 3C |
| Mustard | 9G, 9C | 10C | 10C | 10C |
| Cocklebur | 9G | 5G, 3H | 7G | 7G, 3H |
| Pigweed | 5G | 4G | 9G, 9C | 10C |
| Nutsedge | 10E | 7G | 8G | 10E |
| Cotton | 4G, 3H | 6G | 7G, 5H | 8G, 5H |
| Morningglory | 6G | 7G, 5H | 7G | 10C |
| Cassia | 8G, 5C | 6G, 3C | 8G, 5C | 9G, 6C |
| Teaweed | 5G, 2C | 3G | 5G, 2C | 7G |
| Velvetleaf | 8G, 5H | 7G, 5H | 9G, 9C | 8G, 7C |
| Jimsonweed | 3G | 0 | 4G | 4G |
| Soybean | 0 | 0 | 4G | 5G, 3H |
| Rice | 10E | 8G, 8C | 10E | 10E |
| Wheat | 0 | 2G | 3G | 5G |

TABLE B (Continued)

PRE-EMERGENCE ON FALLSINGTON SILT LOAM

Compound No.

3

| kg/ha | 0.03 | 0.06 | 0.12 | 0.25 |
|---|---|---|---|---|
| Crabgrass | 3G | 0 | 6G | 5G |
| Barnyardgrass | 5G, 2C | 7G, 3H | 8G, 3C | 8G, 3H |
| Sorghum | 9C, 9C | 10C | 9G, 9C | 10C |
| Wild Oats | 4G | 0 | 7G, 3C | 5G |
| Johnsongrass | 7G, 3H | 7G, 5H | 8G, 5C | 8G, 5H |
| Dallisgrass | 6G | 0 | 8G | 6G, 3H |
| Giant Foxtail | 8G, 4C | 8G, 5H | 9G, 9C | 9G, 5H |
| Ky. Bluegrass | 7G, 5C | 7G, 8C | 8G, 9C | 9G, 9C |
| Cheatgrass | 8G, 5C | 8G, 5H | 8G, 8C | 8G, 9C |
| Sugarbeets | 6G, 3C | 7G, 5H | 8G, 8C | 9G, 9C |
| Corn | 2G | 0 | 6G, 3H | 5G, 3H |
| Mustard | 9G, 8C | 9G, 9C | 10C | 10C |
| Cocklebur | 7G | 7G, 3H | 7G | 7G, 5H |
| Pigweed | 6G, 5C | 10C | 10C | 10C |
| Nutsedge | 9G | 7G | 10E | 8G |
| Cotton | 0 | 3G | 4H | 6G, 5H |
| Morningglory | 7G | 3G | 7G | 6G |
| Cassia | 0 | 5G | 8G, 3C | 8G |
| Teaweed | 3G | 7G | 6G, 3C | 8G |
| Velvetleaf | 7G, 5H | 4G, 5H | 8G, 5H | 8G, 5E |
| Jimsonweed | 4G | 0 | 5G | 0 |
| Soybean | 0 | 1C | 3G, 2H | 5G, 2C |
| Rice | 8G, 9C | 10E | 8G, 9C | 10C |
| Wheat | 2G | 2G | 2G | 3G |

TABLE B (Continued)

PRE-EMERGENCE ON FALLSINGTON SILT LOAM

Compound No.

11

| kg/ha | 0.03 | 0.06 | 0.12 | 0.25 |
|---|---|---|---|---|
| Crabgrass | 2G | 4G | 6G | 7G |
| Barnyardgrass | 4G | 6G | 8G, 7C | 8G, 6C |
| Sorghum | 9C, 9C | 10C | 10E | 10C |
| Wild Oats | 6G, 2H | 4G, 3C | 7G, 3H | 6G, 4C |
| Johnsongrass | 8G, 5C | 9C, 7C | 8G | 9G, 9C |
| Dallisgrass | 5G | – | 5G | – |
| Giant Foxtail | 3G, 2C | 5G | 6G, 3C | 6G, 2C |
| Ky. Bluegrass | 6G, 2C | 7G, 4C | 7G, 3C | 8G, 8C |
| Cheatgrass | 7G | 10C | 8G, 3C | 7G, 3C |
| Sugarbeets | 8G, 8C | 10C | 9G, 9C | 10C |
| Corn | 9G, 3C | 6G, 5H | 9G, 5C | 10C |
| Mustard | 10C | 10C | 10C | 10C |
| Cocklebur | 8G, 5H | 7G, 5H | 8G, 5H | 8G, 5H |
| Pigweed | 10C | 8G, 8C | 8G, 9C | 10C |
| Nutsedge | 6G | 0 | 6G | 5G |
| Cotton | 5G, 5H | 8G, 5H | 8G, 5H | 9G, 5H |
| Morningglory | 8G | 8G, 5H | 8G | 9G, 8C |
| Cassia | 9G, 5C | 7G, 3H | 9G, 8C | 9G, 7C |
| Teaweed | 7G, 2C | 5G, 5H | 8G, 2C | 7G, 5H |
| Velvetleaf | 8G, 7C | 7G, 5H | 8G, 7C | 8G, 6C |
| Jimsonweed | 5G, 3C | 8G | 7G, 5C | 8G |
| Soybean | 0 | 0 | 3G, 2H | 5G, 5H |
| Rice | 8G, 8C | 7G, 3H | 10E | 10E |
| Wheat | 4G | 0 | 5G | 5G |

TABLE B (Continued)

PRE-EMERGENCE ON FALLSINGTON SILT LOAM

| | Compound No. | | | |
|---|---|---|---|---|
| | 10 | | 5 | |
| kg/ha | 0.03 | 0.12 | 0.03 | 0.12 |
| Crabgrass | 0 | 2G | 0 | 5G |
| Barnyardgrass | 3G | 3G | 4G | 7G, 3C |
| Sorghum | 3G | 8G, 5H | 9G, 8C | 10C |
| Wild Oats | 0 | 6G, 3H | 3G | 6G |
| Johnsongrass | 0 | 6G, 3H | 7G, 3H | 8G |
| Dallisgrass | 0 | 5G | 0 | 4G |
| Giant Foxtail | 0 | 0 | 4G | 6G, 3C |
| Ky. Bluegrass | 0 | 3G | 6G | 7G, 3C |
| Cheatgrass | 6G | 6G, 5E | 3G | 7G |
| Sugarbeets | 0 | 7G, 5H | 10C | 8G, 7C |
| Corn | 0 | 5G, 3C | 7G, 3H | 8G |
| Mustard | 4G | 8G, 8C | 8G, 8C | 10C |
| Cocklebur | – | 3G | 7G, 3H | 8G, 5H |
| Pigweed | 0 | 5G | 9G, 9C | 10E |
| Nutsedge | 0 | 0 | 5G | 6G |
| Cotton | 0 | 5H | 6G, 5H | 8G, 5H |
| Morningglory | 0 | 5H | 7G, 5H | 8G, 3C |
| Cassia | 0 | 0 | 7G, 5C | 9G |
| Teaweed | 0 | 0 | 0 | 4G, 3H |
| Velvetleaf | 0 | 4G | 6G, 5H | 8G, 5C |
| Jimsonweed | 0 | 0 | 4G, 3C | 7G, 4C |
| Soybean | 0 | 0 | 4G, 3H | 8G, 5H |
| Rice | 2G | 6G, 3C | 5G | 7G, 8C |
| Wheat | 0 | 4G | 0 | 5G |

TABLE B (Continued)

PRE-EMERGENCE ON FALLSINGTON SILT LOAM

Compound No.

12

| kg/ha | 0.03 | 0.06 | 0.12 | 0.25 |
|---|---|---|---|---|
| Crabgrass | 0 | 3G | 5G | 7G |
| Barnyardgrass | 6G | 5G | 8G, 4C | 8G, 3C |
| Sorghum | 9C, 9C | 9G, 9C | 10C | 10C |
| Wild Oats | 9G, 9C | 9G, 4C | 6G, 3H | 6G, 3H |
| Johnsongrass | 7G, 3H | 7G, 3H | 8G, 3H | 8G, 5H |
| Dallisgrass | 0 | – | 0 | – |
| Giant Foxtail | 0 | 0 | 4G | 5G, 3C |
| Ky. Bluegrass | 5G | 5G | 7G, 3C | 7G, 5C |
| Cheatgrass | 0 | 5G, 5C | 6G, 3C | 4G, 3C |
| Sugarbeets | 8G, 7C | 10C | 8G, 8C | 10C |
| Corn | 7G, 3H | 7G, 5H | 7G, 3H | 7G, 5H |
| Mustard | 10C | 9G, 9C | 10C | 10C |
| Cocklebur | 7G, 3H | 8G, 5H | 8G, 5H | 8G, 5H |
| Pigweed | 9G, 9C | 9G, 9C | 10E | 9G, 9C |
| Nutsedge | 6G | 3G | 6G | 6G |
| Cotton | 7G, 5H | 8G, 5H | 8G, 5H | 9G, 5H |
| Morningglory | 8G | 8G, 7C | 9G, 8C | 9G, 3C |
| Cassia | 7G, 3C | 7G, 3H | 8G, 5C | 8G, 7C |
| Teaweed | 5G, 5H | 3H | 6G, 5H | 8G, 5H |
| Velvetleaf | 6G, 5H | 6G, 5H | 8G, 5C | 9G, 9C |
| Jimsonweed | 2G | 5G | 5G | 7G |
| Soybean | 0 | 2G | 2G | 2G |
| Rice | 5G, 3C | 7G, 3H | 9G, 9C | 9G, 9E |
| Wheat | 0 | 3G | 4G | 4G |

TABLE B (Continued)

PRE-EMERGENCE ON FALLSINGTON SILT LOAM

| | Compound No. 6 | |
|---|---|---|
| kg/ha | 0.03 | 0.12 |
| Crabgrass | 2G | 7G |
| Barnyardgrass | 5G | 7G, 2C |
| Sorghum | 9G, 8C | 10C |
| Wild Oats | 2G | 6G, 2H |
| Johnsongrass | 7G, 3H | 8G, 3H |
| Dallisgrass | 0 | 4G |
| Giant Foxtail | 0 | 4G |
| Ky. Bluegrass | 5G | 6G |
| Cheatgrass | 3G | 7G, 3C |
| Sugarbeets | 10C | 8G, 8C |
| Corn | 7G, 5H | 8G, 4C |
| Mustard | 6G | 7G, 2C |
| Cocklebur | 6G, 3H | 7G, 5H |
| Pigweed | 8G, 7C | 10C |
| Nutsedge | 6G | 6G |
| Cotton | 7G, 5H | 8G, 5H |
| Morningglory | 6G, 5H | 8G |
| Cassia | 7G, 3C | 8G, 5C |
| Teaweed | 0 | 4G, 3H |
| Velvetleaf | 4G, 3H | 7G, 5H |
| Jimsonweed | 0 | 7G, 5C |
| Soybean | 0 | 6G, 3H |
| Rice | 6G, 3C | 7G, 8C |
| Wheat | 0 | 3G, 2C |

*Test C*

The high herbicidal activity and selective properties of several of the compounds from within the scope of the invention is evident from the results obtained in this test. The experiment concerned pre-emergence applications on soil. The containers used were 25 cm diameter plastic pots filled with Fallsington silt loam. One set of pots was planted to weeds, the seeds of which were uniformly mixed with the top 1.2 cm layer of soil. The species selected were: johnsongrass (*Sorghum halepense*), barnyardgrass (*Echinochloa crusgalli*), crabgrass (*Digitaria sanguinalis*), giant foxtail (*Setaria faberii*), velvetleaf (*Abutilon theophrasti*), jimsonweed (*Datura stramonium*), mustard (*Brassica arvensis*), and pigweed (*Amaranthus retroflexus*). Another set of pots was planted to the following crops with from one to four species per pot: corn (planting dept 3.7 cm), cotton, soybeans, sunflower, Clinton oats, wheat, Black Velentine beans, rice, sorghum, peas, flax, and peanuts (all at 2.5 cm depth), cucumbers, cabbage, alfalfa, safflower, sugarbeets, tomato, spinach, barley, and Kentucky bluegrass (all at 1.2 cm depth). Immediately after planting, the test chemicals were applied to the soil surfaces dissolved in a non-phytotoxic solvent. In the case of tobacco, a solution containing the test chemical was drenched on the surface of the soil surrounding newly transplanted tobacco seedlings. One pot from the weed phase and one of each of the crop species were left untreated for the purpose of comparison. The treated and untreated pots were promptly watered with approximately 4 mm of simulated rainfall and then held in a greenhouse for several weeks. Visual weed and crop response ratings were made 28 days after treatment utilizing the rating system as described above. The data are given in Table C. The data clearly indicate that compounds from within the scope of the present invention have utility for selective pre-emergence weed control in crops such as soybeans, alfalfa, wheat, oats, barley, cucumber, and tomato. In the case of at least one compound, weed control is obtained by applying the chemical in the form of a drench on the soil around newly-transplanted tobacco seedlings.

TABLE C

PRE-EMERGENCE ON FALLSINGTON SILT LOAM

Compound No. 3

| kg/ha | 1/64 | 1/32 | 1/16 | 1/8 |
|---|---|---|---|---|
| Corn | | | | 7G, 5H |
| Cotton | | | | 7G, 5H |
| Soybean | | | | 7G, 5H |
| Peanut | | | | 7G, 5H |
| Sunflower | | | | 8G, 5H |
| Oats | | | | 4G |
| Wheat | | | | 2G |
| Sorghum | | | | 10E |
| Sugarbeet | | | | 8G, 7C |
| Pea | | | | 9G, 9C |
| Flax | | | | 9G, 9C |
| Alfalfa | | | | 8G, 8C |
| Bean | | | | 7G, 5H |
| Spinach | | | | 9G, 8C |
| Cabbage | | | | 9G, 9C |
| Tomato | | | | 4G |
| Rice | | | | 10E |
| Safflower | | | | 8G, 6C |
| Cucumber | | | | 9G, 5H |
| Ky. Bluegrass | | | | 8G, 9C |
| Barley | | | | 8G, 6C |
| Tobacco | | | | 0 |
| Broadleaves | 2G, 3C | 4G, 4C | 5G, 4C | |
| Grasses | 5G, 2C | 6G, 3C | 7G, 5C | |

TABLE C (Continued)

PRE-EMERGENCE ON FALLSINGTON SILT LOAM

Compound No. 11

| kg/ha | 1/128 | 1/64 | 1/32 | 1/16 |
|---|---|---|---|---|
| Corn | | | | 8G, 8C |
| Cotton | | | | 7G, 5H |
| Soybean | | | | 0 |
| Peanut | | | | - |
| Sunflower | | | | 9G, 8C |
| Oats | | | | 6G, 2C |
| Wheat | | | | 5G |
| Sorghum | | | | 9G, 9C |
| Sugarbeet | | | | 9G, 9C |
| Pea | | | | 10E |
| Flax | | | | 7G, 5E |
| Alfalfa | | | | 7G, 3C |
| Bean | | | | 8G, 5H |
| Spinach | | | | 8G, 8C |
| Cabbage | | | | 8G, 7C |
| Tomato | | | | 7G, 5H |
| Rice | | | | 7G, 7C |
| Safflower | | | | 8G, 3C |
| Cucumber | | | | 9G |
| Ky. Bluegrass | | | | 7G |
| Barley | | | | 3G |
| Tobacco | | | | - |
| Broadleaves | 5G, 3C | 7G, 7C | 8G, 7C | |
| Grasses | 2G | 3G | 4G | |

TABLE C (Continued)

PRE-EMERGENCE ON FALLSINGTON SILT LOAM

Compound No. 12

| kg/ha | 1/128 | 1/64 | 1/32 | 1/16 |
|---|---|---|---|---|
| Corn | | | | 8G, 8C |
| Cotton | | | | 8G, 5H |
| Soybean | | | | 0 |
| Peanut | | | | – |
| Sunflower | | | | 9G, 8C |
| Oats | | | | 5G, 2C |
| Wheat | | | | 4G |
| Sorghum | | | | 8G, 8C |
| Sugarbeet | | | | 9G, 9C |
| Pea | | | | 10E |
| Flax | | | | 8G, 8C |
| Alfalfa | | | | 4G |
| Bean | | | | 5G, 3H |
| Spinach | | | | 8G, 9C |
| Cabbage | | | | 8G, 6C |
| Tomato | | | | 2G |
| Rice | | | | 9G, 8C |
| Safflower | | | | 8G, 3C |
| Cucumber | | | | 5G |
| Ky. Bluegrass | | | | 6G |
| Barley | | | | 3G |
| Tobacco | | | | – |
| Broadleaves | 3G | 2G | 5G, 3C | |
| Grasses | 3G | 0 | 3G | |

*Test D*

Twenty-five cm diameter plastic pots filled with Fallsington silt loam were planted with soybeans, cotton, alfalfa, corn, rice, wheat, sorghum, velvetleaf (*Abutilon theophrasti*), sesbania (*Sesbania exaltata*), Cassia (*Cassia tora*), morningglory (*Ipomoea hederacea*), jimsonweed (*Datura stramonium*), cocklebur (*Xanthium pensylvanicum*), crabgrass (*Digitaria spp.*), nutsedge (*Cyperus rotundus*), barnyardgrass (*Echinochloa crusgalli*), giant foxtail (*Setaria faberii*) and wild oats (*Avena fatua*). In the case of compound No. 13, the following additional species were included: sunflower, mustard, sugarbeets and bushbean. Approximately 2½ weeks after planting, the young plants and the soil around them were sprayed overall with the test chemicals dissolved in a non-phytotoxic solvent. Two weeks after treatment, all species were compared to untreated controls and visually rated for response to treatment. The rating system was as described previously for Test A. The data are presented in Table D. Several of the compounds tested by this procedure are useful for the post-emergence control of weeds in wheat, corn and soybeans.

TABLE D

OVER-THE-TOP SOIL / FOLIAGE TREATMENT

Compound No.

7

| kg/ha | 0.016 | 0.031 | 0.063 | 0.125 |
|---|---|---|---|---|
| Soybeans | 0 | 9G, 4C | 4G, 2C | 10G, 7C |
| Velvetleaf | 8G, 3C | 9G, 4C | 8G, 3C | 10G, 7C |
| Sesbania | 5G, 3C | 7G, 3C | 7G, 3C | 10G, 5C |
| Cassia | 4G, 2C | 9G, 3C | 6G, 2C | 10G, 6C |
| Cotton | 3G, 2C | 7G, 3C | 5G, 3C | 9G, 6C |
| Morningglory | 9G, 4C | 10G, 7C | 10G, 7C | 10G, 8C |
| Alfalfa | 2C, 3G | 5G, 2C | 2C, 3G | 8G, 5C |
| Jimsonweed | 1C | 3G, 2C | 4G, 2C | 7G, 3C |
| Cocklebur | 1C | 10G, 6C | 6G, 3C | 10G, 7C |
| Corn | 1H | 0 | 2G, 1C | 1G, 2H |
| Crabgrass | 1G | 0 | 1G | 4G |
| Rice | 7G, 2C | 8G, 3C | 8G, 5C | 9G, 6C |
| Nutsedge | 2G | 3G, 3C | 2G, 1C | 6G, 2C |
| Barnyardgrass | 1C, 6G | 7G, 3C | 1C, 6G | 10C |
| Wheat | 0 | 1G | 2G | 3G |
| Giant Foxtail | – | 7G, 2C | 2G | 9C |
| Wild Oats | 6G | 7G, 2C | 7G | 7G, 10C |
| Sorghum | 8G, 1C | 9G, 3C | 8G, 4C | 10C |

TABLE D (Continued)

OVER-THE-TOP SOIL / FOLIAGE TREATMENT

| | Compound No. | | |
|---|---|---|---|
| | | 8 | |
| kg/ha | 0.016 | 0.031 | 0.125 |
| Soybeans | 8G, 3C | 10G, 7C | 10G, 7C |
| Velvetleaf | 8G, 3C | 10G, 6C | 10G, 8C |
| Sesbania | 9G, 5C | 9G, 5C | 10G, 8C |
| Cassia | 5G, 3C | 10G, 4C | 10G, 6C |
| Cotton | 8G, 4C | 9G, 4C | 10G, 6C |
| Morningglory | 9G, 5C | 10G, 8C | 10G, 8C |
| Alfalfa | 5G, 3C | 7G, 4C | 8G, 6C |
| Jimsonweed | 1G, 1C | 7G, 3C | 9G, 4C |
| Cocklebur | 6G, 2C | 9G, 3C | 10G, 7C |
| Corn | 7G, 4H | 1H | 6G, 3C |
| Crabgrass | 2G | 4G | 6G |
| Rice | 8G, 3C | 8G, 3C | 8G, 5C |
| Nutsedge | 4G, 1C | 5G, 1C | 7G |
| Barnyardgrass | 6G, 2C | 8G, 5C | 9G, 7C |
| Wheat | 1G | 2G | 2G |
| Giant Foxtail | 5G | 7G, 3C | 8G, 3C |
| Wild Oats | 7G | 8G | 8G |
| Sorghum | 8G, 1U | 9C | 9C |

TABLE D (Continued)

OVER-THE-TOP SOIL / FOLIAGE TREATMENT

Compound No.

9

| kg/ha | 0.016 | 0.031 | 0.063 | 0.125 |
|---|---|---|---|---|
| Soybeans | 10G, 7C | 10G, 6C | 10G, 7C | 10G, 8C |
| Velvetleaf | 8G | 10G, 7C | 10G, 3C | 10G, 9C |
| Sesbania | 10G, 8C | 10G, 8C | 10G, 8C | 10G, 9C |
| Cassia | 9G, 5C | 10G, 7C | 10G, 7C | 10G, 7C |
| Cotton | 7G, 3C | 9G, 3C | 10G, 6C | 10G, 7C |
| Morningglory | 10G, 6C | 10G, 7C | 10G, 7C | 10G, 8C |
| Alfalfa | 8G, 4C | 6G, 2C | 8G, 3C | 7G, 4C |
| Jimsonweed | 0 | 1G | 4G, 1C | 1G |
| Cocklebur | 9G, 6C | 10G, 9C | 10G, 7C | 10G, 9C |
| Corn | 3G, 2H | 0 | 4G, 4H | 1G, 1H |
| Crabgrass | 1G | 7G | 2G | 9G, 5C |
| Rice | 8G, 5C | 9G, 6C | 8G, 7C | 10G, 7C |
| Nutsedge | 5G, 2C | 6G, 2C | 7G, 3C | 8G |
| Barnyardgrass | 6G, 3C | 10C | 8G, 5C | 10C |
| Wheat | 1G | 2G | 4G | 2G |
| Giant Foxtail | 5G, 1C | 8G, 3C | 6G, 1C | 8G, 4C |
| Wild Oats | 5G | 7G, 1C | 7G, 2C | 8G, 2C |
| Sorghum | 9G, 3C | 10G, 8C | 9G, 4C | 9C |

TABLE D (Continued)

OVER-THE-TOP SOIL / FOLIAGE TREATMENT

Compound No.

10

| kg/ha | 0.016 | 0.031 | 0.063 | 0.125 |
|---|---|---|---|---|
| Soybeans | 0 | 0 | 1G, 1C | 5G, 3C |
| Velvetleaf | 8G, 3C | 6C, 3C | 8G, 3C | 7G, 3C |
| Sesbania | 2C | 7G, 3C | 4G, 3C | 7G, 3C |
| Cassia | 2C | 10G, 8C | 4G, 3C | 7G, 3C |
| Cotton | 3G, 2C | 7G, 3C | 5G, 3C | 8G, 3C |
| Morningglory | 8G, 3C | 10G, 4C | 9G, 3C | 10G, 6C |
| Alfalfa | 2C | 7G, 4C | 3G, 2C | 8G, 4C |
| Jimsonweed | 5G, 2C | 7G, 3C | 5G, 2C | 7G, 3C |
| Cocklebur | 1C | 9G, 3C | 3G, 2C | 10G, 5C |
| Corn | 2H, 1C | 6G, 5H | 3G, 2H | 7G, 3U |
| Crabgrass | 1G | 3G, 1C | 1G | 6G, 2C |
| Rice | 7G, 1C | 9G, 3C | 7G, 1C | 10G, 7C |
| Nutsedge | 2G | 0 | 2G, 1C | 0 |
| Barnyardgrass | 1G, 1C | 6G | 1G, 1C | 8G, 2C |
| Wheat | 0 | 1G | 0 | 1G |
| Giant Foxtail | 1G | 6G, 2C | 3G, 1C | 7G, 3C |
| Wild Oats | 2G | 7G | 2G | 7G |
| Sorghum | 8G, 1C | 9G, 3C | 8G, 2U | 9C |

TABLE D (Continued)

OVER-THE-TOP SOIL / FOLIAGE TREATMENT

| | Compound No. | | |
|---|---|---|---|
| | | 11 | |
| kg/ha | 0.016 | 0.031 | 0.125 |
| Soybeans | 1C | 10G, 7C | 10G, 7C |
| Velvetleaf | 8G, 3C | 10G, 8C | 10G, 9C |
| Sesbania | 10G, 6C | 10G, 7C | 10G, 9C |
| Cassia | 8G, 3C | 7G, 4C | 9G, 5C |
| Cotton | 6G, 3C | 10G, 7C | 10G, 7C |
| Morningglory | 10G, 4C | 10G, 8C | 10G, 9C |
| Alfalfa | 7G, 4C | 7G, 7C | 9G, 8C |
| Jimsonweed | 3G, 1C | 9G, 7C | 10G, 7C |
| Cocklebur | 10G, 7C | 10G, 8C | 10G, 8C |
| Corn | 9G, 4C | 10G, 6C | 10G, 6C |
| Crabgrass | 0 | 8G, 5C | 8G, 5C |
| Rice | 6G, 2C | 8G, 6C | 8G, 6C |
| Nutsedge | 1G | 1G | 2G |
| Barnyardgrass | 6G, 1C | 8G, 3C | 10C |
| Wheat | 0 | 0 | 1G |
| Giant Foxtail | 3G | – | 8G, 3C |
| Wild Oats | 1G | 8G, 2C | 8G, 1C |
| Sorghum | 9G, 2C | 9C | 9C |

TABLE D (Continued)

OVER-THE-TOP SOIL / FOLIAGE TREATMENT

| | Compound No. 12 | | |
|---|---|---|---|
| kg/ha | 0.016 | 0.031 | 0.125 |
| Soybeans | 0 | 7G, 3C | 10G, 7C |
| Velvetleaf | 7G, 3C | 10G, 8C | 10G, 8C |
| Sesbania | 6G, 3C | 10G, 8C | 10G, 8C |
| Cassia | 4G, 3C | 10G, 7C | 10G, 4C |
| Cotton | 4G, 3C | 10G, 4C | 10G, 7C |
| Morningglory | 9G, 3C | 10G, 7C | 10G, 9C |
| Alfalfa | 0 | 2G | 5G, 1C |
| Jimsonweed | 2G, 1C | 0 | 6G, 1C |
| Cocklebur | 1C | 10G, 8C | 10G, 8C |
| Corn | 9G, 2C | 9G, 3U | 10G, 8U |
| Crabgrass | 0 | 3G | 7G, 4C |
| Rice | 4G | 7G, 2C | 8G, 3C |
| Nutsedge | 4G | 2G | 3G |
| Barnyardgrass | 3G | 7G, 3C | 8G, 4C |
| Wheat | 0 | 0 | 0 |
| Giant Foxtail | 0 | 3G, 1C | 6G, 2C |
| Wild Oats | 0 | 3G | 7G |
| Sorghum | 8G | 9G, 7C | 9C |

TABLE D (Continued)

OVER-THE-TOP SOIL / FOLIAGE TREATMENT

| | Compound No. 2 | | |
|---|---|---|---|
| kg/ha | 0.016 | 0.031 | 0.125 |
| Soybeans | 5G, 3C | 9G, 7C | 10G, 7C |
| Velvetleaf | 6G, 3C | 9G, 5C | 10G, 9C |
| Sesbania | 5G, 3C | 9G, 4C | 10G, 8C |
| Cassia | 3G, 2C | 10G, 6C | 10G, 8C |
| Cotton | 3G, 2C | 7G, 4C | 10G, 7C |
| Morningglory | 6G, 2C | 10G, 5C | 10G, 9C |
| Alfalfa | 4G, 2C | 7G, 3C | 7G, 4C |
| Jimsonweed | 2G, 1C | 5G, 2C | 7G, 3C |
| Cocklebur | 5G, 2C | 9G, 4C | 10G, 7C |
| Corn | 0 | 2G | 3G |
| Crabgrass | 1G, 1C | 1G | 3G |
| Rice | 4G | 5G, 2C | 7G, 3C |
| Nutsedge | 3G | 6G, 1C | 7G, 1C |
| Barnyardgrass | 5G, 1C | 8G, 3C | 9G, 4C |
| Wheat | 0 | 3G | 4G |
| Giant Foxtail | 0 | 6G, 2C | 7G, 1C |
| Wild Oats | 0 | 4G | 7G, 2C |
| Sorghum | 8G, 1C | 10G | 10G, 2U |

TABLE D (Continued)

OVER-THE-TOP SOIL / FOLIAGE TREATMENT

Compound No.

3

| kg/ha | 0.016 | 0.031 | 0.063 | 0.125 |
|---|---|---|---|---|
| Soybeans | 6G, 3C | 10G, 7C | 9G, 3C | 8G, 5C |
| Velvetleaf | 5G, 3C | 10G, 7C | 8G, 3C | 10G, 9C |
| Sesbania | 5G, 3C | 10G, 8C | 7G, 3C | 10G, 8C |
| Cassia | 4G, 3C | 10G, 8C | 5G, 3C | 10G, 8C |
| Cotton | 3C | 8G, 5C | 7G, 2C | 8G, 7C |
| Morningglory | 6G, 3C | 10G, 9C | 7G, 3C | 10G, 8C |
| Alfalfa | 7G, 3C | 8G, 5C | 7G, 3C | 8G, 5C |
| Jimsonweed | 4G, 1C | 0 | 3G, 1C | 5G, 1C |
| Cocklebur | 5G, 2C | 10G, 7C | 7G, 4C | 10G, 7C |
| Corn | 0 | 0 | 2G, 1C | 0 |
| Crabgrass | 1G | 0 | 3G | 6G, 1C |
| Rice | 5G, 1C | 3G, 3C | 8G, 1C | 5G, 4C |
| Nutsedge | 2G | 7G, 3C | 3G | 6G |
| Barnyardgrass | 4G, 1C | 8G | 6G, 1C | 8G, 3C |
| Wheat | 0 | 0 | 0 | 0 |
| Giant Foxtail | 3G | 7G, 3C | 3G | 8G, 1C |
| Wild Oats | 0 | 4G, 1C | 5G | 3G |
| Sorghum | 9G | 7G, 3C | 9G, 2U | 8G, 3C |

TABLE D (Continued)

OVER-THE-TOP SOIL / FOLIAGE TREATMENT

| | Compound No. | | |
|---|---|---|---|
| | | 5 | |
| kg/ha | 0.016 | 0.031 | 0.125 |
| Soybeans | 6H, 3C | 9G, 5C | 10G, 7C |
| Velvetleaf | 8G, 4C | 10G, 7C | |
| Sesbania | 5G, 3C | 9G, 5C | 10G, 7C |
| Cassia | 4G, 3C | 10G, 8C | 10G, 8C |
| Cotton | 5G, 2C | 9G, 2C | 10G, 6C |
| Morningglory | 7G, 3C | 10G, 7C | 10G, 7C |
| Alfalfa | 4G, 3C | 7G, 3C | 7G, 4C |
| Jimsonweed | 5G, 2C | 6G, 3C | 8G, 3C |
| Cocklebur | 7G, 3C | 9G, 4C | 10G, 7C |
| Corn | 9G, 3C | 8G, 6H | 8G, 8H |
| Crabgrass | 0 | 2G | 3G |
| Rice | 4G | 5G, 2C | 7G, 3C |
| Nutsedge | 1G | 3G | 4G |
| Barnyardgrass | 6G, 1C | 8G, 4C | 9G, 4C |
| Wheat | 0 | 3G | 3G |
| Giant Foxtail | 3G | 4G | 6G |
| Wild Oats | 1G | 2G | 3G, 1C |
| Sorghum | 9G, 2C | 9G, 3U | 9G, 3U |

TABLE D (Continued)

OVER-THE-TOP SOIL / FOLIAGE TREATMENT

| | Compound No. | | |
| --- | --- | --- | --- |
| | | 6 | |
| kg /ha | 0.016 | 0.031 | 0.125 |
| Soybeans | 7G, 3C | 10G, 7C | 10G, 8C |
| Velvetleaf | 7G, 3C | 9G, 4C | 10G, 7C |
| Sesbania | 4G, 3C | 8G, 4C | 10G, 8C |
| Cassia | 4G, 3C | 10G, 7C | 10G, 8C |
| Cotton | 6G, 3C | 9G, 3C | 10G, 6C |
| Morningglory | 8G, 3C | 10G, 7C | 10G, 8C |
| Alfalfa | 4G, 2C | 6G, 2C | 8G, 4C |
| Jimsonweed | 2G, 2C | 7G, 1C | 8G, 2C |
| Cocklebur | 1C | 10G, 6C | 10G, 8C |
| Corn | 9G, 3C | 8G, 4C | 9G, 4C |
| Crabgrass | 0 | 1G | 6G |
| Rice | 4G, 1C | 4G, 2C | 7G, 3C |
| Nutsedge | 0 | 2G | 3G |
| Barnyardgrass | 0 | 8G, 2C | 8G, 4C |
| Wheat | 0 | 0 | 2G |
| Giant Foxtail | 0 | 7G, 1C | 7G |
| Wild Oats | 0 | 2G | 3G, 1C |
| Sorghum | 8G, 1C | 9G, 3U | 9G, 3U |

TABLE D (Continued)

OVER-THE-TOP SOIL / FOLIAGE TREATMENT

Compound No.

13

| kg/ha | 0.016 | 0.031 | 0.062 | 0.125 |
|---|---|---|---|---|
| Soybeans | 3G | 3G | 7G, 1C | 7G, 2C |
| Velvetleaf | 7G, 2C | 7G, 2C | 9G, 3C | 9G, 4C |
| Sesbania | 7G, 3C | 8G, 4C | 9G, 4C | 9G, 4C |
| Cassia | 8G, 4C | 9G, 5C | 9G, 6C | 10G, 5C |
| Cotton | 1C | 4G, 3C | 7G, 3C | 7G, 4C |
| Morningglory | 8G, 4C | 8G, 4C | 9G, 5C | 9G, 5C |
| Alfalfa | 2C | 4G, 2C | 5G, 2C | 6G, 2C |
| Jimsonweed | 3G | – | 6G | 8G |
| Cocklebur | 4G, 3C | 6G, 3C | 7G, 4C | 10G, 3C |
| Corn | 4H | 8G, 4H | 9G, 4C | 9G, 3U |
| Crabgrass | 0 | 0 | 2G | 2G |
| Rice | 2G | 2G | 5G | 6G |
| Nutsedge | 0 | 1G | 2G | 5G |
| Barnyardgrass | 0 | 3G | 5G, 3C | 5G, 3C |
| Wheat | 0 | 1G | 2G | 3G |
| Giant Foxtail | 2G | 2G | 4G | 4G |
| Wild Oats | 0 | 0 | 2G | 2G |
| Sorghum | 4G | 6G | 7G | 7G, 3C |
| Sunflower | 0 | 9G, 7C | 10G, 8C | 10G, 8C |
| Mustard | 9G, 3C | 9G, 3C | 9G, 3C | 9G, 3C |
| Sugarbeets | 1C | 2C | 4G, 3C | 5G, 3C |
| Bushbean | 0 | 0 | 0 | 0 |

*Test E*

Two ten-inch in diameter plastic pans lined with polyethylene liners were filled with prepared Fallsington slit loam soil. One pan was planted with seeds of wheat (*Triticum aestivum*), barley (*Hordeum vulgare*), wild oats (*Avena fatua*), downy brome (*Bromus tectorum*), cheatgrass (*Bromus secalinus*), blackgrass (*Alopecurus myosuroides*), annual bluegrass (*Poa annua*), green foxtail (*Setaria viridis*), quackgrass (*Agropyron repens*), Italian ryegrass (*Lolium multiflorum*) and ripgut brome (*Bromus rigidus*). The other pan was planted with seeds of Russian thistle (*Salsola kali*), tansy mustard (*Descuraina pinnata*), smartweed (*Polygonum pennsylvanicum*), jimhill or tumble mustard (*Sisymbrium altissium*), kochia (*Kochia scoparia*), shepherd's purse (*Capsella bursa-pastoris*), *Matricaria inordora*, black nightshade (*Solanum nigrum*), yellow rocket (*Barbarea vulgaris*), wild mustard (*Brassica kaber*) and wild buckwheat (*Polygonum convolvulus*). The above two pans were treated pre-emergence. At the same time two pans in which the above plant species were growing were treated post-emergence. Plant height at the time of treatment ranged from 1—15 cm depending on plant species.

The compounds applied were diluted with a non-phytotoxic solvent and sprayed over-the-top of the pans. An untreated control and a solvent-alone control were included for comparison. All treatments were maintained in the greenhouse for 20 days at which time the treatments were compared to the controls and the effects visually rated in accordance with the previously described rating system. The recorded data are presented in Table E.

Several of the compounds included in this table provide excellent weed control at very low rates of application, yet are tolerated with little or no injury by wheat and barley at rates which are multiples of those required for weed control. This observation applied to both pre- and post-emergence treatments.

TABLE E

| TEST PLANT | Compound No. 2 | | | |
|---|---|---|---|---|
| kg/ha | 0.02 | 0.06 | 0.02 | 0.06 |
| | Pre-Emergence | | Post-Emergence | |
| Wheat | 0 | 0 | 0 | 1G |
| Barley | 2C, 4G | 5C, 4G | 1G | 4C, 6G |
| Wild Oats | 3C, 2G | 5C, 5G | 1G | 6C, 6G |
| Downy Brome | 7C, 4G | 8C, 7G | 2C, 2G | 10C |
| Cheatgrass | 6C, 5G | 10C | 3C, 5G | 8C, 7G |
| Blackgrass | 7C, 7G | 10C | 5C, 5G | 7C, 7G |
| Annual Bluegrass | 3G | 6C, 7G | 4C | 6C, 7G |
| Green Foxtail | 4C, 2G | 6C, 6G | 2G | 6C, 6G |
| Quackgrass | 3G | 4C, 6G | 2C, 4G | 3C, 7G |
| Italian Ryegrass | 2G | 2C, 5G | 3G | 6G |
| Ripgut Brome | 2C, 4G | 6C, 7G | 1G | 2C, 4G |
| Russian Thistle | 9C, 8G | 9C, 9G | 2C, 2G | 5C, 7G |
| Tansy Mustard | 10C | 10C | 10C | 10C |
| Smartweed | - | - | - | - |
| Jimhill Mustard | 10C | 10C | 10C | 10C |
| Kochia | 4G | 2C, 5G | 7C, 6G | 7C, 7G |
| Shepherd's Purse | 10C | 10C | 7C, 7G | 8C, 7G |
| False Chamomile | 4C, 6G | 7C, 8G | 5C, 5G | 7C, 7G |
| Black Nightshade | 1C, 7G | 2C, 8G | 3G | 1C, 5G |
| Yellow Rocket | 9C, 9G | 9C, 9G | 5C, 6G | 10C |
| Wild Mustard | 8C, 8G | 9C, 9G | 10C | 10C |
| Wild Buckwheat | 3G | 4C, 4G | 2C, 2G | 5C, 5G |

TABLE E (Continued)

Compound No.

3

| TEST PLANT | | | | |
|---|---|---|---|---|
| kh /ha | 0.02 | 0.06 | 0.02 | 0.06 |
| | Pre-Emergence | | Post-Emergence | |
| Wheat | 0 | 1G | 0 | 1C, 1G |
| Barley | 3G | 3C, 7G | 6C, 5G | 7C, 7G |
| Wild Oats | 3G | 5C, 7G | 1C, 2G | 5C, 6G |
| Downy Brome | 7C, 7G | 10C | 7C, 7G | 10C |
| Cheatgrass | 10C | 10C | 8C, 8G | 10C |
| Blackgrass | 10C | 10C | 10C | 10C |
| Annual Bluegrass | 7C, 7G | 7C, 8G | 7C, 6G | 9C, 7G |
| Green Foxtail | 7C, 6G | 7C, 8G | 3C, 6G | 7C, 7G |
| Quackgrass | 7G | 5C, 8G | 1C, 6G | 5C, 7G |
| Italian Ryegrass | 6G | 2C, 7G | 2G | 2C, 4G |
| Ripgut Brome | 3C, 7G | 4C, 8G | 1C, 2G | 3C, 5G |
| Russian Thistle | 4C, 5G | 7C, 7G | 2C, 3G | 10C |
| Tansy Mustard | 10C | 10C | 10C | 10C |
| Smartweed | - | - | - | - |
| Jimhill Mustard | 10C | 10C | 10C | 10C |
| Kochia | 6G | 3C, 7G | 2G | 2C, 4G |
| Shepherd's Purse | 7C, 7G | 9C, 9G | 9C, 8G | 10C |
| False Chamomile | 9C, 9G | 9C, 9G | 9C, 9G | 10C |
| Black Nightshade | 7C, 8G | 7C, 8G | 7C, 8G | 10C |
| Yellow Rocket | 9C, 9G | 9C, 9G | 10C | 10C |
| Wild Mustard | 7C, 8G | 9C, 9G | 10C | 10C |
| Wild Buckwheat | 3C, 4G | 4C, 5G | 1C, 2G | 4C, 4G. |

TABLE E (Continued)

| TEST PLANT | Compound No. 3 | | | |
|---|---|---|---|---|
| kg/ha | 0.02 | 0.06 | 0.02 | 0.06 |
| | Pre-Emergence | | Post-Emergence | |
| Wheat | 0 | 2C, 1G | 0 | 1C, 2G |
| Barley | 5G | 3C, 8G | 5C, 7G | 8C, 7G |
| Wild Oats | 1C, 5G | 5C, 7G | 4C, 5G | 6C, 6G |
| Downy Brome | 6C, 7G | 10C | 7C, 8G | 10C |
| Cheatgrass | 9C, 8G | 10C | 10C | 10C |
| Blackgrass | 10C | 10C | 10C | 10C |
| Annual Bluegrass | 5C, 6G | 9C, 9G | 9C | 10C |
| Green Foxtail | 3C, 4G | 9C, 9G | 6C, 6G | 7C, 6G |
| Quackgrass | 4C, 6G | 9C, 8G | 3C, 6G | 8C, 7G |
| Italian Ryegrass | 3G | 4C, 6G | 1C, 3G | 4C, 5G |
| Ripgut Brome | 3G | 5C, 7G | 3C, 4G | 6C, 7G |
| Russian Thistle | – | – | – | – |
| Tansy Mustard | 10C | 10C | 10C | 10C |
| Smartweed | – | – | – | – |
| Jimhill Mustard | 10C | 10C | 10C | 10C |
| Kochia | 2C, 5G | 5C, 6G | 0 | 2G |
| Shepherd's Purse | 10C | 10C | 10C | 10C |
| False Chamomile | 8C, 8G | 9C, 9G | 10C | 10C |
| Black Nightshade | 3C, 6G | 5C, 6G | 9C, 8G | 10C |
| Yellow Rocket | 9C, 9G | 10C | 10C | 10C |
| Wild Mustard | 9C, 8G | 9C, 9G | 10C | 10C |
| Wild Buckwheat | 2C, 4G | 5C, 6G | 3C, 4G | 4C, 4G |

TABLE E (Continued)

| TEST PLANT | Compound No. 3 | | | |
|---|---|---|---|---|
| kg/ha | 0.02 | 0.06 | 0.02 | 0.06 |
| | Pre-Emergence | | Post-Emergence | |
| Wheat | 0 | 1C, 1G | 0 | 0 |
| Barley | 5G | 6C, 7G | 4C, 6G | 8C, 8G |
| Wild Oats | 5G | 4C, 6G | 1G | 1C, 2G |
| Downy Brome | 7C, 7G | 10C | 6C, 5G | 7C, 8G |
| Cheatgrass | 10C | 10C | 8C, 7G | 8C, 8G |
| Blackgrass | 10C | 10C | 9C, 8G | 10C |
| Annual Bluegrass | 7C, 8G | 7C, 8G | 4C, 6G | 7C, 8G |
| Green Foxtail | 5C, 6G | 8C, 8G | 2C, 2G | 3C, 3G |
| Quackgrass | 7C, 6G | 9C, 8G | 5C, 6G | 5C, 7G |
| Italian Ryegrass | 3G | 1C, 7G | 1G | 3G |
| Ripgut Brome | 2C, 6G | 7C, 7G | 2C, 5G | 3C, 6G |
| Russian Thistle | 0 | 2C, 2G | 10C | 10C |
| Tansy Mustard | 9C, 9G | 10C | 10C | 10C |
| Smartweed | - | - | - | - |
| Jimhill Mustard | 9C, 9G | 10C | 10C | 10C |
| Kochia | 5G | 3C, 8G | 3C, 2G | 2C, 4G |
| Shepherd's Purse | 9C, 9G | 9C, 9G | 8C, 7G | 8C, 8G |
| False Chamomile | 8C, 9G | 9C, 9G | 9C, 8G | 10C |
| Black Nightshade | 2C, 7G | 4C, 7G | 5C, 6G | 7C, 7G |
| Yellow Rocket | 8C, 8G | 9C, 8G | 10C | 10C |
| Wild Mustard | 8C, 8G | 9C, 8G | 10C | 10C |
| Wild Buckwheat | 4G | 3C, 6G | 1C, 5G | 5C, 6G |

TABLE E (Continued)

| TEST PLANT | Compound No. 3 | | |
|---|---|---|---|
| kg/ha | 0.002 | 0.02 | 0.12 |
| | | Pre-Emergence | |
| Wheat | 0 | 0 | 0 |
| Barley | 0 | 0 | 3G |
| Wild Oats | 0 | 0 | 1C, 3G |
| Downy Brome | 1G | 2C, 3G | 3C, 5G |
| Cheatgrass | 2G | 6C, 7G | 7C, 8G |
| Blackgrass | 1C, 4G | 7C, 7G | 10C |
| Annual Bluegrass | 2G | 1C, 4G | 4C, 6G |
| Green Foxtail | 0 | 1C, 1G | 3C, 6G |
| Quackgrass | 0 | 2G | 2C, 6G |
| Italian Ryegrass | 0 | 1G | 1C, 4G |
| Ripgut Brome | 0 | 0 | 5G |
| Russian Thistle | 0 | 0 | 1C, 2G |
| Tansy | 8C, 8G | 8C, 8G | 9C, 9G |
| Smartweed | - | - | - |
| Jimhill Mustard | 2C, 3G | 7C, 8G | 9C, 9G |
| Kochia | 0 | 2G | 3G |
| Shepherd's Purse | 3C, 5G | 9C, 9G | 9C, 9G |
| *Matricaria inodora* | 3C, 4G | 7C, 7G | 7C, 8G |
| Black Nightshade | 0 | 1C, 3G | 3C, 6G |
| Yellow Rocket | 2C, 4G | 3C, 8G | 7C, 9G |
| Wild Mustard | 3C, 4G | 7C, 7G | 8C, 8G |
| Wild Buckwheat | 1C, 1G | 2G | 3C, 4G |

TABLE E (Continued)

| TEST PLANT | Compound No. 3 | | |
|---|---|---|---|
| kg/ha | 0.002 | 0.02 | 0.12 |
| | | Post-Emergence | |
| Wheat | 0 | 1G | 3G |
| Barley | 0 | 3G | 6G |
| Wild Oats | 0 | 1G | 1C, 5G |
| Downy Brome | 2G | 4C, 5G | 6C, 5G |
| Cheatgrass | 5C, 6G | 7C, 6G | 7C, 6G |
| Blackgrass | 6C, 6G | 10C | 10C |
| Annual Bluegrass | 1C, 4G | 7C, 7G | 8C, 8G |
| Green Foxtail | 0 | 2C, 3G | 8C, 8G |
| Quackgrass | 0 | 2G | 1C, 3G |
| Italian Ryegrass | 0 | 0 | 2G |
| Ripgut Brome | 0 | 2G | 1C, 5G |
| Russian Thistle | 2C, 2G | 3C, 4G | 7C, 7G |
| Tansy Mustard | 8C, 7G | 10C | 10C |
| Smartweed | - | - | - |
| Jimhill Mustard | 10C | 10C | 10C |
| Kochia | 0 | 0 | 1G |
| Shepherd's Purse | 10C | 9C, 8G | 10C |
| *Matricaria inodora* | 9C, 7G | 9C, 8G | 10C |
| Black Nightshade | 2C, 3G | 2C, 4G | 4C, 7G |
| Yellow Rocket | 10C | 10C | 10C |
| Wild Mustard | 10C | 10C | 10C |
| Wild Buckwheat | 2C, 2G | 3C, 4G | 3C, 4G |

TABLE E (Continued)

| TEST PLANT | Compound No. 3 | | |
|---|---|---|---|
| kg/ha | 0.002 | 0.02 | 0.12* |
| | | Pre-Emergence | |
| Wheat | 0 | 0 | 0 |
| Barley | 0 | 0 | 3G |
| Wild Oats | 0 | 0 | 3G |
| Downy Brome | 3G | 3G | 4C, 6G |
| Cheatgrass | 2C, 4G | 4C, 6G | 7C, 7G |
| Blackgrass | 1C, 6G | 5C, 6G | 10C |
| Annual Bluegrass | 2G | 1C, 3G | 4C, 6G |
| Green Foxtail | 0 | 3C, 4G | 4C, 7G |
| Quackgrass | 1G | 3G | 2C, 6G |
| Italian Ryegrass | 0 | 2G | 1C, 3G |
| Ripgut Brome | 0 | 2G | 3G |
| Russian Thistle | 0 | 0 | 1C, 2G |
| Tansy Mustard | 5C, 6G | 9C, 9G | 9C, 9G |
| Smartweed | – | – | – |
| Jimhill Mustard | 7C, 7G | 9C, 8G | 9C, 9G |
| Kochia | 0 | 1G | 2C, 3G |
| Shepherd's Purse | 8C, 8G | 9C, 9G | 10C |
| *Matricaria inodora* | 6C, 5G | 7C, 7G | 8C, 8G |
| Black Nightshade | 1C, 4G | 3C, 5G | 5C, 6G |
| Yellow Rocket | 4C, 7G | 7C, 8G | 7C, 8G |
| Wild Mustard | 7C, 7G | 7C, 7G | 8C, 8G |
| Wild Buckwheat | 1G | 1C, 1G | 2C, 3G |

*80% wettable powder formulation

TABLE E (Continued)

| TEST PLANT | Compound No. 3 | | |
|---|---|---|---|
| kg/ha | 0.002 | 0.02 | 0.12* |
| | | Post-Emergence | |
| Wheat | 0 | 1G | 2G |
| Barley | 0 | 2G | 2C, 4G |
| Wild Oats | 0 | 1G | 1G |
| Downy Brome | 2G | 3G | 4C, 5G |
| Cheatgrass | 4G | 1C, 5G | 8C, 5G |
| Blackgrass | 3C, 4G | 5C, 5G | 10C |
| Annual Bluegrass | 1C, 4G | 5C, 6G | 10C |
| Green Foxtail | 0 | 3G | 6C, 6G |
| Quackgrass | 0 | 3G | 4G |
| Italian Ryegrass | 0 | 1G | 1C, 3G |
| Ripgut Brome | 0 | 2G | 2C, 4G |
| Russian Thistle | 0 | 2C, 3G | 2C, 3G |
| Tansy Mustard | 7C, 7G | 10C | 10C |
| Smartweed | – | – | – |
| Jimhill Mustard | 10C | 10C | 10C |
| Kochia | 0 | 1G | 2C, 2G |
| Shepherd's Purse | 10C | 8C, 8G | 9C, 8G |
| *Matricaria inodora* | 6C, 5G | 8C, 8G | 9C, 8G |
| Black Nightshade | 2C, 2G | 3C, 3G | 4C, 5G |
| Yellow Rocket | 7C, 7G | 8C, 7G | 8C, 8G |
| Wild Mustard | 10C | 10C | 10C |
| Wild Buckwheat | 1C, 2G | 2C, 3G | 3C, 4G |

* 80% wettable powder formulation.

TABLE E (Continued)

Compound No.

5

| TEST PLANT kg/ha | 0.02 | 0.06 | 0.02 | 0.06 |
|---|---|---|---|---|
| | Pre-Emergence | | Post-Emergence | |
| Wheat | 0 | 0 | 0 | 0 |
| Barley | 1G | 3G | 0 | 1C, 2G |
| Wild Oats | 0 | 2C, 5G | 0 | 2G |
| Downy Brome | 1C, 2G | 5C, 7G | 0 | 3C, 3G |
| Cheatgrass | 2C, 6G | 6C, 7G | 2G | 7C, 8G |
| Blackgrass | 7C, 8G | 7C, 8G | 7C, 5G | 10C |
| Annual Bluegrass | 1C, 6G | 7G | 2G | 7C, 6G |
| Green Foxtail | 2C, 3G | 3C, 6G | 0 | 3C, 4G |
| Quackgrass | 3G | 5G | 0 | 3C, 5G |
| Italian Ryegrass | 2G | 4G | 0 | 3G |
| Ripgut Brome | 1C, 2G | 2C, 3G | 0 | 1C, 2G |
| Russian Thistle | 3G | 7C, 7G | 10C | 10C |
| Tansy Mustard | 10C | 10C | 10C | 10C |
| Smartweed | - | - | - | - |
| Jimhill Mustard | 8C, 9G | 9C, 9G | 10C | 10C |
| Kochia | 1C, 6G | 5C, 7G | 7C, 8G | 10C |
| Shepherd's Purse | 8C, 8G | 10C | 8C, 8G | 10C |
| False Chamomile | 2G | 6C, 7G | 7C, 7G | 10C |
| Black Nightshade | 6G | 6C, 7G | 6C, 7G | 10C |
| Yellow Rocket | 8C, 8G | 9C, 9G | 8C, 8G | 10C |
| Wild Mustard | 4C, 7G | 9C, 9G | 10C | 10C |
| Wild Buckwheat | 3C, 6G | 5C, 7G | 4C, 5G | 10C |

TABLE E (Continued)

Compound No.

5

TEST PLANT

| kg/ha | 0.02 | 0.06 | 0.02 | 0.06 |
|---|---|---|---|---|
| | Pre-Emergence | | Post-Emergence | |
| Wheat | 0 | 1C, 1G | 0 | 0 |
| Barley | 0 | 2C, 7G | 0 | 2C, 5G |
| Wild Oats | 1C, 2G | 2C, 5G | 0 | 1C, 2G |
| Downy Brome | 4G | 4C, 7G | 2G | 7C, 7G |
| Cheatgrass | 4C, 7G | 5C, 7G | 2C, 3G | 10C |
| Blackgrass | 10C | 7C, 8G | 7C, 6G | 10C |
| Annual Bluegrass | 6G | 7G | 1C,, 2G | 9C |
| Green Foxtail | 1G | 3C, 4G | 0 | 6C, 5G |
| Quackgrass | 0 | 2C, 7G | 0 | 2C, 4G |
| Italian Ryegrass | 0 | 2C, 7G | 0 | 1C, 3G |
| Ripgut Brome | 0 | 3C, 3G | 0 | 2C, 2G |
| Russian Thistle | – | – | – | – |
| Tansy Mustard | 10C | 10C | 10C | 10C |
| Smartweed | – | – | – | – |
| Jimhill Mustard | 2C, 6G | 10C | 10C | 10C |
| Kochia | 2C, 4G | 5C, 6G | 7C, 7G | 10C |
| Shepherd's Purse | 1C, 5G | 10C | 10C | 10C |
| Flase Chamomile | 3G | 5C, 8G | 7C, 6G | 10C |
| Black Nightshade | 1C, 2G | 4C, 7G | 4C, 6G | 10C |
| Yellow Rocket | 7G | 10C | 10C | 10C |
| Wild Mustard | 9C, 9G | 9C, 9G | 10C | 10C |
| Wild Buckwheat | 3C, 5G | 5C, 6G | 6C, 6G | 10C |

TABLE E (Continued)

| TEST PLANT | Compound No. 6 | | | |
|---|---|---|---|---|
| kg/ha | 0.02 | 0.06 | 0.02 | 0.06 |
| | Pre-Emergence | | Post-Emergence | |
| Wheat | 0 | 0 | 0 | 0 |
| Barley | 0 | 2G | 0 | 1G |
| Wild Oats | 0 | 3G | 0 | 1G |
| Downy Brome | 2G | 2C, 6G | 1C, 2G | 3C, 5G |
| Cheatgrass | 3G | 4C, 7G | 1C, 3G | 5C, 7G |
| Blackgrass | 3C, 4G | 7C, 7G | 5C, 6G | 7C, 8G |
| Annual Bluegrass | 3G | 2C, 7G | 3G | 4G |
| Green Foxtail | 1G | 1C, 3G | 0 | 1C, 2G |
| Quackgrass | 0 | 5G | 0 | 2C, 3G |
| Italian Ryegrass | 0 | 2G | 0 | 0 |
| Ripgut Brome | 0 | 1G | 0 | 0 |
| Russian Thistle | 2C, 3G | 7C, 8G | 10C | 10C |
| Tansy Mustard | 10C | 10C | 10C | 10C |
| Smartweed | – | – | – | – |
| Jimhill Mustard | 10C | 10C | 10C | 10C |
| Kochia | 2G | 2C, 5G | 3C, 4G | 4C, 7G |
| Shepherd's Purse | 8C, 9G | 9C, 9G | 7C, 7G | 10C |
| False Chamomile | 2G | 5C, 6G | 5C, 6G | 10C |
| Black Nightshade | 1C, 6G | 5C, 7G | 2C, 6G | 9C, 8G |
| Yellow Rocket | 7G | 4C, 7G | 0 | 2G |
| Wild Mustard | 5G | 8C, 8G | 2C, 3G | 7C, 6G |
| Wild Buckwheat | 2G | 2C, 3G | 1C, 2G | 4C, 5G |

TABLE E (Continued)

| TEST PLANT | Compound No. 6 | | | |
|---|---|---|---|---|
| kg/ha | 0.02 | 0.06 | 0.02 | 0.06 |
| | Pre-Emergence | | Post-Emergence | |
| Wheat | 0 | 1C, 1G | 0 | 0 |
| Barley | 0 | 2C, 3G | 2G | 1C, 3G |
| Wild Oats | 0 | 3C, 4G | 0 | 2G |
| Downy Brome | 2G | 7C, 7G | 2G | 3C, 4G |
| Cheatgrass | 1C, 7G | 7C, 8G | 1C, 3G | 10C |
| Blackgrass | 3C, 7G | 10C | 3C, 4G | 8C, 7G |
| Annual Bluegrass | 6G | 7G | 1G | 2C, 2G |
| Green Foxtail | 2G | 4C, 6G | 1C, 2G | 2C, 3G |
| Quackgrass | 2C, 3G | 3C, 6G | 1G | 1C, 2G |
| Italian Ryegrass | 1C, 2G | 2C, 3G | 1G | 1C, 2G |
| Ripgut Brome | 1G | 1C, 2G | 2G | 1C, 3G |
| Russian Thistle | - | - | - | - |
| Tansy Mustard | 10C | 10C | 10C | 10C |
| Smartweed | - | - | - | - |
| Jimhill Mustard | 3C, 6G | 10C | 8C | 10C |
| Kochia | 2G | 2C, 4G | 0 | 5C, 6G |
| Shepherd's Purse | 5C, 6G | 10C | 10C | 10C |
| False Chamomile | 1G | 7C, 8G | 8C, 8G | 9C, 8G |
| Black Nightshade | 1C, 2G | 2C, 4G | 2C, 4G | 7C, 8G |
| Yellow Rocket | 6G | 7G | 0 | 2C, 4G |
| Wild Mustard | 2C, 5G | 3C, 6G | 8C, 8G | 7C, 6G |
| Wild Buckwheat | 2C, 4G | 2C, 5G | 2C, 2G | 5C, 5G |

TABLE E (Continued)

| TEST PLANT | Compound No. 8 | | | |
|---|---|---|---|---|
| kg/ha | 0.02 | 0.06 | 0.02 | 0.06 |
| | Pre-Emergence | | Post-Emergence | |
| Wheat | 1C, 1G | 3C, 1G | 2G | 2C, 3G |
| Barley | 2C, 6G | 3C, 7G | 6C, 6G | 7C, 8G |
| Wild Oats | 6C, 6G | 6C, 6G | 4C, 5G | 8C, 8G |
| Downy Brome | 8C, 8G | 9C, 9G | 7C, 8G | 10C |
| Cheatgrass | 10C | 10C | 8C, 8G | 10C |
| Blackgrass | 10C | 10C | 8C, 8G | 10C |
| Annual Bluegrass | 8C, 7G | 8C, 8G | 7C, 7G | 9C, 8G |
| Green Foxtail | 7C, 6G | 7C, 8G | 4C, 3G | 7C, 6G |
| Quackgrass | 4C, 6G | 8C, 8G | 7C, 7G | 7C, 7G |
| Italian Ryegrass | 3C, 6G | 8C, 8G | 4C, 6G | 5C, 7G |
| Ripgut Brome | 5C, 7G | 6C, 8G | 3C, 5G | 5C, 7G |
| Russian Thistle | 2C, 4G | 10C | 8C, 7G | 9C, 8G |
| Tansy Mustard | 10C | 10C | 10C | 10C |
| Smartweed | – | – | – | – |
| Jimhill Mustard | 9C, 9G | 10C | 10C | 10C |
| Kochia | 5C, 6G | 5C, 7G | 4G | 4C, 5G |
| Shepherd's Purse | 9C, 9G | 10C | 9C, 7G | 10C |
| False Chamomile | 7C, 8G | 8C, 9G | 9C, 8G | 10C |
| Black Nightshade | 2C, 7G | 3C, 8G | 5G | 6G |
| Yellow Rocket | 9C, 9G | 9C, 9G | 9C, 7G | 9C, 8G |
| Wild Mustard | 9C, 9G | 9C, 9G | 10C | 10C |
| Wild Buckwheat | 2C, 4G | 5C, 6G | 7C, 5G | 7C, 7G |

TABLE E (Continued)

| TEST PLANT | Compound No. 9 | | | |
|---|---|---|---|---|
| kg /ha | 0.02 | 0.06 | 0.02 | 0.06 |
| | Pre-Emergence | | Post-Emergence | |
| Wheat | 1C, 2G | 2C, 2G | 1C, 2G | 2C, 2G |
| Barley | 3C, 7G | 6C, 8G | 8C, 7G | 8C, 8G |
| Wild Oats | 3C, 7G | 6C, 7G | 3C, 4G | 5C, 6G |
| Downy Brome | 8C, 7G | 10C | 8C, 8G | 10C |
| Cheatgrass | 10C | 10C | 10C | 10C |
| Blackgrass | 10C | 10C | 10C | 10C |
| Annual Bluegrass | 7C, 7G | 9C, 8G | 10C | 10C |
| Green Foxtail | 7C, 7G | 7C, 8G | 6C, 7G | 7C, 8G |
| Quackgrass | 8G | 8C, 8G | 7C, 6G | 9C, 7G |
| Italian Ryegrass | 2C, 8G | 7C, 8G | 10C | 10C |
| Ripgut Brome | 8G | 7C, 8G | 4C, 6G | 6C, 7G |
| Russian Thistle | 2G | 5C, 5G | 6C, 5G | 10C |
| Tansy Mustard | 10C | 10C | 10C | 10C |
| Smartweed | - | - | - | - |
| Jimhill Mustard | 10C | 10C | 10C | 10C |
| Kochia | 7G | 3C, 8G | 5C, 6G | 10C |
| Shepherd's Purse | 10C | 10C | 10C | 10C |
| False Chamomile | 7C, 8G | 9C, 9G | 10C | 10C |
| Black Nightshade | 5C, 8G | 6C, 8G | 8C, 8G | 10C |
| Yellow Rocket | 9C, 9G | 10C | 10C | 10C |
| Wild Mustard | 9C, 9G | 9C, 9G | 10C | 10C |
| Wild Buckwheat | 4C, 6G | 5C, 7G | 3C, 3G | 9C, 8G |

TABLE E (Continued)

| TEST PLANT | Compound No. 11 | | | |
|---|---|---|---|---|
| kg/ha | 0.02 | 0.06 | 0.02 | 0.06 |
| | Pre-Emergence | | Post-Emergence | |
| Wheat | 0 | 0 | 0 | 1C, 4G |
| Barley | 2C, 2G | 2C, 4G | 0 | 3C, 3G |
| Wild Oats | 2C, 3G | 3C, 6G | 0 | 6C, 7G |
| Downy Brome | 4C, 6G | 7C, 7G | 2C, 2G | 10C |
| Cheatgrass | 3C, 7G | 10C | 2C, 6G | 9C, 8G |
| Blackgrass | 7C, 8G | 10C | 3C, 7G | 10C |
| Annual Bluegrass | 7G | 7C, 8G | 7C, 7G | 10C |
| Green Foxtail | 2C, 4G | 5C, 8G | 3G | 7C, 7G |
| Quackgrass | 6G | 4C, 7G | 1C, 4G | 5C, 7G |
| Italian Ryegrass | 7G | 5C, 8G | 2C, 4G | 10C |
| Ripgut Brome | 3C, 3G | 3C, 6G | 0 | 2C, 3G |
| Russian Thistle | 3C, 3G | 8C, 7G | 10C | 10C |
| Tansy Mustard | 10C | 10C | 10C | 10C |
| Smartweed | - | - | - | - |
| Jimhill Mustard | 10C | 10C | 10C | 10C |
| Kochia | 3C, 7G | 5C, 8G | 5C, 5G | 10C |
| Shepherd's Purse | 10C | 10C | 10C | 10C |
| False Chamomile | 7C, 7G | 8C, 8G | 10C | 10C |
| Black Nightshade | 7C, 8G | 8C, 8G | 10C | 10C |
| Yellow Rocket | 10C | 9C, 9G | 10C | 10C |
| Wild Mustard | 9C, 9G | 9C, 9G | 10C | 10C |
| Wild Buckwheat | 7C, 7G | 8C, 8G | 8C, 7G | 10C |

TABLE E (Continued)

Compound No.

12

| TEST PLANT kg/ha | 0.02 | 0.06 | 0.02 | 0.06 |
|---|---|---|---|---|
| | Pre-Emergence | | Post-Emergence | |
| Wheat | 0 | 0 | 0 | 0 |
| Barley | 2G | 3G | 0 | 2G |
| Wild Oats | 3G | 6G | 0 | 1C, 3G |
| Downy Brome | 2C, 5G | 3C, 6G | 2G | 5C, 4G |
| Cheatgrass | 1C, 7G | 7C, 7G | 3G | 2C, 6G |
| Blackgrass | 5C, 7G | 8C, 8G | 2C, 2G | 5C, 7G |
| Annual Bluegrass | 5G | 4C, 7G | 2G | 3G |
| Green Foxtail | 1C, 2G | 2C, 5G | 0 | 1G |
| Quackgrass | 2G | 5G | 0 | 1C, 2G |
| Italian Ryegrass | 3G | 5G | 0 | 3G |
| Ripgut Brome | 2G | 3G | 0 | 0 |
| Russian Thistle | 2C, 2G | 2C, 3G | 2C, 3G | 10C |
| Tansy Mustard | 10C | 10C | 10C | 10C |
| Smartweed | – | – | – | – |
| Jimhill Mustard | 10C | 10C | 10C | 10C |
| Kochia | 2C, 5G | 3C, 6G | 0 | 7C, 7G |
| Shepherd's Purse | 9C, 9G | 9C, 9G | 10C | 10C |
| False Chamomile | 5C, 6G | 7C, 8G | 10C | 10C |
| Black Nightshade | 7C, 7G | 7C, 8G | 10C | 10C |
| Yellow Rocket | 7C, 8G | 7C, 8G | 0 | 1C, 2G |
| Wild Mustard | 9C, 9G | 9C, 9G | 10C | 10C |
| Wild Buckwheat | 7C, 8G | 7C, 8G | 4C, 3G | 8C, 7G |

*Test F*

The results obtained from this test demonstrate the high herbicidal activity of a compound selected from the scope of the invention when it is mixed with the water of simulated rice paddies.

The test samples were applied directly into the water of paddies three days after transplanting of M7 rice plants. The test was maintained in a greenhouse and plant response ratings were taken 34 days after application, utilizing the rating system as previously described.

TABLE F

Compound No.

3

| Treatment | Rice | Barnyardgrass | Water Chestnut | Arrowhead |
|---|---|---|---|---|
| 10 g/ha | 6G | 5E | 8G | 0 |
| 40 g/ga | 10G, 4C | 10E | 10G, 3C | 5F |

A field study was conducted to determine the crop selectivity and herbicidal activities of various chemicals. The test was conducted on a Wea silt loam having an organic matter content of 2.8 percent and a pH of 7.2.

The plots were planted to sugarbeets, oats, giant foxtail, wild oats, barley, rape, wheat, soybeans, velvetleaf, dry beans, corn and sunflower. The area had a natural infestation of pigweed.

The test included both pre- and post-emergence treatments. The pre-emergence treatments were applied directly after planting. The post-emergence treatments were applied 21 days after planting. Ratings were made on the percent control of both crops and weeds at various times after application.

*Pre-Emergence Test — Table G—I*

The *n*-propyl-dimethoxypyrimidine material showed crop selectivity on wheat, corn and soybeans while controlling giant foxtail, velvetleaf and pigweed at selected rates. The isopropyl-dimethoxypyrimidine material exhibited crop selectivity on wheat and corn. The compound controls giant foxtail and pigweed.

*Post-Emergence Test — Table G—II*

The isopropyl-dimethoxypyrimidine compound exhibited selectivity in all of the cereal crops tested (oats, barley and wheat) while some corn tolerance was noted. The compound provided control of broadleaf weeds including velvetleaf and pigweed.

The *n*-propyldimethoxypyrimidine exhibited selectivity in wheat, oats, corn and soybeans — it controlled pigweed and velvetleaf.

*Test G*

A field study was conducted to determine the crop selectivity and herbicidal activities of various chemicals. The test was conducted on a Wea silt loam having an organic matter content of 2.8 percent and a pH of 7.2.

The plots were planted to sugarbeets, oats, giant foxtail, wild oats, barley, rape, wheat, soybeans, velvetleaf, dry beans, corn and sunflower. The area had a natural infestation of pigweed.

The test included both pre- and post-emergence treatments. The pre-emergence treatments were applied directly after planting. The post-emergence treatments were applied 21 days after planting. Ratings were made on the percent control of both crops and weeds and various times after application.

*Pre-Emergence Test — Table G—I*

The *n*-propyl-dimethoxypyrimidine material showed crop selectivity on wheat, corn and soybeans while controlling giant foxtail, velvetleaf and pigweed at selected rates. The isopropyl-dimethoxypyrimidine material exhibited crop selectivity on wheat and corn. The compound controls giant foxtail and pigweed.

*Post-Emergence Test — Table G—II*

The isopropyl-dimethoxypyrimidine compound exhibited selectivity in all of the cereal crops tested

(oats, barley and wheat) while some corn tolerance was noted. The compound provided control of broadleaf weeds including velvetleaf and pigweed.

The *n*-propyldimethoxy-pyrimidine exhibited selectivity in wheat, oats, corn and soybeans- it controlled pigweed and velvetleaf.

TABLE G–I

PRE-EMERGENCE FIELD TEST

| | Compound No. | | | | | | | | |
|---|---|---|---|---|---|---|---|---|---|
| | 9 | | | | | | | | |
| kg ai / ha | 1/2 | | | 1/4 | | | 1/16 | | |
| | * | + | o | * | + | o | * | + | o |
| Sugarbeets | 8.0 | 8.5 | 7.0 | 9.0 | 9.0 | 9.0 | 8.0 | 8.0 | 6.0 |
| Oats | 5.5 | 5.0 | 5.5 | 4.0 | 5.0 | 3.0 | 2.0 | 1.5 | 1.0 |
| Giant Foxtail | 10.0 | 9.5 | 10.0 | 9.5 | 9.0 | 5.0 | 8.5 | 5.0 | 6.0 |
| Wild Oats | 6.5 | 8.0 | 6.5 | 5.0 | 6.0 | 5.0 | 4.0 | 2.5 | 2.5 |
| Barley | 7.5 | 8.5 | 8.0 | 5.0 | 6.0 | 2.5 | 5.0 | 4.0 | 2.5 |
| Rape | 9.0 | 10.0 | 10.0 | 9.0 | 10.0 | 10.0 | 8.5 | 10.0 | 10.0 |
| Wheat | 1.0 | 2.0 | 0.5 | 1.0 | 1.0 | 1.0 | 1.0 | 0.0 | 0.0 |
| Soybeans | 6.0 | 3.0 | 5.5 | 3.5 | 0.5 | 1.0 | 2.0 | 1.0 | 0.0 |
| Velvetleaf | 6.0 | 9.5 | 9.5 | 3.5 | 9.0 | 8.0 | 0.5 | 2.5 | 1.5 |
| Dry Beans | 5.5 | 8.5 | 9.0 | 4.0 | 7.5 | 5.0 | 1.5 | 2.5 | 1.5 |
| Corn | 3.0 | 2.5 | 4.0 | 1.0 | 0.5 | 1.0 | 1.5 | 0.0 | 1.0 |
| Sunflower | 10.0 | 10.0 | 9.5 | 8.0 | 9.0 | 8.5 | 9.5 | 9.5 | 9.5 |
| Pigweed | 10.0 | 10.0 | 10.0 | 9.5 | 10.0 | 9.5 | 9.5 | 10.0 | 9.5 |

* Averaged plant response of two replications taken 21 days after treatment.

\+ Averaged plant response of two replications taken 38 days after treatment.

o Averaged plant response of rwo replications taken 55 days after treatment.

Rating Scale – 0–10, 0 = no control, 10 = 100% control.

TABLE G-I (Continued)

PRE-EMERGENCE FIELD TEST

| Compound No. | 3 | | | | | | | | |
|---|---|---|---|---|---|---|---|---|---|
| kg ai /ha | 1/2 | | | 1/4 | | | 1/16 | | |
| | * | + | o | * | + | o | * | + | o |
| Sugarbeets | 9.0 | 10.0 | 10.0 | 9.0 | 9.5 | 8.5 | 9.5 | 9.0 | 8.0 |
| Oats | 6.0 | 6.5 | 6.0 | 4.0 | 4.0 | 2.5 | 2.5 | 2.0 | 1.5 |
| Giant Foxtail | 10.0 | 9.5 | 8.5 | 9.5 | 8.5 | 7.5 | 10.0 | 8.5 | 6.5 |
| Wild Oats | 7.0 | 7.0 | 5.5 | 5.0 | 5.0 | 3.0 | 2.0 | 2.0 | 2.0 |
| Barley | 6.0 | 7.0 | 4.0 | 7.0 | 6.5 | 4.0 | 4.5 | 4.5 | 3.0 |
| Rape | 9.5 | 10.0 | 10.0 | 9.5 | 9.5 | 10.0 | 9.5 | 10.0 | 9.5 |
| Wheat | 2.0 | 2.5 | 0.0 | 1.0 | 1.0 | 0.0 | 0.5 | 0.5 | 0.0 |
| Soybeans | 8.0 | 8.0 | 9.0 | 3.5 | 2.5 | 2.5 | 5.0 | 2.0 | 3.5 |
| Velvetleaf | 4.0 | 8.5 | 7.5 | 4.5 | 6.0 | 1.5 | 5.0 | 4.0 | 3.5 |
| Dry Beans | 5.0 | 7.0 | 4.0 | 4.0 | 4.5 | 2.0 | 4.5 | 4.0 | 2.5 |
| Corn | 4.5 | 4.0 | 3.5 | 1.0 | 1.0 | 1.5 | 3.0 | 0.5 | 0.5 |
| Sunflower | 10.0 | 10.0 | 10.0 | 8.5 | 9.0 | 8.5 | 4.0 | 9.5 | 8.0 |
| Pigweed | 10.0 | 10.0 | 10.0 | 1.00 | 10.0 | 9.5 | 10.0 | 10.0 | 9.0 |

* Averaged plant response of two replications taken 21 days after treatment.

\+ Averaged plant response of two replications taken 38 days after treatment.

o Averaged plant response of two replications taken 55 days after treatment.

Rating Scale – 0–10, 0 = no control, 10 = 100% control.

TABLE G–II

POST-EMERGENCE FIELD TEST

| | Compound No. | | | | | |
|---|---|---|---|---|---|---|
| | | | 3 | | | |
| kg ai /ha | 1/4 | 1/16 | 1/64 | 1/4 | 1/16 | 1/64 |
| | + | o | + | o | + | o |
| Sugarbeets | 8.5 | 6.5 | 8.5 | 6.0 | 5.5 | 2.5 |
| Oats | 2.5 | 1.5 | 2.0 | 1.0 | 0.5 | 0.0 |
| Giant Foxtail | 2.5 | 3.0 | 2.5 | 2.0 | 3.5 | 2.0 |
| Wild Oats | 3.5 | 1.0 | 2.5 | 0.5 | 2.5 | 0.0 |
| Barley | 6.5 | 1.5 | 3.0 | 1.0 | 2.5 | 2.0 |
| Rape | 9.0 | 10.0 | 8.5 | 8.0 | 7.5 | 4.5 |
| Wheat | 0.0 | 0.0 | 0.0 | 0.0 | 1.0 | 0.0 |
| Soybeans | 7.0 | 6.5 | 5.5 | 3.0 | 1.5 | 1.0 |
| Velvetleaf | 8.5 | 8.0 | 8.5 | 4.5 | 7.0 | 2.5 |
| Dry Beans | 8.0 | 7.0 | 8.0 | 5.0 | 7.5 | 3.5 |
| Corn | 4.0 | 4.5 | 4.0 | 2.5 | 1.0 | 1.0 |
| Sunflower | 9.0 | 10.0 | 8.5 | 8.5 | 8.0 | 5.5 |
| Pigweed | 9.5 | 8.5 | 9.0 | 7.5 | 7.0 | 5.0 |

+ Averaged plant response of two replications taken 17 days after treatment.

o Averaged plant response of two replications taken 34 days after treatment.

Rating scale 0–10, 0 = no control; 10 = 100% control.

TABLE G-II.(Continued)

POST-EMERGENCE FIELD TEST

Compound No.

9

| kg ai/ha | 1/4 | 1/16 | 1/64 | 1/4 | 1/16 | 1/64 |
|---|---|---|---|---|---|---|
| | + | o | + | o | + | o |
| Sugarbeets | 7.5 | 4.0 | 9.0 | 6.5 | 6.0 | 4.5 |
| Oats | 4.0 | 1.0 | 3.0 | 0.5 | 2.0 | 0.5 |
| Giant Foxtail | 6.5 | 3.5 | 7.0 | 6.5 | 4.5 | 4.5 |
| Wild Oats | 4.0 | 1.0 | 4.0 | 1.5 | 2.5 | 1.0 |
| Barley | 7.0 | 4.0 | 4.5 | 2.0 | 3.5 | 2.0 |
| Rape | 9.0 | 10.0 | 9.0 | 9.0 | 8.0 | 7.0 |
| Wheat | 1.0 | 0.0 | 0.0 | 0.0 | 1.0 | 0.0 |
| Soybeans | 4.0 | 2.5 | 5.0 | 2.0 | 1.5 | 0.5 |
| Velvetleaf | 10.0 | 10.0 | 9.5 | 8.5 | 8.5 | 5.0 |
| Dry Beans | 9.0 | 9.0 | 9.0 | 8.0 | 8.0 | 4.0 |
| Corn | 2.5 | 3.0 | 2.0 | 4.0 | 0.5 | 1.0 |
| Sunflower | 9.5 | 10.0 | 9.0 | 10.0 | 9.0 | 8.5 |
| Pigweed | 9.0 | 9.0 | 9.5 | 8.5 | 9.0 | 8.5 |

\+ Averaged plant response of two replications taken 17 days after treatment.

o Averaged plant response of two replications taken 34 days after treatment.

Rating Scale 0–10, 0 = no control; 10 = 100% control.

*Test H*

A field test site containing Matapeake silt loam soil with 10% organic matter content was plowed and disced in preparation for seeding. Just prior to final discing, 560 kg/hm$^2$ (500 lbs/a) of 10—10—10 fertilizer was applied to the soil. Twenty-eight crops and five weed species were drilled. Shortly after planting chemical treatments were applied to the soil surface of the plots. All treatments were replicated twice. Within five days after the application, 2.5 cm of water was applied to plots using overhead sprinkler irrigation. Evaluation of the effect of the chemicals began 30 days after application. The effect of the chemicals on crops and weeds was rated on a scale of 0—100 where 0 represented no effect and 100 represented complete kill. Untreated controls were included in the experiment for comparison. The results obtained are presented in Table H. It will be seen that certain compounds from the scope of the invention can be used for selective pre-emergence weed control in such crops as soybeans, corn, wheat, cotton, flax and lima beans.

TABLE H

PRE-EMERGENCE MULTICROP FIELD TEST

% EFFECT[1]

| | Compound No. | | | | | |
|---|---|---|---|---|---|---|
| | 3 | | | 9 | | |
| kg ai /ha | .031 | .062 | .125 | .062 | .125 | .250 |
| Safflower | 0 | 0 | 0 | 5 | 65 | 90 |
| Sunflower | 0 | 0 | 0 | 0 | 45 | 90 |
| Corn | 0 | 0 | 5 | 0 | 5 | 0 |
| Sorghum | 5 | 0 | 5 | 0 | 30 | 90 |
| Clover | 0 | 0 | 0 | 0 | 0 | 20 |
| Alfalfa | 0 | 0 | 0 | 0 | 10 | 30 |
| Soybeans | 0 | 0 | 5 | 0 | 0 | 0 |
| Sugarbeets | 0 | 0 | 0 | 0 | 0 | 30 |
| Rape | 0 | 0 | 5 | 15 | 85 | 100 |
| Squash | 0 | 0 | 0 | 0 | 0 | 60 |
| Potatoes | 0 | 0 | 5 | 0 | 15 | 40 |
| Cucumbers | 10 | 0 | 0 | 20 | 5 | 20 |
| Lespedela | 0 | 0 | 10 | 0 | 0 | 30 |
| Wheat | 0 | 0 | 0 | 0 | 0 | 0 |
| Rice | 45 | 0 | 10 | 0 | 0 | 70 |
| Okra | 0 | 0 | 5 | 0 | 0 | 10 |
| Oats | 0 | 0 | 0 | 0 | 0 | 60 |
| Cotton | 0 | 0 | 0 | 0 | 0 | 0 |
| Carrots | 0 | 0 | 0 | 0 | 5 | 80 |
| Red Beets | 0 | 0 | 0 | 5 | 5 | 20 |
| Cabbage | 0 | 0 | 0 | 0 | 35 | 90 |
| Endive | 0 | 0 | 10 | 0 | 0 | – |
| Flax | 5 | 0 | 0 | 0 | 0 | 0 |
| Spinach | 10 | 0 | 5 | 0 | 0 | 70 |
| Snap Beans | 10 | 0 | 5 | 0 | 0 | 0 |
| Tomatoe | 0 | 0 | 5 | 10 | 0 | 10 |
| Peanuts | – | – | – | – | – | – |
| Lima Beans | 0 | 0 | 0 | 15 | 0 | 0 |
| Velvetleaf | 0 | 0 | 0 | 0 | 0 | 70 |
| Wild Oats | 0 | 0 | 0 | 0 | 25 | 10 |
| Ryegrass | 0 | 0 | 5 | 5 | 35 | 20 |
| Barnyardgrass | 0 | 0 | 0 | 0 | 25 | 60 |
| Foxtail | 5 | 5 | 15 | 10 | 35 | 70 |
| Crabgrass | 0 | 0 | 0 | 0 | 30 | 70 |
| Ragweed | 10 | 0 | 5 | 5 | 35 | 70 |
| Lambsquarter | 15 | 0 | 25 | 10 | 25 | 60 |
| Smartweed | 0 | – | 10 | 0 | 30 | 70 |
| Purscane | – | 0 | 0 | – | – | – |
| Jimsonweed | 0 | – | – | 0 | 0 | 60 |
| Pigweed | 0 | 0 | – | 0 | – | 60 |
| Morningglory | 0 | 0 | 0 | 0 | 0 | 50 |
| Nutsedge | 0 | 0 | 0 | 0 | 0 | – |
| Flower-of-an Hour | 15 | 0 | 10 | 10 | 10 | 70 |

[1] 0 = no effect; 100 = complete kill.

*Test I*

A post-emergence field test was conducted using the same plantings as described in Test H. Twenty-one days after planting, chemical treatments were applied to emerged crops and weeds in each plot. All treatments were applied in water containing 0.1% surfactant and were replicated twice. Evaluation began 30 days after application. Again, the effect of a treatment on crops and weeds was rated on a scale of 0—100, where 0 = no effect and 100 = complete kill. Untreated controls were included in the experiment for comparison. The data are summarised in Table I.

The compounds tested provided excellent post-emergence weed control in a number of crops, including corn, wheat, oats, potatoes, flax and tomatoes.

TABLE I

POST-EMERGENCE MULTICROP FIELD TEST

% EFFECT[1]

| | Compound No. | | | | | |
|---|---|---|---|---|---|---|
| | | 3 | | | 9 | |
| kg ai /ha | .015 | .031 | .062 | .031 | .062 | .125 |
| Safflower | 85 | 90 | 95 | 90 | 100 | 100 |
| Sunflower | 90 | 90 | 90 | 95 | 100 | 100 |
| Corn | 0 | 20 | 45 | 0 | 0 | 0 |
| Sorghum | 90 | 90 | 90 | 95 | 90 | 95 |
| Clover | 25 | 50 | 65 | 65 | 75 | 85 |
| Alfalfa | 55 | 55 | 75 | 65 | 70 | 70 |
| Soybeans | 25 | 50 | 65 | 25 | 50 | 70 |
| Sugarbeets | 15 | 15 | 50 | 25 | 30 | 35 |
| Rape | 55 | 75 | 65 | 95 | 95 | 100 |
| Squash | 65 | 70 | 80 | 85 | 85 | 95 |
| Potatoes | 0 | 5 | 10 | 0 | 0 | 15 |
| Cucumbers | 30 | 45 | 45 | 5 | 10 | 35 |
| Lespedela | 70 | 65 | 70 | 70 | 70 | 95 |
| Wheat | 0 | 0 | 20 | 0 | 0 | 0 |
| Rice | 25 | 30 | 30 | 35 | 40 | 45 |
| Okra | 10 | 60 | 55 | 55 | 60 | 65 |
| Oats | 0 | 5 | 10 | 0 | 0 | 15 |
| Cotton | 45 | 50 | 70 | 80 | 70 | 70 |
| Carrots | 20 | 40 | 85 | 90 | 85 | 95 |
| Red Beets | 30 | 45 | 90 | 90 | 90 | 95 |
| Cabbage | 25 | 60 | 75 | 80 | 90 | 100 |
| Endive | 20 | – | – | – | – | – |
| Flax | 0 | 20 | 15 | 10 | 25 | 45 |
| Spinach | 45 | 90 | 90 | 90 | 95 | 100 |
| Snap Beans | 55 | 65 | 75 | 55 | 80 | 85 |
| Tomatoe | 5 | 0 | 10 | 0 | 5 | 15 |
| Peanuts | – | – | – | – | – | – |
| Lima Beans | 5 | 60 | 70 | 80 | 85 | 90 |
| Velvetleaf | 55 | 95 | 95 | 90 | 100 | 100 |
| Wild Oats | 10 | 10 | 15 | 5 | 40 | 30 |
| Ryegrass | 5 | 5 | 25 | 20 | 70 | 60 |
| Barnyardgrass | 0 | 0 | 10 | 80 | 90 | 90 |
| Foxtail | 10 | 5 | 35 | 5 | 40 | 50 |
| Crabgrass | – | 0 | 40 | – | 10 | – |
| Ragweed | 55 | 70 | 85 | 70 | 80 | 85 |
| Lambsquarter | 30 | 90 | 75 | 55 | 70 | 70 |
| Smartweed | – | – | 80 | 60 | 90 | 80 |
| Purscane | – | – | 90 | – | 90 | 85 |
| Jimsonweed | – | – | 30 | 0 | – | 0 |
| Pigweed | 40 | 90 | 85 | 80 | 90 | 85 |
| Morningglory | – | – | – | – | – | – |
| Nutsedge | – | – | – | 0 | – | – |
| Flower-of-an Hour | 70 | 80 | 75 | 70 | 90 | 80 |

[1] 0 = no effect; 100 = complete kill.

*Test J*

In a pre-emergence field test study, Matapeake silt loam soil with 1% organic matter content was plowed and disked in preparation for seeding. Just prior to final discing 560 kg/hm$^2$ (500 lbs/a) of 10—10—10 fertilizer was applied to the soil. Corn was planted into single row plots. Shortly after planting, chemical treatments were applied to the soil surface of the plots. All treatments were applied in water. The experiment was designed as a partially randomized complete block with 3 replications. The first replication was layed out in numerical sequence and not randomized. Evaluation of treatments began 43 days after application. The effect of a treatment on crops and indigenous weeds was rated on a scale of 0—100, where 0 = no effect and 100 = complete kill. Untreated controls were included in the experiment for comparison. The results obtained are given in Table J.

It will be apparent from Table J that the compound tested can be used for selective pre-emergence weed control in corn.

TABLE J

PRE-EMERGENCE CORN FIELD TEST

% EFFECT [1]

| | Compound No. 9 | | | | Untreated Control | |
|---|---|---|---|---|---|---|
| kg ai /ha | 0.031 | 0.062 | 0.125 | 0.250 | Cult. | Uncult. |
| Corn | 0 | 3 | 0 | 7 | 0 | 0 |
| Fall Panicum | 53 | 73 | 80 | 93 | — | 0 |
| Crabgrass | 43 | 57 | 70 | 90 | — | 0 |
| Yellow Foxtail | 53 | 63 | 80 | 90 | — | 0 |
| Purslane | 70 | 87 | 86 | 96 | — | 0 |
| Flower-of-an-Hour | 53 | 70 | 80 | 90 | — | 0 |
| Galinsoga | — | — | — | — | — | 0 |
| Velvetleaf | 33 | 57 | 90 | 90 | — | 0 |

[1] % effect: 0 = no effect; 100 = complete kill.

Average of 3 replications.

*Test K*

For a post-emergence field test, a site as described in Test J was selected. Twenty-one days after planting the corn chemical treatments were applied to the emerged crop and weeds in each plot. Each treatment included applications to both previously untreated plots and to plots which earlier had received a pre-emergence application of alachlor herbicide. All treatments were applied in water. The experiment was designed as a partially randomized complete block with 3 replications. The first replication was layed out in numerical sequence and not randomized. Evaluation of treatments began 39 days after application. The effect of a chemical on corn and indigenous weeds was rated on a sacle of 0—100, where 0 is no effect and 100 is complete kill. Untreated controls were included in the experiment for comparison. The data, shown in Table K, indicate the potential utility of a compound from the scope of the present invention for selective post-emergence weed control in corn, both when applied by itself or in conjuction with a prior pre-emergence treatment of alachlor.

TABLE K

POST-EMERGENCE CORN FIELD TEST

% EFFECT

| | Compound No. 9 | | | | | |
|---|---|---|---|---|---|---|
| kg ai /ha | 0.031 | 0.062 | 0.125 | 0.250 | Cult. | Uncult. |
| w/o Alachlor | | | | | | |
| Corn | 0 | 7 | 0 | 30 | 0 | 3 |
| Fall Panicum | 0 | 7 | 20 | 27 | – | 0 |
| Purslane | 37 | 60 | 53 | 70 | – | 0 |
| Galinsoga | – | – | – | – | – | 0 |
| Flower-of-an-Hour | – | – | – | 80 | – | 0 |
| with Alachlor | | | | | | |
| Corn | 0 | 3 | 0 | 23 | 0 | 0 |
| Fall Panicum | 100 | 100 | 100 | 97 | – | 100 |
| Purslane | 100 | 100 | 100 | 100 | – | 97 |
| Galinsoga | 100 | 100 | – | 100 | – | 100 |
| Flower-of-an-Hour | 95 | 100 | – | 100 | – | 87 |

[1] % Effect, 0 = no effect, 100 = complete kill.

*Test L*

This field test was conducted on a site as previously described for tests H through K. Wheat, wild oats and mustard were drilled into plots. Herbicide treatments were applied in the spring when wheat was fully tillered and 12—20 cm tall. All treatments were applied in water. The experiment was designed as a partially randomized complete block with 3 replications. The first replication was layed out in numerical sequence and not randomized. Evaluation of treatments began 25 days after application. The effect of a treatment on crops and weeds was rated on a scale of 0—100, where O = no effect and 100 = complete kill. Untreated controls were included in the experiment for comparison. The data, presented in Table L, indicate that the test compound provides control of certain weed species without injuring the crop when applied post-emergence to wheat.

TABLE L

POST-EMERGENCE SPRING WHEAT FIELD TEST

% EFFECT [1]

| | Compound No. 3 | | | | |
|---|---|---|---|---|---|
| kg ai /ha | .008 | .015 | .031 | .062 | Untreated Control |
| Wheat | 0 | 0 | 0 | 0 | 0 |
| Yellow Foxtail | 0 | 3 | 7 | 7 | 0 |
| Ragweed | 0 | 7 | 17 | 27 | 0 |
| Wild Oats | 0 | 0 | 0 | 0 | 0 |
| Velvetleaf | 3 | 7 | 3 | 3 | 0 |
| Flower-of-an-Hour | 3 | 10 | 3 | 3 | 0 |
| Mustard | 40 | 65 | – | – | 0 |
| Wheat Yield [2] | 169 | 223 | 190 | 259 | 203 |

[1] % Effect – 0 = no effect, 100 = complete kill;

Average of 3 replicates.

[2] Wheat Yield – Yield from all 4 rows /plot.

Data Average of 3 replicates.

*Test M*

For a pre-plant incorporated field test on winter wheat, a Keyport silt loam with 1% organic matter content was plowed and disked in preparation for fall seeding. Just prior to final disking 560 kg/hm$^2$ (500 lbs/a) of 10—10—10 fertilizer was applied to the field. Chemical treatments were then applied to the soil surface of plots. All chemicals were applied in water. Immediately after application the chemical was incorporated into the upper 5 cm of soil utilizing a rototille. Wheat, wild oats and wild mustard were then drilled into plots. The experiment was designed as a partially randomized complete block with 3 replications. The first replication was layed out in numerical sequence and not randomized. Evaluation of the treatments was made 31 days after application. The effect of a chemical on wheat and weeds was rated on a scale of 0—100, where 0 = no effect and 100 = complete kill. Untreated controls were included in the experiment for comparison. The test results are tabulated in Table L. The test chemicals used as pre-plant incorporated treatments provided excellent control of mustard without causing injury to the wheat crop.

TABLE M

PRE-PLANT INCORPORATED FIELD TEST ON WHEAT

% EFFECT[1]

(31 days after treatment)

| Compound No. | kg ai/ha | Wheat | Wild Oats | Wild Mustard |
|---|---|---|---|---|
| 3 | .062 | 0 | 0 | 60 |
| | .125 | 0 | 0 | 90 |
| | .250 | 0 | 3 | 90 |
| 9 | .062 | 0 | 0 | 90 |
| | .125 | 0 | 0 | 90 |
| | .250 | 3 | 3 | 90 |
| Untreated Control | – | 0 | 0 | 0 |

[1] % Effect – 0 = no effect, 100 = complete kill.

*Test N*

For a pre-emergence field test on winter wheat, a site as described in Test M was selected. Wheat, wild oats and wild mustard were drilled into the plots and herbicide treatments were applied to the soil surface of the plots immediately after planting. All treatments were applied in water. The experiment was designed as a partially randomized complete block with 3 replications. The first replication was layed out in numerical sequence and not randomized. Evaluation of treatments were made 31 days after application. The effect of a chemical on wheat and weeds was rated on a scale of 0—100, where 0 = no effect and 100 = complete kill. Untreated controls were included in the experiment for comparison. The ratings are presented in Table N.

The chemicals used in this test provide excellent pre-emergence weed control in wheat.

TABLE N

PRE-EMERGENCE FIELD TEST ON WHEAT

% EFFECT[1]

(31 days after treatment)

| Compound No. | kg ai/ha | Wheat | Wild Oats | Wild Mustard |
|---|---|---|---|---|
| 3 | .062 | 0 | 0 | 83 |
| | .125 | 0 | 0 | 90 |
| | .250 | 0 | 0 | 90 |
| 9 | .062 | 3 | 0 | 90 |
| | .125 | 0 | 0 | 90 |
| | .250 | 3 | 10 | 90 |
| Untreated Control | – | 0 | 0 | 0 |

[1] % Effect – 0 = no effect, 100 = complete kill.

**Claims for the Contracting States: BE CH DE FR GB IT LI LU NL SE**

1. A compound selected from

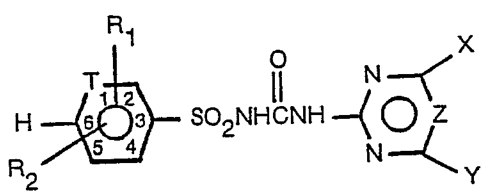

(I)

wherein

$R_1$ is $R_3S[O]_n$;
$R_3$ is $C_1$—$C_4$ alkyl, $C_3$—$C_4$ alkenyl, cyclopentyl or cyclopropylmethyl;
$R_2$ is H, F, Cl, Br, $CH_3$, $OCH_3$, $CF_3$, $NO_2$, CN or $NH_2$;
n is 0, 1 or 2;
W is 0 or S;
T is CH or N;
Z is CH or N;
X is $CH_3$, $C_2H_5$, $OCH_3$, $OC_2H_5$, or $CH_2OCH_3$; and
Y is $CH_3$ or $OCH_3$;

and their agriculturally suitable salts; provided that

(1) when $R_2$ is CN, then T is CH and $R_2$ is not ortho to the sulfonylurea group;
(2) when W is S, then n is 0 or 2
(3) when T is N, then n is 2;
(4) when T is CH, then $R_3$ is not $C_1$—$C_2$ alkyl and $R_1$ is at the 2- or 4-position; and
(5) when T is N, then $R_3$ is not cyclopropylmethyl and $R_2$ is not $NH_2$, $CF_3$ or $NO_2$.

2. A compound of Claim 1 wherein W is 0 and $R_2$ is H, F, Cl, Br, $CH_3$ or $OCH_3$.

3. A compound of Claim 2 wherein $R_1$ is at the 2- or 4-position when T is N.
4. A compound of Claim 3 wherein n is 2.
5. A compound of Claim 4 wherein $R_2$ is H.
6. A compound of Claim 5 wherein X is $CH_3$ or $OCH_3$.
7. A compound of Claim 6 wherein $R_3$ is $C_1$—$C_4$ alkyl.
8. The compounds of Claim 1, which are
N-[(4,6-dimethylpyrimidin-2-yl)aminocarbonyl]-2-[(1-methylethyl)sulfonyl]benzenesulfonamide,
N-[(4-methoxy-6-methylpyrimidin-2-yl)aminocarbonyl]-2-[(1-methylethyl)sulfonyl]benzenesulfonamide,
N-[(4,6-dimethoxypyrimidin-2-yl)aminocarbonyl]-2-[(1-methylethyl)sulfonyl]benzenesulfonamide,
N-[(4,6-dimethylpyrimidin-2-yl)aminocarbonyl]-2-(propylsulfonyl)benzenesulfonamide,
N-[(4-methoxy-6-methylpyrimidin-2-yl)aminocarbonyl]-2-(propylsulfonyl)benzenesulfonamide,
N-[(4,6-dimethoxypyrimidin-2-yl)aminocarbonyl]-2-(propylsulfonyl)benzenesulfonamide,
N-[(4,6-dimethyl-1,3,5-triazin-2-yl)aminocarbonyl]-2-(propylsulfonyl)benzenesulfonamide; and their agriculturally suitable salts.
9. The compounds of Claim 1 which are
N-[(4-methoxy-6-methyl-1,3,5-triazin-2-yl)aminocarbonyl]-2-(propylsulfonyl)benzenesulfonamide,
N-[(4,6-dimethoxy-1,3,5-triazin-2-yl)aminocarbonyl]-2-(propylsulfonyl)benzenesulfonamide,
N-[(4,6-dimethyl-1,3,5-triazin-2-yl)aminocarbonyl]-2-[(1-methylethyl)sulfonyl]benzenesulfonamide,
N-[(4-methoxy-6-methyl-1,3,5-triazin-2-yl)aminocarbonyl]-2-[(1-methylethyl)sulfonyl]benzenesulfonamide,
N-[(4,6-dimethoxy-1,3,5-triazin-2-yl)aminocarbonyl]-2-[(1-methylethyl)sulfonyl]benzenesulfonamide; and their agriculturally suitable salts.
10. The compounds of Claim 1 which are
N-[(4,6-dimethylpyrimidin-2-yl)aminocarbonyl]-2-(methylsulfonyl)-3-pyridinesulfonamide,
N-[(4-methoxy-6-methylpyrimidin-2-yl)aminocarbonyl]-2-(methylsulfonyl)-3-pyridinesulfonamide,
N-[(4,6-dimethoxy-1,3,5-triazin-2-yl)aminocarbonyl]-2-(methylsulfonyl)-3-pyridinesulfonamide,
N-[(4,6-dimethoxypyrimidin-2-yl)aminocarbonyl]-2-(methylsulfonyl)-3-pyridinesulfonamide,
N-[(4,6-dimethyl-1,3,5-triazin-2-yl)aminocarbonyl]-2-(methylsulfonyl)-3-pyridinesulfonamide, and
N-[(4-methoxy-6-methyl-1,3,5-triazin-2-yl)aminocarbonyl]-2-(methylsulfonyl)-3-pyridinesulfonamide; and their agriculturally suitable salts.
11. A compound of claim 1 wherein $R_1$ is $R_3SO_2$; $R_3$ is $C_1$—$C_4$ alkyl, $C_{3-4}$ alkenyl or cyclopentyl; $R_2$ is H, F, Cl, Br, $CH_3$ or $OCH_3$; and T is N.
12. A compound of claim 1 wherein:

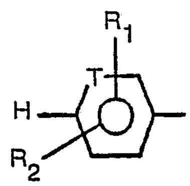 is 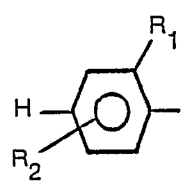 ;

$R_3$ is $C_3$ or $C_4$ alkyl or alkenyl.
13. A compound of claim 1 which is selected from
N-[(4,6-dimethylpyrimidin-2-yl)aminocarbonyl]-2-(allylthio)benzenesulfonamide;
N-[(4,6-dimethyl-1,3,5-triazin-2-yl)aminocarbonyl)-2-(allylsulfonyl)benzenesulfonamide;
N-[(4-methoxy-6-methyl-1,3,5-triazin-2-yl)aminocarbonyl]-2-(allylsulfonyl)benzenesulfonamide;
N-[(4,6-dimethoxy-1,3,5-triazin-2-yl)aminocarbonyl]-2-(allylsulfonyl)benzenesulfonamide;
N-[(4,6-dimethyl-1,3,5-triazin-2-yl)aminocarbonyl]-2-(3-buten-2-ylsulfonyl)benzenesulfonamide;
N-[(4-methoxy-6-methyl-1,3,5-triazin-2-yl)aminocarbonyl]-2-(3-buten-2-ylsulfonyl)benzenesulfonamide; or
N-[(4,6-dimethoxy-1,3,5-triazin-2-yl)aminocarbonyl]-2-(3-buten-2-ylsulfonyl)benzenesulfonamide.
14. A composition suitable for controlling the growth of undesired vegetation which comprises an effective amount of a herbicidal compound and at least one of the following: surfactant, solid or liquid diluent, characterised in that said herbicidal compound comprises a compound of any of claims 1 to 13.
15. A method for controlling the growth of undesired vegetation by applying to the locus to be protected an effective amount of a herbicidal compound, characterised in that said herbicidal compound comprises a compound of any of claims 1 to 13.
16. The method of claim 15 wherein said undesired vegetation is selectively controlled in the presence of crop plants.
17. A method for regulating the growth of plants by applying to the locus of said plants an effective but substantially non-phytotoxic amount of a plant growth regulant, characterised in that said plant growth regulant comprises a compound of any of claims 1 to 13.
18. A process for preparing a compound of claim 1 which comprises
(a) reacting together compounds of general formulae

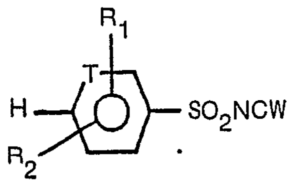

and

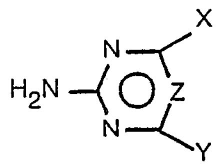

wherein
$R_1$ is $R_3SO_2$ or $R_3S$,
$R_2$ is as defined in claim 1, other than $NH_2$,
$R_3$, T, W, X, Y and Z are as defined in claim 1; or
(b) reacting together compounds of general formulae

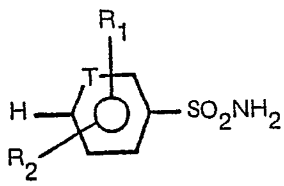

and

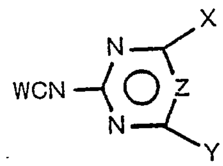

wherein $R_1$ is $R_3S$ or $R_3SO_2$ and $R_2$, T, W, X, Y, and Z are as defined in claim 1.

19. A process for preparing a compound of claim 1 wherein $R_2$ is $NH_2$, which comprises reducing a corresponding compound wherein $R_2$ is $NO_2$.

20. A process for preparing a compound of claim 1 wherein n is 1 and W is 0, which comprises oxidizing a corresponding compound wherein n is zero.

**Claims for the Contracting State: AT**

1. A process for the preparation of a compound selected from

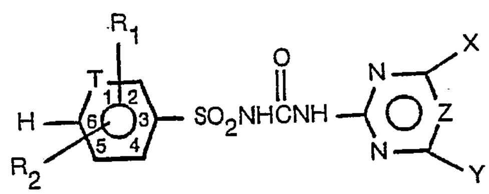

(I)

wherein
$R_1$ is $R_3S[O]_n$;
$R_3$ is $C_1$—$C_4$ alkyl, $C_3$—$C_4$ alkenyl, cyclopentyl or cyclopropylmethyl;
$R_2$ is H, F, Cl, Br, $CH_3$, $OCH_3$, $CF_3$, $NO_2$, CN or $NH_2$;
n is 0, 1 or 2;
W is 0 or S;
T is CH or N;
Z is CH or N;
X is $CH_3$, $C_2H_5$, $OCH_3$, $OC_2H_5$, or $CH_2OCH_3$; and
Y is $CH_3$ or $OCH_3$;
and their agriculturally suitable salts; provided that
(1) when $R_2$ is CN, then T is CH and $R_2$ is not ortho to the sulfonylurea group;
(2) when W is S, then n is 0 or 2
(3) when T is N, then n is 2;
(4) when T is CH, then $R_3$ is not $C_1$—$C_2$ alkyl and $R_1$ is at the 2- or 4-position; and
(5) when T is N, then $R_3$ is not cyclopropylmethyl and $R_2$ is not $NH_2$, $CF_3$ or $NO_2$;
which comprises:
(a) reacting together compounds of general formula

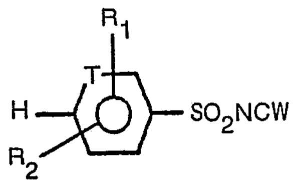 and 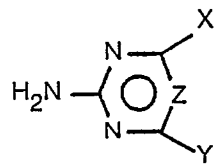

wherein

$R_1$ is $R_3SO_2$ or $R_3S$,

$R_2$ is as defined above, other than $NH_2$,

$R_3$, T, W, Y and Z are as defined above;

(b) reacting together compounds of general formulae

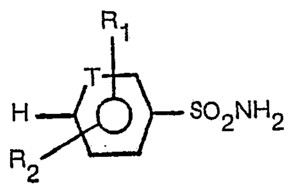 and 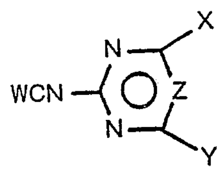

wherein $R_1$ is $R_3S$ or $R_3SO_2$ and $R_2$, T, W, X, Y and Z are as defined above;

(c) to prepare a compound of formula (I) wherein $R_2$ is $NH_2$, reducing a corresponding compound wherein $R_2$ is $NO_2$; or

(d) to prepare a compound of formula (I) wherein n is 1 and W is 0, oxidizing a corresponding compound wherein n is zero.

2. A process of claim 1 wherein $R_2$ is $R_2SO_2$; $R_3$ is $C_1$—$C_4$ alkyl, $C_{3-4}$ alkenyl or cyclopentyl; $R_2$ is H, F, Cl, Br, $CH_3$ or $OCH_3$; and T is N:

3. A process of claim 1 wherein:

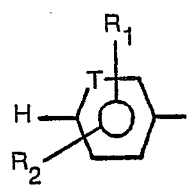 is 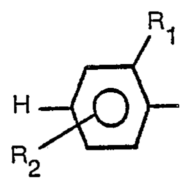 ; and

$R_3$ is $C_3$ or $C_4$ alkyl or alkenyl.

4. A process of claim 1, wherein W is 0 and $R_2$ is H, F, Cl, Br, $CH_3$ or $OCH_3$.

5. A process of claim 4 wherein $R_1$ is at the 2- or 4- position when T is N.

6. A process of claim 5 wherein n is 2.

7. A process of claim 6 wherein $R_2$ is H.

8. A process of claim 7 wherein X is $CH_3$ or $OCH_3$.

9. A process of claim 8 wherein $R_3$ is $C_1$—$C_4$ alkyl.

10. A process of claim 1, wherein the product is a compound selected from

N-[(4,6-dimethylpyrimidin-2-yl)aminocarbonyl]-2-[(1-methylethyl)sulfonyl]benzenesulfonamide,

N-[(4-methoxy-6-methylpyrimidin-2-yl)aminocarbonyl]-2-[(1-methylethyl)sulfonyl]benzenesulfonamide,

N-[(4,6-dimethoxypyrimidin-2-yl)aminocarbonyl]-2-[(1-methylethyl)sulfonyl]benzenesulfonamide,

N-[(4,6-dimethylphrimidin-2-yl)aminocarbonyl]-2-(propylsulfonyl)benzenesulfonamide,

N-[(4-methoxy-6-methylpyrimidin-2-yl)aminocarbonyl]-2-(propylsulfonyl)benzenesulfonamide,

N-[(4,6-dimethoxypyrimidin-2-yl)aminocarbonyl]-2-(propylsulfonyl)benzenesulfonamide,

N-[(4,6-dimethyl-1,3,5-triazin-2-yl)aminocarbonyl]-2-(propylsulfonyl)benzenesulfonamide; or an agriculturally suitable salt of any of these.

11. The process of claim 1 wherein the product is a compound selected from

N-[(4-methoxy-6-methyl-1,3,5-triazin-2-yl)aminocarbonyl]-2-(propylsulfonyl)benzenesulfonamide,

N-[(4,6-dimethoxy-1,3,5-triazin-2-yl)aminocarbonyl]-2-(propylsulfonyl)benzenesulfonamide,

N-[(4,6-dimethyl-1,3,5-triazin-2-yl)aminocarbonyl]-2-[(1-methylethyl)sulfonyl]benzenesulfonamide,

N-[(4-methoxy-6-methyl-1,3,5-triazin-2-yl)aminocarbonyl]-2-[(1-methylethyl)sulfonyl]benzenesulfonamide,

N-[(4,6-dimethoxy-1,3,5-triazin-2-yl)aminocarbonyl]-2-[(1-methylethyl)sulfonyl]benzenesulfonamide; or an agriculturally suitable salt of any of these.

12. A process of claim 1 wherein the product is a compound selected from
N-[(4,6-dimethylpyrimidin-2-yl)aminocarbonyl]-2-(methylsulfonyl)-3-pyridinesulfonamide,
N-[(4-methoxy-6-methylpyrimidin-2-yl)aminocarbonyl]-2-(methylsulfonyl)-3-pyridinesulfonamide,
N-[(4,6-dimethoxy-1,3,5-triazin-2-yl)aminocarbonyl]-2-(methylsulfonyl)-3-pyridinesulfonamide,
N-[(4,6-dimethoxypyrimidin-2-yl)aminocarbonyl]-2-(methylsulfonyl)-3-pyridinesulfonamide,
N-[(4,6-dimethyl-1,3,5-triazin-2-yl)aminocarbonyl]-2-(methylsulfonyl)-3-pyridinesulfonamide, and
N-[(4-methoxy-6-methyl-1,3,5-triazin-2-yl)aminocarbonyl]-2-(methylsulfonyl)-3-pyridinesulfonamide;
or an agriculturally suitable salt of any of these.

13. A method for controlling the growth of undesired vegetation by applying to the locus to be protected an effective amount of a herbicidal compound, characterised in that said herbicidal compound comprises a compound of formula (I) as defined in any of claims 1 to 12.

14. A method for regulating the growth of plants by applying to the locus of said plants an effective but substantially non-phytotoxic amount of a plant growth regulant, characterised in that said plant growth regulant comprises a compound of formula (I) as defined in any of claims 1 to 12.

**Patentansprüche für die Vertragsstaaten: BE CH LI DE FR GB IT LU NL SE**

1. Verbindung, ausgewählt aus

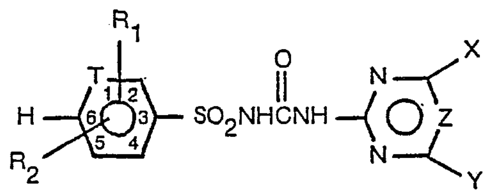

(I)

worin
$R_1$ $R_3S[O]_n$;
$R_3$ $C_1$—$C_4$-Alkyl, $C_3$—$C_4$-Alkenyl, Cyclopentenyl oder Cyclopropylmethyl;
$R_2$ H, F, Cl, Br, $CH_3$, $OCH_3$, $CF_3$, $NO_2$, CN oder $NH_2$;
n 0, 1 oder 2;
W O oder S;
T CH oder N;
Z CH oder N;
X $CH_3$, $C_2H_5$, $OCH_3$, $OC_2H_5$ oder $CH_2OCH_3$; und
Y $CH_3$ oder $OCH_3$ ist;
und ihre landwirtschaftlich geeigneten Salze; mit der Massgabe dass
(1) wenn $R_2$ CN ist, T CH und $R_2$ nicht orthoständig zur Sulfonylharnstoffgruppe ist;
(2) wenn W S ist, dann ist n 0 oder 2;
(3) wenn T N ist, dann ist n 2;
(4) wenn T CH ist, dann ist $R_3$ nicht $C_1$—$C_2$-Alkyl und $R_1$ ist in der 2- oder 4-Stellung; und
(5) wenn T N ist, dann ist $R_3$ nicht Cyclopropylmethyl und $R_2$ nicht $NH_2$, $CF_3$ oder $NO_2$.

2. Verbindung nach Anspruch 1, in der W = O und $R_2$ = H, F, Cl, Br, $CH_3$ oder $OCH_3$.

3. Verbindung nach Anspruch 2, worin $R_1$ in der 2- oder 4-Stellung ist, wenn T n ist.

4. Verbindung nach Anspruch 3, worin n = 2.

5. Verbindung nach Anspruch 4, worin $R_2$ = H.

6. Verbindung nach Anspruch 5, worin X = $CH_3$ oder $OCH_3$.

7. Verbindung von Anspruch 6, worin $R_3$ = $C_1$—$C_4$-Alkyl.

8. Die Verbindung von Anspruch 1, nämlich
N-[(4,6-Dimethylpyrimidin-2-yl)-aminocarbonyl]-2-[(1-methylethyl)-sulfonyl]-benzolsulfonamid,
N-[(4-Methoxy-6-methylpyrimidin-2-yl)-aminocarbonyl]-2-[(1-methylethyl)-sulfonyl]-benzolsulfonamid,
N-[(4,6-Dimethoxypyrimidin-2-yl)-aminocarbonyl]-2-[(1-methylethyl)-sulfonyl]-benzolsulfonamid,
N-[(4,6-Dimethylpyrimidin-2-yl)-aminocarbonyl]-2-(propylsulfonyl)-benzolsulfonamid,
N-[(4-Methoxy-6-methylpyrimidin-2-yl)-aminocarbonyl]-2-(propylsulfonyl)-benzolsulfonamid,
N-[(4,6-Dimetoxypyrimidin-2-yl)-aminocarbonyl]-2-(propylsulfonyl)-benzolsulfonamid,
N-[(4,6-Dimethyl-1,3,5-triazin-2-yl)-aminocarbonyl]-2-(propylsulfonyl)-benzolsulfonamid;
und ihre landwirtschaftlich brauchbaren Salze.

9. Die Verbindungen nach Anspruch 1, nämlich
N-[(4-Methoxy-6-methyl-1,3,5-triazin-2-yl)-aminocarbonyl]-2-(propylsulfonyl)-benzolsulfonamid,
N-[(4,6-Dimethoxy-1,3,5-triazin-2-yl)-aminocarbonyl]-2-(propylsulfonyl)-benzolsulfonamid,
N-[(4-Dimethyl-1,3,5-triazin-2-yl)-aminocarbonyl]-2-[(1-methylethyl)-sulfonyl]-benzolsulfonamid,
N-[(4-Methoxy-6-methyl-1,3,5-triazin-2-yl)-aminocarbonyl]-2-[(1-methylethyl)-sulfonyl]-benzolsulfonamid,

N-[(4,6-Dimethoxy-1,3,5-triazin-2-yl)-aminocarbonyl]-2-[(1-methylethyl)-sulfonyl]-benzolsulfonamid, und ihre landwirtschaftlich brauchbaren Salze.

10. Die Verbindungen von Anspruch 1, nämlich
N-[(4,6-Dimethylpyrimidin-2-yl)-aminocarbonyl]-2-(methylsulfonyl)-3-pyridinsulfonamid,
N-[(4-Methoxy-6-methylpyrimidin-2-yl)-aminocarbonyl]-2-(methylsulfonyl)-3-pyridinsulfonamid,
N-[(4,6-Dimethoxy-1,3,5-triazin-2-yl)-aminocarbonyl]-2-(methylsulfonyl)-3-pyridinsulfonamid,
N-[(4,6-Dimethoxypyrimidin-2-yl)-aminocarbonyl]-2-(methylsulfonyl)-3-pyridinsulfonamid,
N-[(4,6-Dimethyl-1,3,5-triazin-2-yl)-aminocarbonyl]-2-(methylsulfonyl)-3-pyridinsulfonamid; und
N-[(4-Methoxy-6-methyl-1,3,5-triazin-2-yl)-aminocarbonyl]-2-(methylsulfonyl)-3-pyridinsulfonamid;
und ihre landwirtschaftlich brauchbaren Salze.

11. Verbindung von Anspruch 1, worin R = $R_3SO_2$; $R_3$ = $C_1$—$C_4$-Alkyl, $C_3$—$C_4$-Alkenyl oder Cyclopentyl; $R_2$ = H, F, Cl, Br, $CH_3$ oder $OCH_3$; und T = N.

12. Verbindung nach Anspruch 1, worin

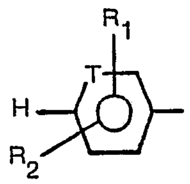
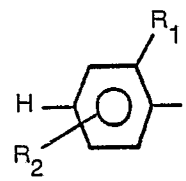 ist;

und $R_3$ = $C_3$— oder $C_4$-Alkyl oder -Alkenyl.

13. Verbindung nach Anspruch 1, ausgewählt aus
N-[(4,6-Dimethylpyrimidin-2-yl)-aminothiocarbonyl]-2-(allylthio)benzolsulfonamid;
N-[(4,6-Dimethyl-1,3,5-triazin-2-yl)-aminocarbonyl]-2-(allylsulfonyl)-benzolsulfonamid;
N-[(4-Methoxy-6-methyl-1,3,5-triazin-2-yl)-aminocarbonyl]-2-(allylsulfonyl)-benzolsulfonamid;
N-[(4,6-Dimethoxy-1,3,5-triazin-2-yl)-aminocarbonyl]-2-(allylsulfonyl)-benzolsulfonamid;
N-[(4,6-Dimethyl-1,3,5-triazin-2-yl)-aminocarbonyl]-2-(3-buten-2-ylsulfonyl)benzolsulfonamid;
N-[(4-Methoxy-6-methyl-1,3,5-triazin-2-yl)-aminocarbonyl]-2-(3-buten-2-ylsulfonyl)-benzolsulfonamid;
oder
N-[(4,6-Dimethoxy-1,3,5-triazin-2-yl)-aminocarbonyl]-2-(3-buten-2-yl-sulfonyl)-benzolsulfonamid.

14. Zusammensetzung, die für die Kontrolle des Wachstums unerwünschter Vegetation geeignet ist, enthaltend eine wirksame Menge einer herbiziden Verbindung und mindestens einen der folgenden Zusätze: oberflächenaktives Mittel, festes oder flüssiges Verdünnungsmittel, dadurch gekennzeichnet, dass die herbizide Verbindung eine Verbindung nach einem der Ansprüche 1 bis 13 enthält.

15. Verfahren zur Kontrolle des Wachstums von unerwünschter Vegetation durch Aufbringung einer wirksamen Menge einer herbiziden Verbindung auf die zu schützende Stelle, dadurch gekennzeichnet, dass die herbizide Verbindung eine Verbindung nach einem der Ansprüche 1 bis 13 enthält.

16. Verfahren nach Anspruch 15, bei dem die unerwünschte Vegetation in Gegenwart von Kulturpflanzen selektiv kontrolliert wird.

17. Verfahren zur Regulierung des Wachstums von Pflanzen durch Aufbringung einer wirksamen, aber im wesentlichen nicht-phytotoxischen Menge eines Wachstumsregulators auf die betreffende Stelle, dadurch gekennzeichnet, dass der Wachstumsregulator eine Verbindung nach einem der Ansprüche 1 bis 13 enthält.

18. Verfahren zur Herstellung einer Verbindung von Anspruch 1, bei dem man
(a) Verbindung der allgemeinen Formeln

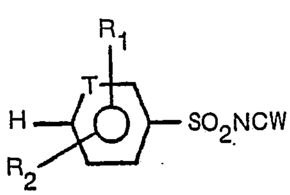
und
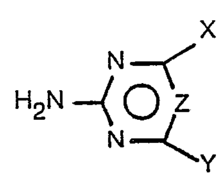

worin $R_1$ = $R_3SO_2$ oder $R_3S$, $R_2$ = wie in Anspruch 1 definiert, ausser $NH_2$, $R_3$, T, W, X, Y und Z wie in Anspruch 1 definiert sind, miteinander zur Umsetzung bringt; oder
(b) zur Herstellung einer Verbindung von Anspruch 1, in der $R_2$ $NH_2$ ist, eine entsprechende Verbindung, in der $R_2$ $NO_2$ ist, reduziert; oder
(c) Verbindung der allgemeinen Formeln

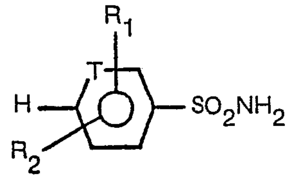

und

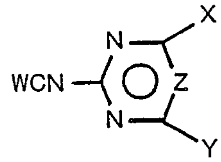

worin $R_1$ $R_3S$ oder $R_3SO_2$ und $R_2$, T, W, X, Y und Z wie in Anspruch 1 definiert sind, miteinander zur Umsetzung bringt.

19. Verfahren zur Herstellung einer Verbindung von Anspruch 1, in der $R_2$ $NH_2$ ist, dadurch gekennzeichnet, dass man eine entsprechende Verbindung, in der $R_2$ $NO_2$ ist, reduziert.

20. Verfahren zur Herstellung einer Verbindung von Anspruch 1, worin n 1 und W 0 ist, bei dem man eine entsprechende Verbindung in der N Null ist, oxidiert.

**Patentansprüche für den Vertragsstaat: AT**

1. Verfahren zur Herstellung einer Verbindung, ausgewählt aus

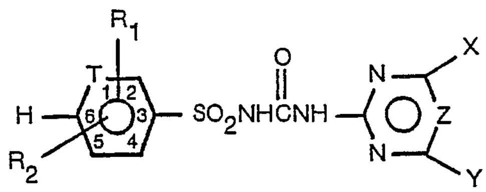

(I)

worin
$R_1$ $R_3S[O]_n$;
$R_3$ $C_1$—$C_4$-Alkyl, $C_3$—$C_4$-Alkenyl, Cyclopentenyl oder Cyclopropylmethyl;
$R_2$ H, F, Cl, Br, $CH_3$, $OCH_3$, $CF_3$, $NO_2$, CN oder $NH_2$;
n 0, 1 oder 2;
W O oder S;
T CH oder N;
Z CH oder N;
X $CH_3$, $C_2H_5$, $OCH_3$, $OC_2H_5$ oder $CH_2OCH_3$; und
Y $CH_3$ oder $OCH_3$ ist;
und ihre landwirtschaftlich geeigneten Salze; mit der Massgabe dass
(1) wenn $R_2$ CN ist, T CH und $R_2$ nicht orthoständig zur Sulfonylharnstoffgruppe ist;
(2) wenn W S ist, dann ist n 0 oder 2;
(3) wenn T N ist, dann ist n 2;
(4) wenn T CH ist, dann ist $R_3$ nicht $C_1$—$C_2$-Alkyl und $R_1$ ist in der 2- oder 4-Stellung; und
(5) wenn T N ist, dann ist $R_3$ nicht Cyclopropylmethyl und $R_2$ nicht $NH_2$, $CF_3$ oder $NO_2$;
bei dem man
(a) Verbindung der allgemeinen Formeln

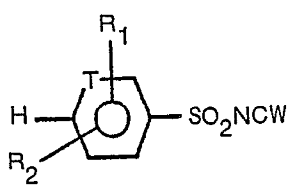

und

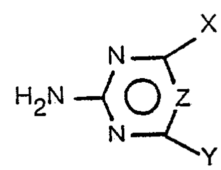

worin
$R_1$ $R_3SO_2$ oder $R_3S$ ist,
$R_2$ wie oben definiert ausser $NH_2$ ist,
$R_3$, T, W, X, Y und Z wie oben definiert sind, miteinander zur Reaktion bringt;
(b) Verbindung der allgemeinen Formeln

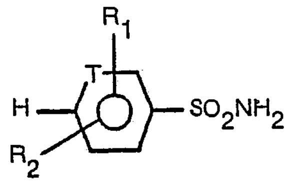

und

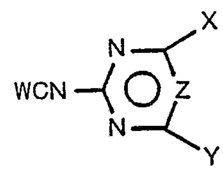

worin $R_1$ $R_3S$ oder $R_3SO_2$ und $R_2$, T, W, X, Y und Z wie oben definiert sind, miteinander zur Reaktion bringt;

(c) zur Herstellung einer Verbindung der Formel (I), in der $R_2$ $NH_2$ ist, eine entsprechende Verbindung, in der $R_2$ $NO_2$ ist, reduziert; oder

(d) zur Herstellung einer Verbindung der Formel (I), in der n 1 und W 0 ist, eine entsprechende Verbindung, in der n Null ist, oxidiert.

2. Verfahren nach Anspruch 1, worin $R_1$ $R_3SO_2$ ist, $R_3$ $C_1$—$C_4$-Alkyl, $C_3$—$C_4$-Alkenyl oder Cyclopentyl ist, $R_2$ H, F, Cl, Br, $CH_3$ oder $OCH_3$ ist, und T N ist.

3. Verfahren nach Anspruch 1, worin

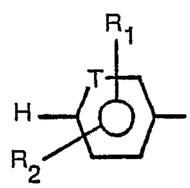

-

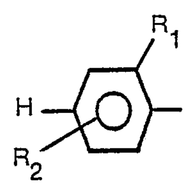

ist und $R_3$ $C_3$- oder $C_4$-Alkyl oder -Alkenyl ist.

4. Verfahren nach Anspruch 1, worin W, O und $R_2$ H, F, Cl, Br, $CH_3$ oder $OCH_3$ ist.

5. Verfahren nach Anspruch 4, worin $R_1$ in 2- oder 4-Stellung ist, wenn T N ist.

6. Verfahren nach Anspruch 5, bei dem n 2 ist.

7. Verfahren nach Anspruch 6, bei dem $R_2$ H ist.

8. Verfahren nach Anspruch 7, bei dem X $CH_3$ oder $OCH_3$ ist.

9. Verfahren nach Anspruch 8, bei dem $R_3$ $C_1$—$C_4$-Alkyl ist.

10. Verfahren nach Anspruch 1, bei dem das Produkt eine Verbindung ist, ausgewählt aus

N-[(4,6-Dimethylpyrimidin-2-yl)-aminocarbonyl]-2-[(1-methylethyl)-sulfonyl]-benzolsulfonamid,

N-[(4-Methoxy-6-methylpyrimidin-2-yl)-aminocarbonyl]-2-[(1-methylethyl)-sulfonyl]-benzolsulfonamid,

N-[(4,6-Dimethoxypyrimidin-2-yl)-aminocarbonyl]-2-[(1-methylethyl)-sulfonyl]-benzolsulfonamid,

N-[(4,6-Dimethylpyrimidin-2-yl)-aminocarbonyl]-2-(propylsulfonyl)-benzolsulfonamid,

N-[(4-Methoxy-6-methylpyrimidin-2-yl)-aminocarbonyl]-2-(propylsulfonyl)-benzolsulfonamid,

N-[(4,6-Dimethoxypyrimidin-2-yl)-aminocarbonyl]-2-(propylsulfonyl)-benzolsulfonamid,

N-[(4,6-Dimethyl-1,3,5-triazin-2-yl)-aminocarbonyl]-2-(propylsulfonyl)-benzolsulfonamid;

oder ein landwirtschaftlich geeignetes Salz dieser Verbindungen.

11. Verfahren nach Anspruch 1, worin das Produkt eine Verbindung ist, ausgewählt aus

N-[(4-Methoxy-6-methyl-1,3,5-triazin-2-yl)-aminocarbonyl]-2-(propylsulfonyl)-benzolsulfonamid,

N-[(4,6-Dimethoxy-1,3,5-triazin-2-yl)-aminocarbonyl]-2-(propylsulfonyl)-benzolsulfonamid,

N-[(4-Dimethyl-1,3,5-triazin-2-yl)-aminocarbonyl]-2-[(1-methylethyl)-sulfonyl]-benzolsulfonamid,

N-[(4-Methoxy-6-methyl-1,3,5-triazin-2-yl)-aminocarbonyl]-2-[(1-methylethyl)-sulfonyl]-benzolsulfonamid,

N-[(4,6-Dimethoxy-1,3,5-triazin-2-yl)-aminocarbonyl]-2-[(1-methylethyl)-sulfonyl]-benzolsulfonamid;

oder ein landwirtschaftlich geeignetes Salz dieser Verbindungen.

12. Verfahren nach Anspruch 1, worin das Produkt eine Verbindung ist, ausgewählt aus

N-[(4,6-Dimethylpyrimidin-2-yl)-aminocarbonyl]-2-(methylsulfonyl)-3-pyridinsulfonamid,

N-[(4-Methoxy-6-methylpyrimidin-2-yl)-aminocarbonyl]-2-(methylsulfonyl)-3-pyridinsulfonamid,

N-[(4,6-Dimethoxy-1,3,5-triazin-2-yl)-aminocarbonyl]-2-(methylsulfonyl)-3-pyridinsulfonamid,

N-[(4,6-Dimethoxypyrimidin-2-yl)-aminocarbonyl]-2-(methylsulfonyl)-3-pyridinsulfonamid,

N-[(4,6-Dimethyl-1,3,5-triazin-2-yl)-aminocarbonyl]-2-(methylsulfonyl)-3-pyridinsulfonamid; und

N-[(4-Methoxy-6-methyl-1,3,5-triazin-2-yl)-aminocarbonyl]-2-(methylsulfonyl)-3-pyridinsulfonamid;

oder ein landwirtschaftlich geeignetes Salz dieser Verbindungen.

13. Verfahren zur Kontrolle des Wachstums unerwünschter Vegetation durch Aufbringung einer wirksamen Menge einer herbiziden Verbindung auf die zu schützende Stelle, dadurch gekennzeichnet, dass die herbizide Verbindung eine Verbindung der Formel (I), wie in einem der Ansprüche 1 bis 12 definiert, enthält.

14. Verfahren zur Regulierung des Wachstums von Pflanzen durch Aufbringung einer wirksamen, aber im wesentlichen nicht-phytotoxischen Menge eines Wachstumsregulators auf die betreffende Stelle, dadurch gekennzeichnet, dass der Wachstumsregulator eine Verbindung von Formel (I), wie in einem der Ansprüche 1 bis 12 definiert, enthält.

**Revendications pour les Etats contractants: BE CH LI DE FR GB IT LU NL SE**

1. Un composé choisi parmi ceux de formule:

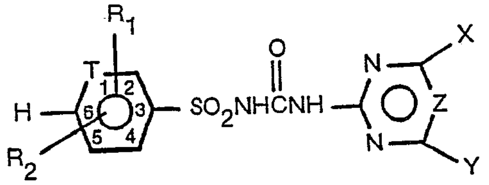

(I)

où

$R_1$ est $R_3S[O]_n$;
$R_3$ est un radical alcoyle en $C_1$—$C_4$, alcényle en $C_3$—$C_4$, cyclopentyle ou cyclopropylméthyle;
$R_2$ est H, F, Cl, Br, $CH_3$, $OCH_3$, $CF_3$, $NO_2$, CN ou $NH_2$;
n est 0, 1 ou 2;
W est 0 ou S;
T est CH ou N;
Z est CH ou N;
X est $CH_3$, $C_2H_5$, $OCH_3$, $OC_2H_5$ ou $CH_2OCH_3$; et
Y est $CH_3$ ou $OCH_3$;
et leurs sels convenant pour l'agriculture; avec les conditions que
(1) lorsque $R_2$ est CN, alors T est CH et $R_2$ n'est pas en position ortho par rapport au groupe sulfonylurée;
(2) lorsque W est S, alors n est 0 ou 2;
(3) lorsque T est N, alors n est 2;
(4) lorsque T est CH, alors $R_3$ n'est pas un radical alcoyle en $C_1$—$C_2$ et $R_1$ est dans la position 2 ou 4; et
(5) lorsque T est N, alors $R_3$ n'est pas un radical cyclopropylméthyle et $R_2$ n'est pas $NH_2$, $CF_3$ ou $NO_2$.

2. Un composé selon la revendication 1, dans lequel W est O et $R_2$ est H, F, Cl, Br, $CH_3$ ou $OCH_3$.

3. Un composé selon la revendication 2, dans lequel $R_1$ est dans la position 2 ou 4 lorsque T est N.

4. Un composé selon la revendication 3, dans lequel n est 2.

5. Un composé selon la revendication 4, dans lequel $R_2$ est H.

6. Un composé selon la revendication 5, dans lequel X est $CH_3$ ou $OCH_3$.

7. Un composé selon la revendication 6, dans lequel $R_3$ est un radical alcoyle en $C_1$—$C_4$.

8. Les composés selon la revendication 1, qui sont
le N-[(4,6-diméthylpyrimidin-2-yl)aminocarbonyl]-2-[(1-méthyléthyl)sulfonyl]benzènesulfonamide,
le N-[(4-méthoxy-6-méthylpyrimidin-2-yl)aminocarbonyl]-2-[(1-méthyléthyl)sulfonyl]benzène sulfonamide,
le N-[(4,6-diméthoxypyrimidin-2-yl)aminocarbonyl]-2-[(1-méthyléthyl)sulfonyl]benzènesulfonamide,
le N-[(4,6-diméthylpyrimidin-2-yl)aminocarbonyl]-2-(propylsulfonyl)benzènesulfonamide,
le N-[(4-méthoxy-6-méthylpyrimidin-2-yl)aminocarbonyl]-2-(propylsulfonyl)benzènesulfonamide,
le N-[(4,6-diméthoxypyrimidin-2-yl)aminocarbonyl]-2-(propylsulfonyl)benzènesulfonamide,
le N-[(4,6-diméthyl-1,3,5-triazin-2-yl)aminocarbonyl]-2-(propylsulfonyl)benzènesulfonamide; et leurs sels convenant pour l'agriculture.

9. Les composés selon la revendication 1 qui sont
le N-[(4-méthoxy-6-méthyl-1,3,5-triazin-2-yl)aminocarbonyl]-2-(propylsulfonyl)benzènesulfonamide,
le N-[(4,6-diméthoxy-1,3,5-triazin-2-yl)aminocarbonyl]-2-(propylsulfonyl)benzènesulfonamide,
le N-[(4,6-diméthyl-1,3,5-triazin-2-yl)aminocarbonyl]-2-[(1-méthyléthyl)sulfonyl]benzènesulfonamide,
le N-[(4-méthoxy-6-méthyl-1,3,5-triazin-2-yl)aminocarbonyl]-2-[(1-méthyléthyl)sulfonyl]benzène sulfonamide,
le N-[(4,6-diméthoxy-1,3,5-triazin-2-yl)aminocarbonyl]-2-[(1-méthyléthyl)sulfonyl]benzène sulfonamide; et leurs sels convenant pour l'agriculture.

10. Les composés selon la revendication 1, qui sont
le N-[(4,6-diméthylpyrimidin-2-yl)aminocarbonyl]-2-(méthylsulfonyl)-3-pyridinesulfonamide,
le N-[(4-méthoxy-6-méthylpyrimidin-2-yl)aminocarbonyl]-2-(méthylsulfonyl)-3-pyridinesulfonamide,
le N-[(4,6-diméthoxy-1,3,5-triazin-2-yl)aminocarbonyl]-2-(méthylsulfonyl)-3-pyridinesulfonamide,
le N-[(4,6-diméthoxypyrimidin-2-yl)aminocarbonyl]-2-(méthylsulfonyl)-3-pyridinesulfonamide,
le N-[(4,6-diméthyl-1,3,5-triazin-2-yl)aminocarbonyl]-2-(méthylsulfonyl)-3-pyridinesulfonamide; et
le N-[(4-méthoxy-6-méthyl-1,3,5-triazin-2-yl)aminocarbonyl]-2-(méthylsulfonyl)-3-pyridine sulfonamide; et leurs seuls convenant pour l'agriculture.

11. Un composé selon la revendication 1, dans lequel $R_1$ est $R_3SO_2$; $R_3$ est un radical alcoyle en $C_1$—$C_4$; alcényle en $C_3$—$C_4$ ou cyclopentyle; $R_2$ est H, F, Cl, Br, $CH_3$ ou $OCH_3$; et T est N.

12. Un composé selon la revendication 1, dans lequel

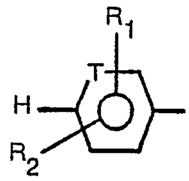 est 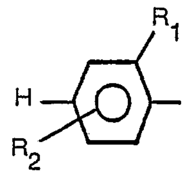 ;

et $R_3$ est un radical alcoyle en $C_3$ ou $C_4$ ou alcényle.

13. Un composé selon la revendication 1, qui est choisi parmi
le N-[(4,6-diméthylpyrimidin-2-yl)aminothiocarbonyl]-2-(allylthio)benzènesulfonamide;
le N-[(4,6-diméthyl-1,3,5-triazin-2-yl)aminocarbonyl]-2-(allylsulfonyl)benzènesulfonamide;
le N-[(4-méthoxy-6-méthyl-1,3,5-triazin-2-yl)aminocarbonyl]-2-(allylsulfonyl)benzènesulfonamide;
le N-[(4,6-diméthoxy-1,3,5-triazin-2-yl)aminocarbonyl]-2- (3-butèn-2-ylsulfonyl)benzènesulfonamide;
le N-[(4,6-diméthyl-1,3,5-triazin-2-yl)aminocarbonyl]-2-(3-butèn-2-ylsulfonyl)benzènesulfonamide;
le N-[(4-méthoxy-6-méthyl-1,3,5-triazin-2-yl)aminocarbonyl]-2-(3-butèn-2-ylsulfonyl)benzène-sulfonamide; ou
le N-[(4,6-diméthoxy-1,3,5-triazin-2-yl)aminocarbonyl]-2-(3-butèn-2-ylsulfonyl)benzènesulfonamide.

14. Une composition propre à maîtriser la croissance de végétation indésirable, qui comprend une quantité efficace d'un composé herbicide et d'au moins un des ingrédients suivants: agent tensio-actif, diluant solide ou liquide, caractérisée en ce que ledit composé herbicide comprend un composé selon l'une quelconque des revendications 1 à 13.

15. Un procédé pour maîtriser la croissance de végétation indésirable par application au lieu à protéger d'une quantité efficace d'un composé herbicide, caractérisé en ce que ledit composé herbicide comprend un composé selon l'une quelconque des revendications 1 à 13.

16. Le procédé de la revendication 15, dans lequel la végétation indésirable est sélectivement maîtrisée en présence de plantes cultivées.

17. Un procédé pour régler la croissance de plantes par application au lieu de ces plantes d'une quantité efficace mais substantiellement non phytotoxique d'un agent de réglage de la croissance de plantes, caractérisé en ce que ledit agent de réglage de la croissance de plantes comprend un composé selon l'une quelconque des revendications 1 à 13.

18. Un procédé de préparation d'un composé selon la revendication 1, qui comprend:
(a) la réaction mutuelle de composés des formules générales

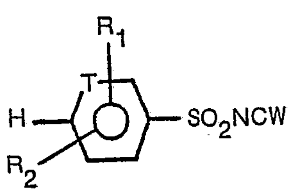 et 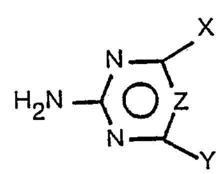

où $R_1$ est $R_3SO_2$ ou $R_3S$, $R_2$ est tel que défini dans la revendication 1, à l'exception de $NH_2$, $R_3$, T, W, X, Y et Z sont tels que définis dans la revendication 1;
ou bien
(b) la préparation d'un composé selon la revendication 1 dans lequel $R_2$ est $NH_2$, en réduisant un composé correspondant dans lequel $R_2$ est $NO_2$;
ou bien
(c) la réaction mutuelle de composés des formules générales

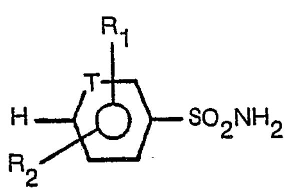 et 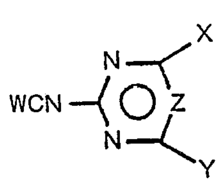

où $R_1$ est $R_2S$ ou $R_3SO_2$ et $R_2$, T, W, X, Y et Z sont tels que défnis dans la revendication 1.

19. Un procédé de préparation d'un composé selon la revendication 1, dans lequel $R_2$ est $NH_2$, qui comprend la réduction d'un composé correspondant dans lequel $R_2$ est $NO_2$.

20. Un procédé de préparation d'un composé selon la revendication 1, dans lequel n est 1 et W est 0, qui comprend l'oxydation d'un composé correspondant dans lequel n vaut zéro.

**Revendications pour l'Etat contractant: AT**

1. Un procédé de préparation d'un composé choisi parmi ceux de formule

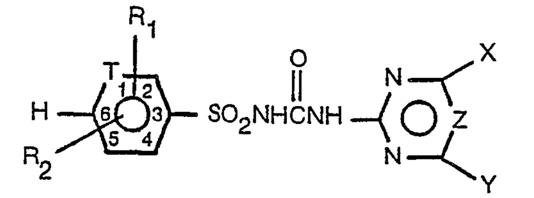

(I)

où

$R_1$ est $R_3S[O]_n$;
$R_3$ est un radical alcoyle en $C_1$—$C_4$, alcényle en $C_3$—$C_4$, cyclopentyle ou cyclopropylméthyle;
$R_2$ est H, F, Cl, Br, $CH_3$, $OCH_3$, $CF_3$, $NO_2$, CN ou $NH_2$;
n est 0, 1 ou 2;
W est 0 ou S;
T est CH ou N;
Z est CH ou N;
X est $CH_3$, $C_2H_5$, $OCH_3$, $OC_2H_5$ ou $CH_2OCH_3$; et
Y est $CH_3$ ou $OCH_3$;
et leurs sels convenant pour l'agriculture; avec les conditions que

(1) lorsque $R_2$ est CN, alors T est CH et $R_2$ n'est pas en position ortho par rapport au groupe sulfonylurée;
(2) lorsque W est S, alors n est 0 ou 2;
(3) lorsque T est N, alors n est 2;
(4) lorsque T est CH, alors $R_3$ n'est pas un radical alcoyle en $C_1$—$C_2$ et $R_1$ est dans la position 2 ou 4; et
(5) lorsque T est N, alors $R_3$ n'est pas un radical cyclopropylméthyle et $R_2$ n'est pas $NH_2$, $CF_3$ ou $NO_2$;

qui comprend

(a) la réaction mutuelle de composés des formules générales

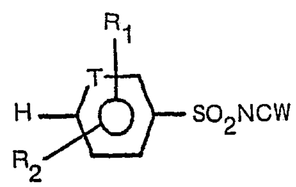

et

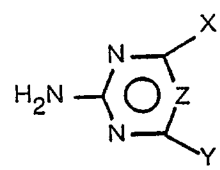

où $R_1$ est $R_3SO_2$ ou $R_3S$, $R_2$ est tel que défini ci-dessus, à l'exception de $NH_2$, $R_3$, T, W, X, Y et Z sont tels que définis ci-dessus;

(b) la réaction mutuelle de composés des formules générales

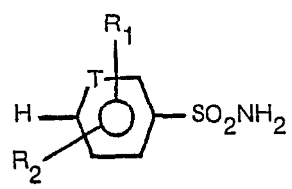

et

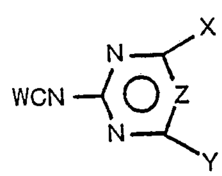

où $R_1$ est $R_3S$ ou $R_3SO_2$ et $R_2$, T, W, X, Y et Z sont tels que définis ci-dessus;

(c) la préparation d'un composé de formule I dans lequel $R_2$ est $NH_2$, en réduisant un composé correspondnat dans lequel $R_2$ est $NO_2$; ou bien

(d) la préparation d'un composé de formule I dans lequel n est 1 et W est 0, en oxydant un composé correspondant dans lequel n vaut zéro.

2. Un procédé selon la revendication 1, dans lequel $R_1$ est $R_3SO_2$; $R_3$ est un radical alcoyle en $C_1$—$C_4$, alcényle en $C_3$—$C_4$ ou cyclopentyle; $R_2$ est H, F, Cl, Br, $CH_3$ ou $OCH_3$; et T est N.

3. Un procédé selon la revendication 1, dans lequel

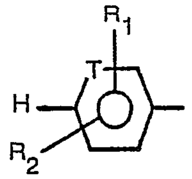 est 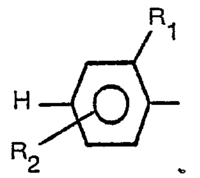 ;

et $R_3$ est un radical alcoyle en $C_3$ ou $C_4$ ou alcényle.

4. Un procédé selon la revendication 1, dans lequel W est O et $R_2$ est H, F, Cl, Br, $CH_3$ ou $OCH_3$.

5. Un procédé selon la revendication 4, dans lequel $R_1$ est dans la position 2 ou 4 lorsque T est N.

6. Un procédé selon la revendication 5 dans lequel n est 2.

7. Un procédé selon la revendication 6, dans lequel $R_2$ est H.

8. Un procédé selon la revendication 7, dans lequel X est $CH_3$ ou $OCH_3$.

9. Un procédé selon la revendication 8, dans lequel $R_3$ est un radical alcoyle en $C_1$—$C_4$.

10. Un procédé selon la revendication 1, dans lequel le produit est un composé choisi parmi
le N-[(4,6-diméthylpyrimidin-2-yl)aminocarbonyl]-2-[(1-méthyléthyl)sulfonyl]benzènesulfonamide,
le N-[(4-méthoxy-6-méthylpyrimidin-2-yl)aminocarbonyl]-2-[(1-méthyléthyl)sulfonyl]benzène-sulfonamide,
le N-[(4,6-diméthoxypyrimidin-2-yl)aminocarbonyl]-2-[(1-méthyléthyl)sulfonyl]benzènesulfonamide,
le N-[(4,6-diméthylpyrimidin-2-yl)aminocarbonyl]-2-(propylsulfonyl)benzènesulfonamide,
le N-[(4-méthoxy-6-méthylpyrimidin-2-yl)aminocarbonyl]-2-(propylsulfonyl)benzènesulfonamide,
le N-[(4,6-diméthoxypyrimidin-2-yl)aminocarbonyl]-2-(propylsulfonyl)benzènesulfonamide,
le N-[(4,6-diméthyl-1,3,5-triazin-2-yl)aminocarbonyl]-2-(propylsulfonyl)benzènesulfonamide; et leurs sels convenant pour l'agriculture.

11. Un procédé selon la revendication 1, dans lequel le produit est un composé choisi parmi
le N-[(4-méthoxy-6-méthyl-1,3,5-triazin-2-yl)aminocarbonyl]-2-(propylsulfonyl)benzènesulfonamide,
le N-[(4,6-diméthoxy-1,3,5-triazin-2-yl)aminocarbonyl]-2-(propylsulfonyl)benzènesulfonamide,
le N-[(4,6-diméthyl-1,3,5-triazin-2-yl)aminocarbonyl]-2-[(1-méthyléthyl)sulfonyl]benzènesulfonamide,
le N-[(4-méthoxy-6-méthyl-1,3,5-triazin-2-yl)aminocarbonyl]-2-[(1-méthyléthyl)sulfonyl]benzène-sulfonamide,
le N-[(4,6-diméthoxy-1,3,5-triazin-2-yl)aminocarbonyl]-2-[(1-méthyléthyl)sulfonyl]benzène-sulfonamide; et leurs sels convenant pour l'agriculture.

12. Un procédé selon la revendication 1, dans lequel le produit est composé choisi parmi
le N-[(4,6-diméthylpyrimidin-2-yl)aminocarbonyl]-2-(méthylsulfonyl)-3-pyridinesulfonamide,
le N-[(4-méthoxy-6-méthylpyrimidin-2-yl)aminocarbonyl]-2-(méthylsulfonyl)-3-pyridinesulfonamide,
le N-[(4,6-diméthoxy-1,3,5-triazin-2-yl)aminocarbonyl]-2-(méthylsulfonyl)-3-pyridinesulfonamide,
le N-[(4,6-diméthoxypyrimidin-2-yl)aminocarbonyl]-2-(méthylsulfonyl)-3-pyridinesulfonamide,
le N-[(4,6-diméthyl-1,3,5-triazin-2-yl)aminocarbonyl]-2-(méthylsulfonyl)-3-pyridinesulfonamide; et
le N-[(4-méthoxy-6-méthyl-1,3,5-triazin-2-yl)aminocarbonyl]-2-(méthylsulfonyl)-3-pyridine-sulfonamide; et leurs seuls convenant pour l'agriculture.

13. Un procédé pour maîtriser la croissance de végétation indésirable par application au lieu à protéger d'une quantité efficace d'un composé herbicide, caractérisé en ce que ledit composé herbicide comprend un composé de formule I tel que défini à l'une quelconque des revendications 1 à 12.

14. Un procédé pour régler la croissance de plantes par application au lieu de ces plantes d'une quantité efficace mais substantiellement non-phytotoxique d'un agent de réglage de la croissance de plantes, caractérisé en ce que ledit agent de réglage de la croissance de plantes comprend un composé de formule I tel que défini à l'une quelconque des revendications 1 à 12.